# EUROPEAN PATENT APPLICATION

(11) **EP 4 702 986 A1**
(43) Date of publication of application: **04.03.2026**
(21) Application number: 24796044.6
(22) Date of filing: 23.04.2024
(51) Int. Cl.: A61K 38/22, A61K 38/16, A61P 3/00, C07K 14/00, C07K 1/04

(54) **POLYPEPTIDE AND USE THEREOF**

(30) Priority: 26.04.2023 CN 202310461975; 27.12.2023 CN 202311825930
(71) Applicant: Biocells (Beijing) Biotech Co., Ltd., Beijing 100070 (CN)
(72) Inventor: HAN, Huamin, Beijing 100070 (CN); JI, Qi, Beijing 100070 (CN); FENG, Siliang, Beijing 100070 (CN)
(74) Representative: Mewburn Ellis LLP
(86) International application number: PCT/CN2024/089274
(87) International publication number: WO 2024/222663

(57) **Abstract**

The present invention belongs to the field of biotechnology. Provided is a polypeptide and the use thereof. The polypeptide is established on the basis of a mechanism study on an ETBR agonist, and belongs to a highly selective agonist of ETBR. The polypeptide prolongs the half-life of a drug, increases in vivo exposure, and reduces the frequency of administration by means of study on structure-activity relationship and structural modification, and has a lower side effect than that of IRL-1620 in animal experiments, and a larger safety window of a drug. The polypeptide can be used for treating diseases related to an ETBR receptor, such as rehabilitation treatment of a patient with ischemic stroke in the acute stage and the recovery stage, so as to promote better recovery of the patient with stroke.

## Description

This application claims priority to Chinese Patent Application No. 202310461975.3, filed on April 26, 2023 with the China National Intellectual Property Administration, titled "POLYPEPTIDE AND USE THEREOF", the entire content of which is incorporated herein by reference.

This application claims priority to Chinese Patent Application No. 202311825930.6, filed on December 27, 2023 with the China National Intellectual Property Administration, titled "POLYPEPTIDE AND USE THEREOF", the entire content of which is incorporated herein by reference.

### TECHNICAL FIELD

The present invention relates to the field of biotechnology, and particularly to polypeptides and use thereof.

### BACKGROUND

Current therapeutic measures and medications for stroke primarily include vascular intervention, thrombolysis, antiplatelet therapy, anticoagulant therapy, defibrinolytic therapy, and neuroprotectant therapy. Generally, vascular intervention and thrombolysis are required to be performed in a very early stage after the onset of stroke, and vascular intervention needs to be completed in a hospital with certain capabilities. Antiplatelet drugs and anticoagulant drugs are generally used in combination with thrombolytic drugs in an advanced stage, and they have limited efficacy and certain bleeding risks. Neuroprotectants have good potential and are generally used as therapeutic drugs for stroke at the acute phase. However, there is still no marketed therapeutic drug for promoting the rehabilitation of a stroke patient at the subacute phase and recovery phase. Previous studies have shown that endothelin receptor B agonists have demonstrated great potential in promoting long-term recovery and later-stage rehabilitation of stroke patients.

Endothelins (ETs) are polypeptides containing 21 amino acids, which can be divided into three subtypes: ET-1, ET-2 and ET-3. These subtypes have structural homology with snake venom sarafotoxins b and c (S6b, S6c). Endothelins are known as the most potent and longest-acting vasoactive peptides at present, and have a strong vasoconstrictive effect and can promote the migration and proliferation of smooth muscle cells. They exert extensive effects on various systems in the body and are closely associated with diseases such as hypertension, congestive heart failure, diabetes, cancer and fibrosis.

Endothelins exert their biological effects mainly by binding to an endothelin receptor (ETR) on the membrane of a target cell, and play an important role in the occurrence and progression of various diseases, including cardiovascular and cerebrovascular diseases, kidney diseases, diabetes, autoimmune diseases and tumors. Human ETRs are divided into two subtypes: ETA and ETB, both of which are G-protein-coupled receptors (GPCRs), but have different functions. ETA is considered as a primary vasoconstrictive and growth-promoting receptor, while ETB inhibits cell growth and vasoconstriction in the vascular system, and is called a "clearance receptor" as it is involved in the clearance of ET-1. Endothelins and their receptors are diversely and widely distributed in various tissues, mediate complex biological effects through different subtypes and signaling systems, and are closely associated with a variety of diseases, where ETA and ETB receptors play important roles.

Studies have shown that ETBR agonists have demonstrated great potential in promoting the long-term recovery and later-stage rehabilitation of stroke patients. Among the currently discovered ETBR agonist compounds, sovateltide (IRL-1620, Suc-DEEAVYFAHLDIIW) is the only ETBR agonist that has been advanced to the clinical research stage. However, IRL-1620 has a very short half-life (less than 2 minutes) and a very low exposure. Repeated administrations are required in preclinical studies and clinical studies, which greatly reduces its duration of efficacy and patient compliance.

### SUMMARY

In view of the above, provided in the present invention are polypeptides and use thereof.

Provided in the invention are polypeptides and use thereof. The polypeptides identified in this patent are established on the basis of a mechanism study on ETBR agonists, and belong to highly selective ETBR agonists. The half-lives of the polypeptides developed in this patent are prolonged by means of studies on structure-activity relationships and structural modification, increasing *in vivo* exposure and reducing the frequency of administration. The polypeptides have lower side effects than those of IRL-1620 and a larger therapeutic window in an animal experiment. The polypeptides in this invention may be used for treating an ETBR receptor-related disease, such as for the rehabilitation of a patient with ischemic stroke at the recovery stage, and to promote improved recovery of the patient with stroke.

To achieve the above-mentioned objectives of the invention, the following technical solutions are provided in the present invention.

Provided in the present invention is a polypeptide comprising:
(I) a structure as shown in Formula I,

   X1-X2-X3-(A5)-X4-X5-(A1)-X6(A2)-X7-X8-X9-(A3)-X10-X11-(A4)- X12-X13-X14-X15-X16 Formula I;

   or
(II) a sequence having one or more amino acid substitutions, deletions, additions and/or replacements in the structure as shown in (I); or
(III) a sequence having 90% or more similarity to the structure as shown in (I); or
(IV) a pharmaceutically acceptable salt, solvate, chelate or noncovalent complex formed from the structure as shown in Formula I; and/or
(V) a prodrug based on the structure as shown in Formula I; and/or
(VI) any mixture comprising (IV) and/or (V);

wherein preferably, X1 is selected from Suc, Fum, MeO-Fum, Ac, SA, MA, FA, A1-D or A 1-NH-C₂H₄-NH-Suc;
preferably, X2 is selected from D, E or K(A1);
X1 and X2 as a whole can be: 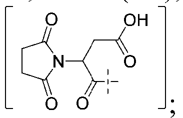
preferably, X3 is selected from E, D or K(A1);
preferably, X4 is selected from E, E(C16) or D;
preferably, X5 is selected from A, K, R, H, E or D;
preferably, X6 is selected from V, I, K, Dab, Orn, L, F, W or C;
preferably, X7 is selected from Y, F or W;
preferably, X8 is selected from F, Y or W;
preferably, X9 is selected from A, K, R, H, D, E, Dab or Orn;
preferably, X10 is selected from H, K or R;
preferably, X11 is selected from K, L, L(N-Me), I, V, Cit, H, R, L-2-amino-4-guanidinobutyric acid or Orn;
preferably, X12 is selected from D or E;
preferably, X13 is selected from I, I(N-Me), 2-Abu, L, P or V;
preferably, X14 is selected from I, 2-Abu, L or V;
preferably, X15 is selected from W, F, Y or 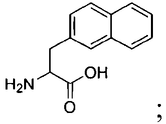
preferably, X16 is selected from OH or NH₂;
preferably, A5 is selected from C or absent;
preferably, A1, A2, A3 or A4 independently comprises (Y1-Y2-Y3-Y4);
preferably, Y1 is selected from -NH-(CH₂-CH₂-O)m1-(CH₂)m2-CO-, L/D-Glu, or absent, wherein L/D-Glu is attached to the amino group on the side chain of an amino acid on the backbone of the polypeptide through the α- or γ-carboxyl group and to the carboxyl group of Y2 through the amino group;
preferably, Y2 is selected from -NH-(CH₂-CH₂-O)m3-(CH₂)m4-CO-, 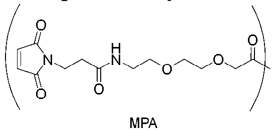 or absent;
preferably, Y3 is selected from -NH-(CH₂-CH₂-O)m5-(CH₂)m6-CO-, L/D-Glu or absent, wherein L/D-Glu is attached to the amino group of Y2 through the α- or γ-carboxyl group and to the carboxyl group of Y4 through the amino group;
preferably, Y4 is selected from D-Biotin, a structure shown in Formula 2, HOOC-(CH₂)ₙ-CO, CH₃-(CH₂)ₙ-CO or absent; 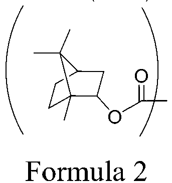
wherein m1, m2, m3, m4, m5 or m6 is an integer from 1 to 10;
wherein n is an integer selected from 10 to 25;
preferably, A1, A2, A3, or A4 can only exist individually, or all of them is absent; and
wherein the polypeptide having the structure of Formula I is not Suc-DEEAVYFAHLDIIW.

In some specific embodiments of the invention, wherein
preferably, X1 is selected from Suc, Fum, MeO-Fum, Ac, SA, MA, FA(ODDA-γE-AEEA-AEEA-)D or (ODDA-γE-AEEA-AEEA-)NH-C₂H₄-NH-Suc;
preferably, X2 is D or K(A1);
preferably, X1 and X2 as a whole can be 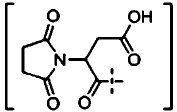
preferably, X3 is E or K(A1);
preferably, X4 is selected from E or E(C16);
preferably, X5 is selected from A, K or E;
preferably, X6 is selected from V, K, Dab, L, F or C;
preferably, X7 is Y;
preferably, X8 is F;
preferably, X9 is selected from A, K or E;
preferably, X10 is H;
preferably, X11 is selected from K, L, L(N-Me), R, Cit, L-2-amino-4-guanidinobutyric acid or Orn;
preferably, X12 is D;
preferably, X13 is selected from I, I(N-Me), 2-Abu, P or V;
preferably, X14 is I;
preferably, X15 is selected from W or 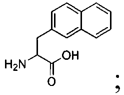
preferably, A1 is selected from -γE-AEEA-AEEA-ODDA, ODDA-γE-AEEA-AEEA-, Biotin, -AEEA-AEEA-yE-ODDA or absent;
preferably, A2 is selected from -γE-AEEA-AEEA-ODDA or absent;
preferably, A3 is selected from -γE-AEEA-AEEA-ODDA or absent;
preferably, A4 is selected from -AEEA-AEEA-γE-ODDA, -γE-AEEA-AEEA-C18, -γE-AEEA-AEEA-C16, -γE-AEEA-AEEA-C12, -γE-AEEA-AEEA-B, -γE-AEEA-AEEA-ODDA, CPAK-MPA-, -AEEA-γE-C18, -AEEA-γE-C16, -AEEA-γE-C12, -AEEA-E-C12 or absent.

In some specific embodiments of the present invention, the structure shown in Formula I is subjected to cyclization through any one of:
(I) introducing a C to form a disulfide bond; and/or
(II) forming an amide bond between the carboxyl group on the side chain of a D or a E and the amino group on the side chain of a K or a Dab.

In some specific embodiments of the invention, the cyclization includes any one of:
(I) inserting a C between X3 and X4 while replacing X6 with a C, and cyclizing through a disulfide bond formed by the two Cs; and/or
(II) cyclizing through an amide bond formed by the E of X3 and the K or Dab of X6; and/or
(III) cyclizing through an amide bond formed by the E of X5 and the K of X11.

In some specific embodiments of the invention, the polypeptide comprises a sequence as follows:

| Serial Number SEQ ID No. | ID | Sequence | MW |
|---|---|---|---|
| 2 | BX-5 | Suc-DEEAVYFAHKDIIW | 1836 |
| 3 | F-BX-5 | Fum-DEEAVYFAHKDIIW | 1834 |
| 4 | BX-7 | Suc-DEEAVYFAHLDVIW | 1806 |
| 5 | BX-8 | Suc-DEEAVYFAHLD(2-Abu)IW | 1792 |
| 6 | BX-34 | Fum-DEEAVYFAHK(-AEEA-AEEA-γE-ODDA )DIIW | 2549 |
| 7 | BX-43 | Suc-DEEAKYFAHLDIIW | 1850 |
| 8 | BX-45 | Suc-DEEEVYFAHKDIIW | 1893 |
| 9 | BX-48 | Suc-D cyclo(EEAK)YFAHLDIIW | 1832 |
| 10 | BX-49 | Suc-D cyclo(EEADab)YFAHLDIIW | 1804 |
| 11 | BX-50 | Suc-DEE cyclo(EVYFAHK)DIIW | 1876 |
| 12 | BX-51 | Fum-DEEAVYFAHOrn(-AEEA-AEEA-γE-ODDA)DIIW | 2535 |
| 13 | BX-52 | Fum-DEEEVYFAHKDIIW | 1891 |
| 14 | BX-53 | Suc-DEEEVYFAHOrnDIIW | 1880 |
| 15 | BX-54 | Suc-DEEEVYFAHK(-AEEA-AEEA-γE-ODDA)DIIW | 2610 |
| 16 | BX-57 | Suc-DE cyclo(CEEC)YFAHKDIIW | 1999 |
| 17 | BX-58 | MeO-Fum-DEEEVYFAHKDIIW | 1906 |
| 18 | BX-59 | Fum-DEEEVYFAHOrnDIIW | 1878 |
| 19 | BX-60 | Fum-DEEEVYFAHK(-AEEA-AEEA-γE - ODDA)DIIW | 2608 |
| 20 | BX-61 | Suc-DEEAVYFAHOrn(-AEEA-AEEA-γE - ODDA)DIIW | 2538 |
| 21 | BX-62 | Fum-DEE cyclo(EVYFK)HKD(2-Abu)IW | 1903 |
| 22 | BX-63 | Suc-D cyclo(EEAK)YFAHLD(2-Abu)IW | 1804 |
| 23 | BX-64 | Fum-D cyclo(EEAK)YFAHLD(2-Abu)IW | 1802 |
| 24 | BX-65 | MeO-Fum-D cyclo(EEAK)YFAHLD(2-Abu)IW | 1814 |
| 25 | BX-66 | Fum-DEEK(-γE-AEEA-AEEA-ODDA)VYFAHLDIIW | 2592 |
| 26 | BX-67 | Fum-DEEAK(-γE-AEEA-AEEA-ODDA)YFAHLDIIW | 2564 |
| 27 | BX-68 | Fum-DEEAVYFK(-γE-AEEA-AEEA-ODDA)HLDIIW | 2592 |
| 28 | BX-69 | Fum-DEEAVYFAHK(-γE-AEEA-AEEA-C18)DIIW | 2520 |
| 29 | BX-70 | Fum-DEEAVYFAHK(-γE-AEEA-AEEA-C16)DIIW | 2492 |
| 30 | BX-71 | Fum-DEEAVYFAHK(-γE-AEEA-AEEA-C12)DIIW | 2436 |
| 31 | BX-72 | Fum-DEEAVYFAHK(-γE-AEEA-AEEA-B)DIIW | 2433 |
| 32 | BX-74 | Suc-DEEAVYFAHK(-γE-AEEA-AEEA-ODDA )DIIW | 2552 |
| 33 | BX-75 | MeO-Fum-DEEAVYFAHK(-γE-AEEA-AEEA-ODDA)DIIW | 2564 |
| 34 | BX-76 | Suc-D cyclo(EEAK)YFAHLDVIW | 1818 |
| 35 | BX-77 | Suc-D cyclo(EEAK)YFAHK(-γE-AEEA-AEEA-ODDA)DIIW | 2563 |
| 36 | BX-78 | Suc-DEEAVYFAHK(-γE-AEEA-AEEA-ODDA)DVIW | 2538 |
| 37 | BX-79 | Suc-D cyclo(EEAK)YFAHK(-γE-AEEA-AEEA-ODDA)DVIW | 2550 |
| 38 | BX-86 | Fum-DEEAVYFAHK(-γE-AEEA-AEEA-ODDA)DIIW-NH2 | 2549 |
| 39 | BX-95 | Fum-DEEAVYFAHK(CPAK-MPA-)DIIW | 2661 |
| | | 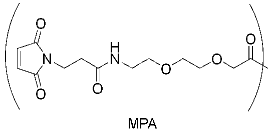 | |
| 40 | BX-99 | Fum-D cyclo(EEAK)YFAHLDVIW | 1916 |
| 41 | BX-100 | Fum-D cyclo(EEAK)YFAHK(-γE-AEEA-AEEA-ODDA)DVIW | 2547 |
| 42 | BX-101 | Fum-DEEAVYFAHK(-γE-AEEA-AEEA-ODDA)DVIW | 2536 |
| 43 | BX-102 | Fum-D cyclo(EEAK)YFAHLDIIW | 1829 |
| 44 | BX-103 | Fum-D cyclo(EEAK)YFAHK(-γE-AEEA-AEEA-ODDA)DIIW | 2561 |
| 45 | BX-104 | Fum-D cyclo(EEAK)YFAHK(-γE-AEEA-AEEA-ODDA)D(2-Abu)IW | 2534 |
| 46 | BX-105 | Fum-DEEAKYFAHLDIIW | 1847 |
| 47 | BX-106 | Fum-DEEAKYFAHLDVIW | 1836 |
| 48 | BX-107 | Fum-DEEAKYFAHK(-γE-AEEA-AEEA-ODDA)DIIW | 2579 |
| 49 | BX-108 | Fum-DEEAKYFAHK(-γE-AEEA-AEEA-ODDA)DVIW | 2567 |
| 50 | BX-109 | Fum-DEEAK(-γE-AEEA-AEEA-ODDA)YFAHLDIIW | 2566 |
| 51 | BX-110 | Fum-DEEAK(-γE-AEEA-AEEA-ODDA)YFAHLDVIW | 2549 |
| 52 | BX-111 | SA-DEEAVYFAHLDIIW | 1844 |
| 53 | BX-112 | MA-DEEAVYFAHLDIIW | 1844 |
| 54 | BX-13-2 | Fum-DEEAVYFAHKDIIW1 | 1843.8 |
| | | 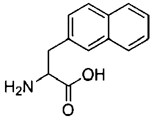 W1: | |
| 55 | BX-9 | suc-DEEAVYFAHRIDIIW (R1=L-2-amino-4-guanidinobutyric acid) | 1849.98 |
| 56 | BX-31 | Fum-DEEAVYFAHOrnDIIW | 1819 |
| 57 | BX-32 | Fum-DEEAVYFAHCitDIIW | 1862 |
| 58 | BX-38 | Suc-DE cyclo(CEAC)YFAHLDIIW | 1927 |
| 59 | BX-23 | Fum-DEEAVYFAHKDVIW | 1704.9 |
| 60 | BX-24 | Fum-DEEAVYFAHKD(2-Abu)IW | 1805.9 |
| 61 | BX-25 | Suc-DEEAVYFAHLDI(N-Me)IW | 1833.85 |
| 62 | BX-26 | Suc-DEEAVYFAHL(N-Me)DIIW | 1833.85 |
| 63 | BX-27 | Suc-DEEAVYFAHL(N-Me)DI(N-Me)IW | 1847.86 |
| 64 | BX-28 | Fum-DEEAVYFAHL(N-Me)DIIW | 1845.85 |
| 65 | BX-29 | Fum-DEEAVYFAHL(N-Me)DI(N-Me)IW | 1847.86 |
| 66 | FBX-7 | Fum-DEEAVYFAHLDVIW | 1805.9 |
| 67 | FBX-8 | Fum-DEEAVYFAHLD(2-Abu)IW | 1790.9 |
| 68 | QP-1 | 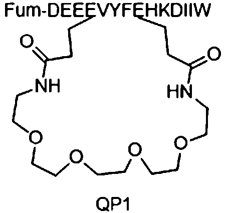 | 1848 |
| 69 | QP-3 | 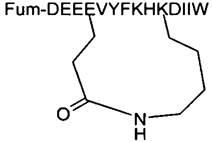 | 1931 |
| 70 | BX-15 | FA-DEEAVYFAHKDIIW | 1912.87 |
| 71 | BX-16 | Fum-DEEE(Biotin)VYFAHKDIIW | 2160.4 |
| 72 | BX-10 | suc-DEEAVYFAHLDPIW | 1804.96 |
| 73 | BX14-1 | Fum-DEE(C16)AVYFAHLDIIW | 2044.1 |
| 74 | BX-6 | Suc-DEEAVYFAHRDIIW | 1864.03 |
| 75 | BX-21 | Fum-DEEALYFAHKDIIW | 1848 |
| 76 | BX-22 | Fum-DEEAFYFAHKDIIW | 1882 |
| 77 | BX-223 | Fum-DEEAVYFAHK(-AEEA-γE-C18 )DIIW | 2374 |
| 78 | BX-224 | Fum-DEEAVYFAHK(-AEEA-γE-C16 )DIIW | 2346 |
| 79 | BX-225 | Fum-DEEAVYFAHK(-AEEA-γE-C12 )DIIW | 2290 |
| 80 | BX-253 | Fum-K(-AEEA-AEEA-γE-ODDA )EEAVYFAHLDIIW | 2547 |
| 81 | BX-254 | Fum-DK(-AEEA-AEEA-γE-ODDA )EAVYFAHLDIIW | 2533 |
| 82 | BX-255 | (ODDA-γE-AEEA-AEEA-)DDEEAVYFAHLDIIW | 2550 |
| 83 | BX-256 | (ODDA-γE-AEEA-AEEA-)NH-C₂H₄-NH-Suc-DEEAVYFAHLDIIW | 2594 |
| 84 | BX-221 | 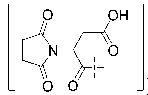 | 2533 |
| | | EEAVYFAHK(ODDA-γE-AEEA-AEEA-)DIIW | |
| 86 | BX-229 | Fum-DEEAVYFAHK(-AEEA-γE-C12)DVIW | 2276 |
| 87 | BX-238 | Fum-DEEAVYFAHK(-AEEA-E-C12)DVIW | 2276 |
| 88 | BX-56 | Suc-DE cvclo(CEEC)YFAHLDIIW | 1984 |

Also provided in the present invention is a method of preparing the polypeptide, comprising the following steps:
Step 1: obtaining a peptide resin of the polypeptide via solid-phase polypeptide synthesis; and
Step 2: cleaving and purifying to obtain the polypeptide.

Specifically, the present invention provides a method of preparing polypeptide BX-34, comprising the following steps.

Step 1. 0.50 g (0.25 mmol) of Fmoc-Trp(Boc)-Wang Resin is weighed and added into a reactor. It is swelled with 10 mL of DCM for 10 minutes, filtered by suction, and washed twice with DMF. 25% 4-methylpiperidine/DMF (by volume) is added and allowed to react for 30 minutes to remove the Fmoc group. After filtering by suction, the resin is washed four times with DMF and twice with DCM. A ninhydrin test is performed, and the resin solution is found to be blue. Fmoc-Ile-OH (1 mmol, 4 eq.) and HBTU (1 mmol, 4 eq.) are weighed, dissolved in DMF, and added with DIEA (2 mmol, 8 eq.) and mixed to uniformity. The mixture is added to the resin and allowed to react for 1 hour under magnetic stirring at room temperature (25°C±5°C). After filtering by suction, it is washed sequentially with DMF, MeOH, and DCM, three times each. A ninhydrin test is performed, and the resin is found to be colorless and transparent, while the solution is found to be light yellow, indicating a complete reaction. 25% 4-methylpiperidine/DMF (by volume) is added to remove the Fmoc group. This cycle is repeated to sequentially complete the coupling of Ile, Asp(OtBu), Lys(Dde), His(Trt), Ala, Phe, Tyr(tBu), Val, Ala, Glu(OtBu), Glu(OtBu), Asp(OtBu), and OtBu-Fum. Subsequently, 2% hydrazine hydrate/DMF is added to the peptide resin and allowed to react for 10 minutes×3 times to remove the Dde on the side chain of Lys. AEEA, AEEA, Glu-α-OtBu, and OtBu-ODDA are then condensed sequentially.

Step 2. The resin is dried under suction, and 15 mL of a pre-cooled cleavage solution (TFA: TIS: H₂O = 95:2.5:2.5) is added. The reaction is allowed to proceed for 3 hours at room temperature (25°C±5°C) and under stirring. The reaction solutions are extracted by suction, and the resin is washed twice with a small amount of TFA and then extracted by suction. The reaction solutions are combined, and approximately 200 mL of pre-cooled methyl tert-butyl ether is added. A white precipitate is formed. After centrifuging (5000 rpm×5 mins), the supernatant is discarded. Methyl tert-butyl ether is added again to the precipitate. After vortexing and centrifuging, the supernatant is discarded. The precipitate is placed in a vacuum dryer and dried for 12 hours to obtain a crude product.

Step 3. The crude peptide is dissolved in approximately 1% aqueous ammonia and filtered. The filtrate is purified with preparative liquid phases and subjected to a gradient elution to obtain a purified target compound. Fractions of the target compound with a purity >95% are combined, and acetonitrile is removed under reduced pressure at around 40°C using a water pump and a rotary evaporator. The resulting concentrated solution is frozen into a solid using liquid nitrogen and then freeze-dried for 48 hours using a freeze-dryer, to obtain the target compound, whose molecular weight is identified by mass spectrometry.

In some specific embodiments of the present invention, the preparative column employed in the purification comprises C18-10-100, 30×250 mm, and the flow rate employed is 25 mL/min; and the preparative liquid phases comprise a phase A (0.1%TFA/water), and a phase B (0.1%TFA/90% acetonitrile/water).

In some specific embodiments of the present invention, the method of preparing polypeptide BX-34 may also be applied to the preparation of BX-5, F-BX-5, BX-7, BX-8, BX-43, BX-45, BX-51, BX-52, BX-53, BX-54, BX-58, BX-59, BX-60, BX-61, BX-66, BX-67, BX-68, BX-69, BX-70, BX-71, BX-72, BX-73, BX-74, BX-75, BX-78, BX-86, BX-101, BX-105, BX-106, BX-107, BX-108, BX-109, BX-110, BX-111, BX-112, BX-229, and BX-238.

Specifically, also provided in the present invention is a method of preparing polypeptide BX-76, comprising the following steps.

Step 1. Synthesis of a backbone is done according to the method of synthesizing BX-34.

Step 2. A DCM solution of 0.3 equivalents of tetrakis(triphenylphosphine)palladium and 12 equivalents of phenylsilane is added to the peptide resin under nitrogen protection, and allowed to react at room temperature (25°C±5°C) for 2 h to remove the allyl ester group on the side chain of Glu and the allyloxycarbonyl group on the side chain of Lys. After filtration by suction, it is washed 5 times with DCM solvent and 3 times with DMF. 2 equivalents of PYBOP, 2 equivalents of HOBt and 4 equivalents of DIEA are added, dissolved in DMF, and allowed to react at room temperature (25°C±5°C) for 5 h. After filtration by suction, it is washed 3 times with DMF and 3 times with DCM. A ninhydrin test is performed. If the reaction is not complete, the condensation is repeated once.

Step 3. It is then subjected to cleavage and purification according to the method of BX-34, to give a purified target compound, whose molecular weight is identified by mass spectrometry.

In some specific embodiments of the present invention, the method of preparing polypeptide BX-76 may also be applied to the preparation of BX-48, BX-49, BX-50, BX-62, BX-63, BX-64, BX-65, BX-77, BX-79, BX-100, BX-103, BX-104, BX-99, BX-102.

Specifically, also provided in the present invention is a method of preparing polypeptide BX-56, comprising the following steps.

Step 1. Synthesis of a backbone is done according to the method of synthesizing BX-34.

Step 2. A DMF solution of 5 equivalents of iodine is added to the peptide resin and allowed to react at room temperature (25°C±5°C) for 6 h to remove the protecting group Trt on the side chain of Cys while oxidizing. After filtration by suction, it is washed 6 times with DMF and 3 times with DCM, and dried.

Step 3. It is then subjected to cleavage and purification according to the method of synthesizing BX-34, to give a purified target compound, whose molecular weight is identified by mass spectrometry.

In some specific embodiments of the present invention, the method of preparing polypeptide BX-56 may also be applied to the preparation of BX-57.

Specifically, also provided in the present invention is a method of preparing polypeptide BX-95, comprising the following steps.

Step 1. A purified peptide backbone is obtained according to the method of synthesizing BX-34, and a purified CPAK peptide is obtained according to the method of synthesizing BX-34.

Step 2. Equal equivalents of the purified peptide backbone and the purified CPAK peptide are dissolved in 50% aqueous acetonitrile solution, with pH adjusted to 8-9 with triethylamine. The reaction is allowed to proceed at room temperature (25°C±5°C) until completion.

Step 3. It is then subjected to purification according to the method of BX-34, to give a purified target compound, whose molecular weight is identified by mass spectrometry.

In view of the above studies, also provided in the present invention is a polypeptide prepared by the preparation method.

Also provided in the present invention is use of the polypeptide in the manufacture of a medicament for the treatment of an ETBR receptor-related disease.

In some specific embodiments of the present invention, the ETBR receptor-related disease includes one or more of stroke, Alzheimer's disease, spinal cord injury, aortic stenosis, or neonatal hypoxic-ischemic encephalopathy;
wherein the stroke includes ischemic stroke and/or hemorrhagic stroke.

Also provided in the present invention is a medicament comprising the polypeptide and a pharmaceutically acceptable excipient or adjuvant.

Also provided in the present invention is a pharmaceutical composition including the medicament and any other active ingredient.

Also provided in the present invention is a method of treating an ETBR receptor-related disease, comprising administering to a subject any one of:
(I) the polypeptide; and/or
(II) the medicament; and/or
(III) the pharmaceutical composition.

In some specific embodiments of the present invention, the ETBR receptor-related disease includes one or more of stroke, Alzheimer's disease, spinal cord injury, aortic stenosis, or neonatal hypoxic-ischemic encephalopathy;
wherein the stroke includes ischemic stroke and/or hemorrhagic stroke.

The polypeptides identified in this patent are established on the basis of a mechanism study on ETBR agonists, and belong to highly selective ETBR agonists. The half-lives of the polypeptides developed in this patent are prolonged by means of studies on structure-activity relationships and structural modification, increasing *in vivo* exposure and reducing the frequency of administration. The polypeptides have lower side effects than those of IRL-1620 and a larger therapeutic window in an animal experiment. The polypeptides in this invention can be used for treating an ETBR receptor-related disease, such as for the rehabilitation of a patient with ischemic stroke at the recovery stage, and to promote improved recovery of the patient with stroke.

### BRIEF DESCRIPTION OF THE DRAWINGS

In order that the technical solution of the Examples of the present invention or of the prior art may be described more clearly, a brief description of the accompanying drawings required for use in the description of the Examples or the prior art will be made below.
FIG.1 shows an EC50 assay of the activity of the polypeptides in Example 3 on a human ETB receptor; wherein A shows the EC50 of BX-15; B shows the EC50 of BX-6; C shows the EC50 of BX-10; D shows the EC50 of BX-21; E shows the EC50 of BX-22; and F shows the EC50 of BX-5.
FIG.2 shows an EC50 assay of the activity of the polypeptides in Example 3 on a human ETB receptor; wherein A shows the EC50 of BX-9; B shows the EC50 of BX-8; C shows the EC50 of BX-7; D shows the EC50 of F-BX-5; E shows the EC50 of BX-13-2; and F shows the EC50 of BX-31.
FIG.3 shows an EC50 assay of the activity of the polypeptides in Example 3 on a human ETB receptor; wherein A shows the EC50 of BX-32; B shows the EC50 of BX-38; C shows the EC50 of BX-43; D shows the EC50 of BX-45; E shows the EC50 of QP-1; and F shows the EC50 of QP-3.
FIG.4 shows an EC50 assay of the activity of the polypeptides in Example 3 on a human ETB receptor; wherein A shows the EC50 of BX-24; B shows the EC50 of BX-53; C shows the EC50 of BX-54; D shows the EC50 of BX-58; E shows the EC50 of FBX-8; and F shows the EC50 of BX-34.
FIG.5 shows an EC50 assay of the activity of the polypeptides in Example 3 on a human ETB receptor; wherein A shows the EC50 of BX-48; B shows the EC50 of BX-49; and C shows the EC50 of BX-50.
FIG.6 shows an EC50 assay of the activity of the polypeptides in Example 3 on a human ETB receptor; wherein A shows the EC50 of BX-69 and its assay results; B shows the EC50 of BX-70 and its assay results; and C shows the EC50 of BX-71 and its assay results.
FIG.7 shows an EC50 assay of the activity of the polypeptides in Example 3 on a human ETB receptor; wherein A shows the EC50 of BX-76 and its assay results; B shows the EC50 of BX-95 and its assay results; and C shows the EC50 of BX-99 and its assay results.
FIG.8 shows an EC50 assay of the activity of the polypeptides in Example 6 on a dog ETB receptor; wherein A shows the EC50 of Dog-BX-34 and its assay results; B shows the EC50 of Dog-BX-76 and its assay results; and C shows the EC50 of Dog-BX-74 and its assay results.
FIG.9 shows an EC50 assay of the activity of the polypeptides in Example 6 on a dog ETB receptor; wherein A shows the EC50 of Dog-BX-99 and its assay results; B shows the EC50 of Dog-BX-86 and its assay results; and C shows the EC50 of Dog-BX-69 and its assay results.
FIG.10 shows an EC50 assay of the activity of the polypeptides in Example 6 on a dog ETB receptor; wherein A shows the EC50 of Dog-BX-70 and its assay results; B shows the EC50 of Dog-BX-71 and its assay results; and C shows the EC50 of Dog-BX-95 and its assay results.
FIG.11 shows an EC50 assay of the activity of the polypeptides in Example 4 on a rat ETB receptor; wherein A shows the EC50 of Rat-BX-34 and its assay results; B shows the EC50 of Rat-F-BX-5 and its assay results; and C shows the EC50 of Rat-BX-69 and its assay results.
FIG.12 shows an EC50 assay of the activity of the polypeptides in Example 4 on a rat ETB receptor; wherein A shows the EC50 of Rat-BX-99 and its assay results; B shows the EC50 of Rat-BX-71 and its assay results; and C shows the EC50 of Rat-BX-95 and its assay results.
FIG.13 shows an EC50 assay of the activity of the polypeptides in Example 4 on a rat ETB receptor; wherein A shows the EC50 of Rat-BX-74 and its assay results; and B shows the EC50 of Rat-BX-76 and its assay results.
FIG.14 shows an EC50 assay of the activity of the polypeptides in Example 5 on a mouse ETB receptor; wherein A shows the EC50 of Mouse-BX-34 and its assay results; B shows the EC50 of Mouse-BX-69 and its assay results; and C shows the EC50 of Mouse-BX-70 and its assay results.
FIG.15 shows an EC50 assay of the activity of the polypeptides in Example 5 on a mouse ETB receptor; wherein A shows the EC50 of Mouse-BX-71 and its assay results; B shows the EC50 of Mouse-BX-95 and its assay results; and C shows the EC50 of Mouse-F-BX-5 and its assay results.
FIG.16 shows the effect of polypeptide BX34 in Example 7 on the amount of cerebral blood flow perfusion in a mouse model of dMACO.

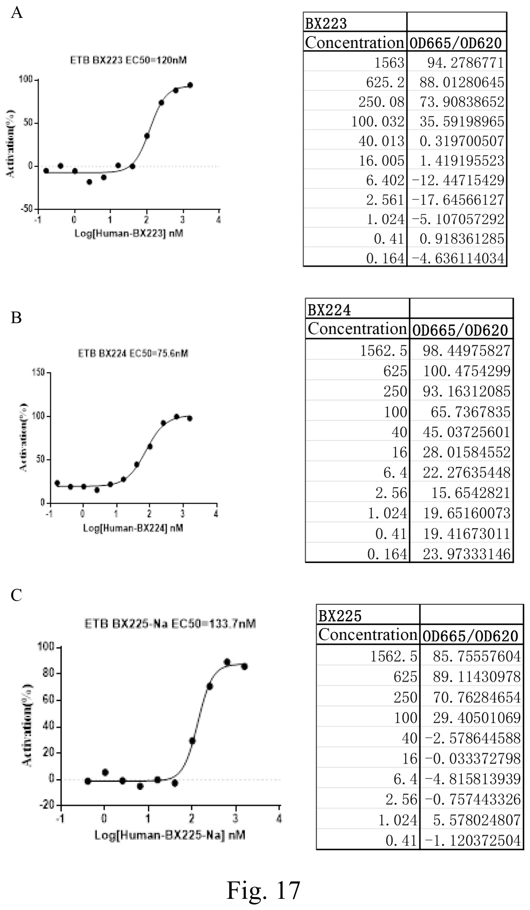

FIG.17 shows an EC50 assay of the activity of the polypeptides in Example 3 on a human ETB receptor; wherein A shows the EC50 of BX-223; B shows the EC50 of BX-224; and C shows the EC50 of BX-225-Na.

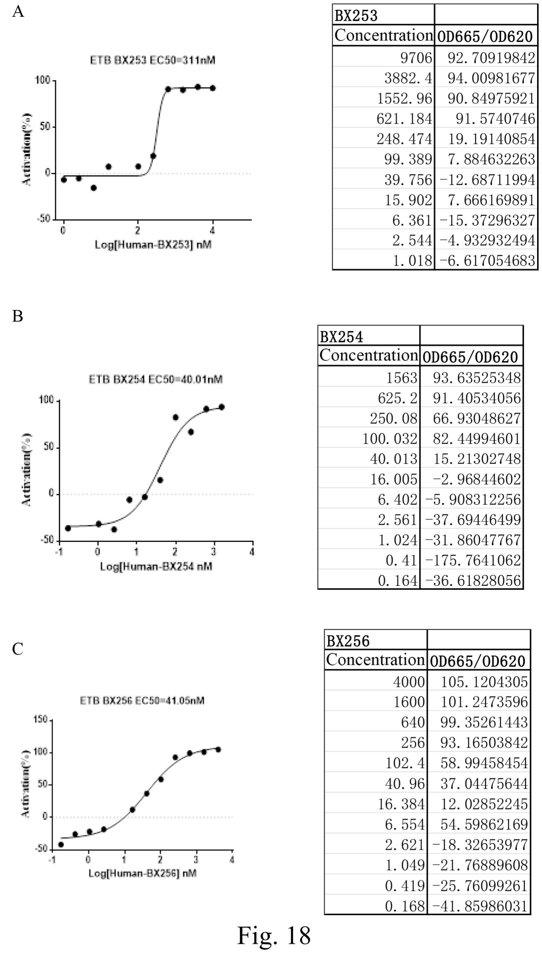

FIG.18 shows an EC50 assay of the activity of the polypeptides in Example 3 on a human ETB receptor; wherein A shows the EC50 of BX-253; B shows the EC50 of BX-254; and C shows the EC50 of BX-256.

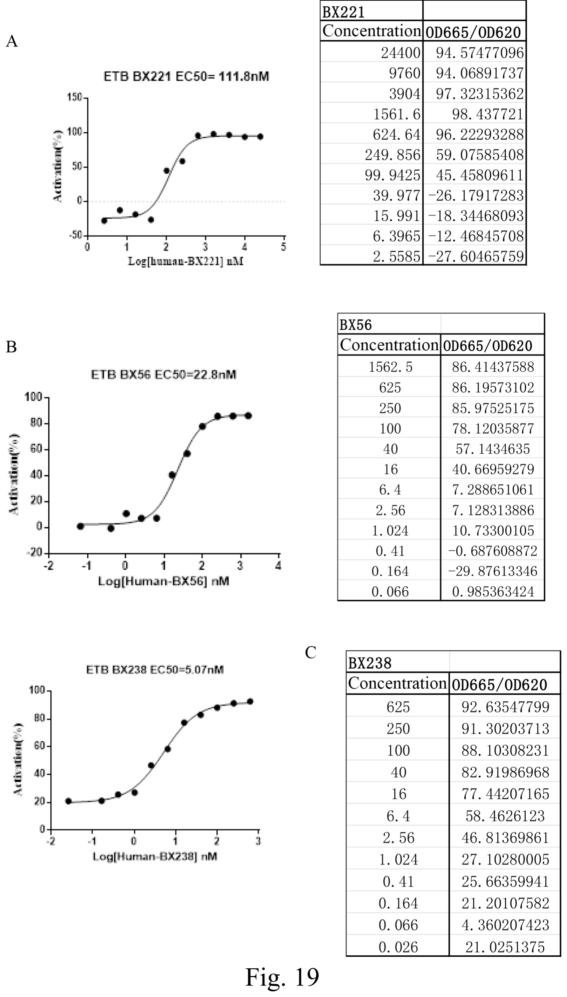

FIG.19 shows an EC50 assay of the activity of the polypeptides in Example 3 on a human ETB receptor; wherein A shows the EC50 of BX-221; B shows the EC50 of BX-56; and C shows the EC50 of BX-238.

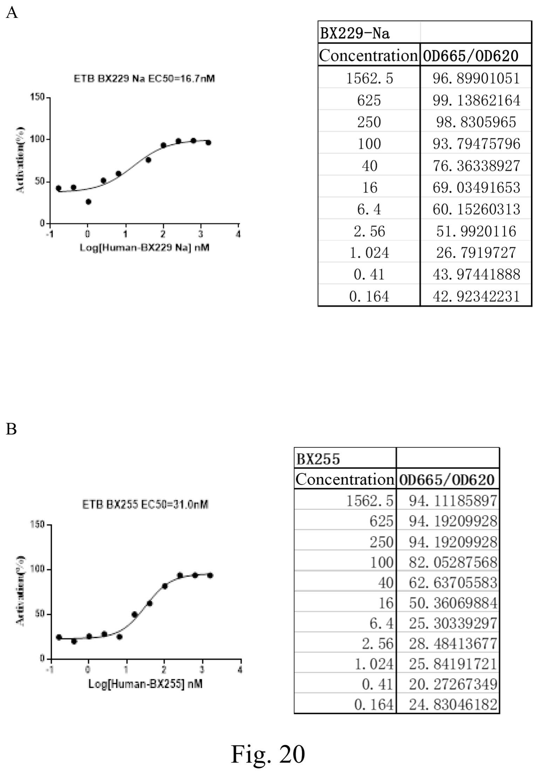

FIG.20 shows an EC50 assay of the activity of the polypeptides in Example 3 on a human ETB receptor; wherein A shows the EC50 of BX-229; and B shows the EC50 of BX-255.

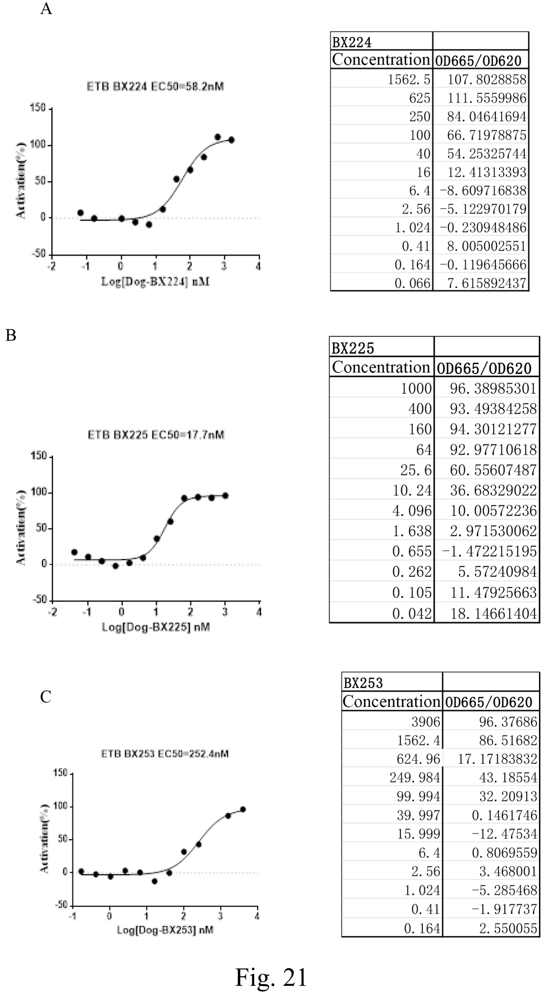

FIG.21 shows an EC50 assay of the activity of the polypeptides in Example 6 on a dog ETB receptor; wherein A shows the EC50 of Dog-BX-224 and its assay results; B shows the EC50 of Dog-BX-225 and its assay results; and C shows the EC50 of Dog-BX-253 and its assay results.

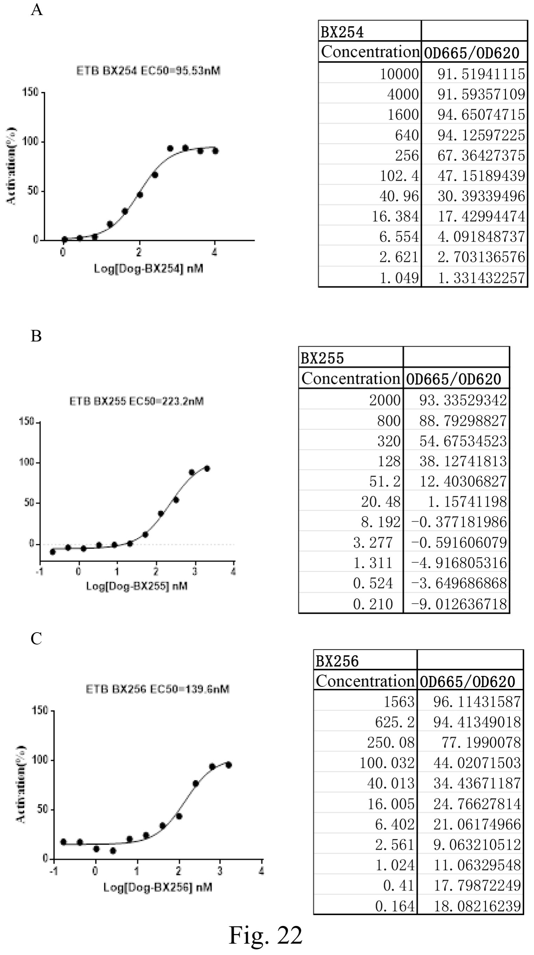

FIG.22 shows an EC50 assay of the activity of the polypeptides in Example 6 on a dog ETB receptor; wherein A shows the EC50 of Dog-BX-254 and its assay results; B shows the EC50 of Dog-BX-255 and its assay results; and C shows the EC50 of Dog-BX-256 and its assay results.

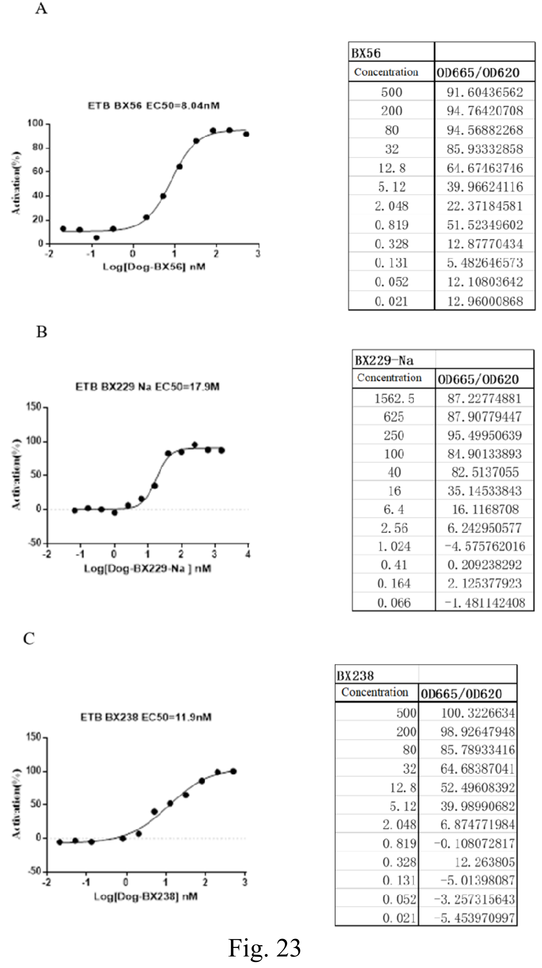

FIG.23 shows an EC50 assay of the activity of the polypeptides in Example 6 on a dog ETB receptor; wherein A shows the EC50 of Dog-BX-56 and its assay results; B shows the EC50 of Dog-BX-229-Na and its assay results; and C shows the EC50 of Dog-BX-238 and its assay results.

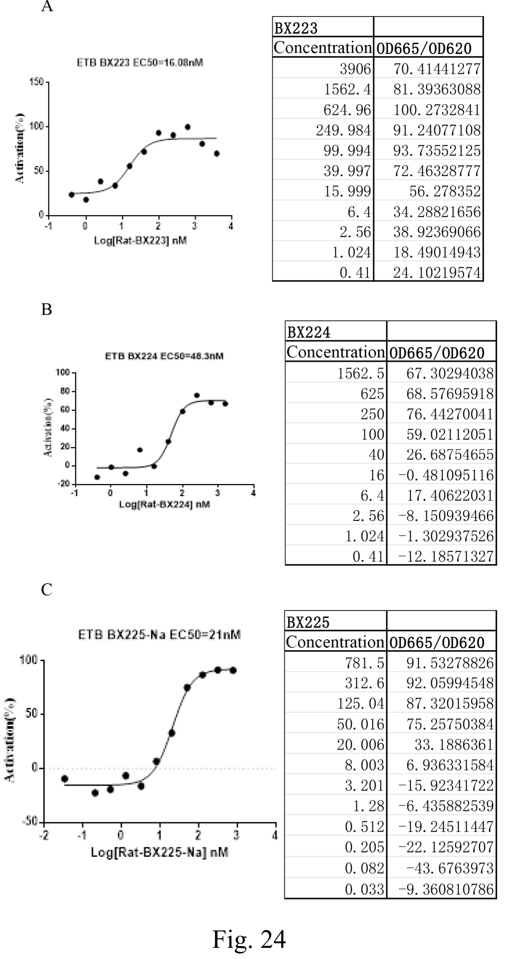

FIG.24 shows an EC50 assay of the activity of the polypeptides in Example 4 on a rat ETB receptor; wherein A shows the EC50 of Rat-BX-223 and its assay results; B shows the EC50 of Rat-BX-224 and its assay results; and C shows the EC50 of Rat-BX-225 and its assay results.

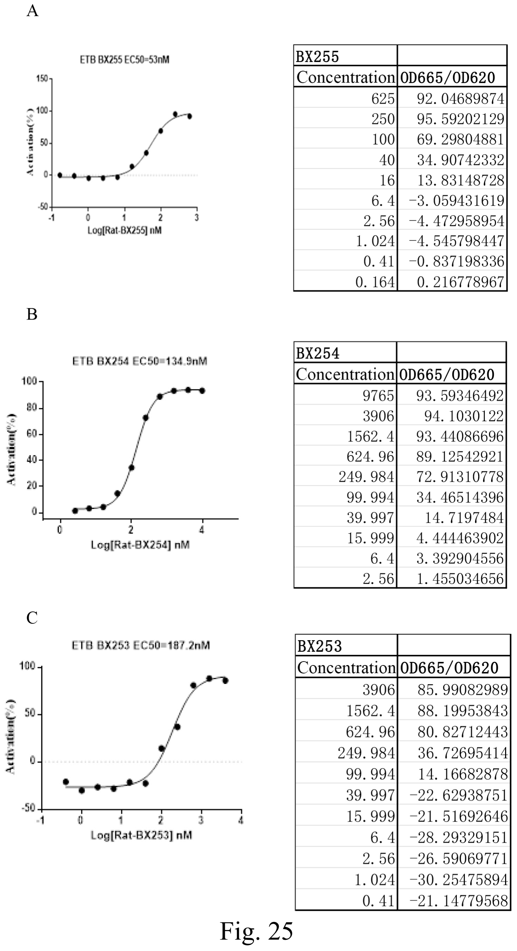

FIG.25 shows an EC50 assay of the activity of the polypeptides in Example 4 on a rat ETB receptor; wherein A shows the EC50 of Rat-BX-255 and its assay results; B shows the EC50 of Rat-BX-254 and its assay results; and C shows the EC50 of Rat-BX-253 and its assay results.

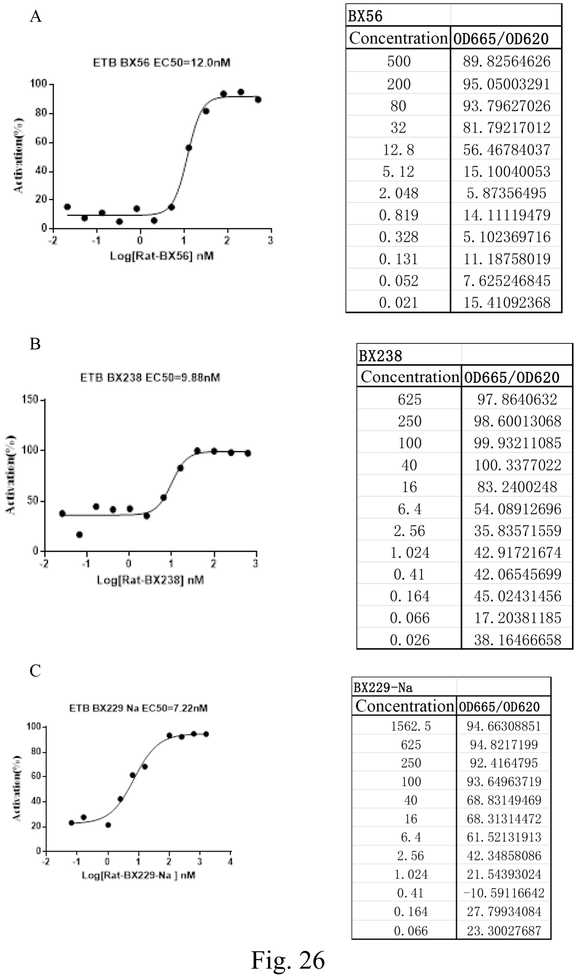

FIG.26 shows an EC50 assay of the activity of the polypeptides in Example 4 on a rat ETB receptor; wherein A shows the EC50 of Rat-BX-56 and its assay results; B shows the EC50 of Rat-BX-238 and its assay results; and C shows the EC50 of Rat-BX-229 and its assay results.

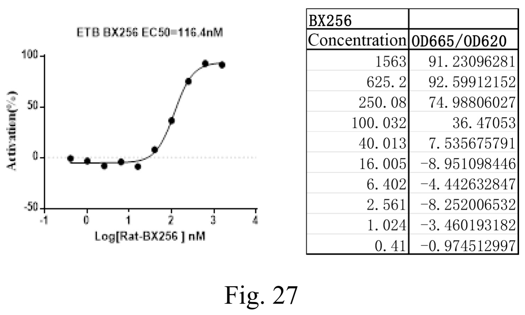

FIG.27 shows an EC50 assay of the activity of the polypeptides in Example 4 on a rat ETB receptor, and specifically, shows the EC50 of Rat-BX-256 and its assay results.

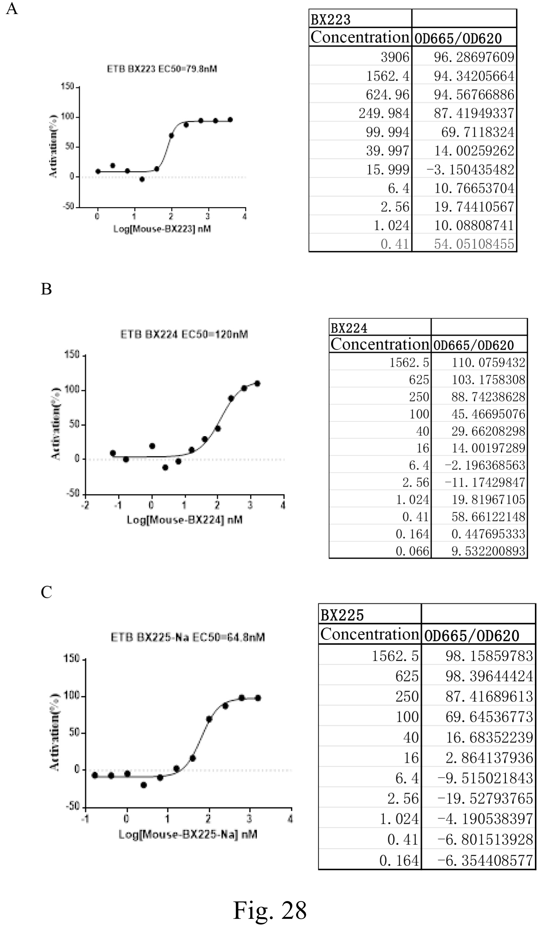

FIG.28 shows an EC50 assay of the activity of the polypeptides in Example 5 on a mouse ETB receptor; wherein A shows the EC50 of Mouse-BX-223 and its assay results; B shows the EC50 of Mouse-BX-224 and its assay results; and C shows the EC50 of Mouse-BX-225 and its assay results.

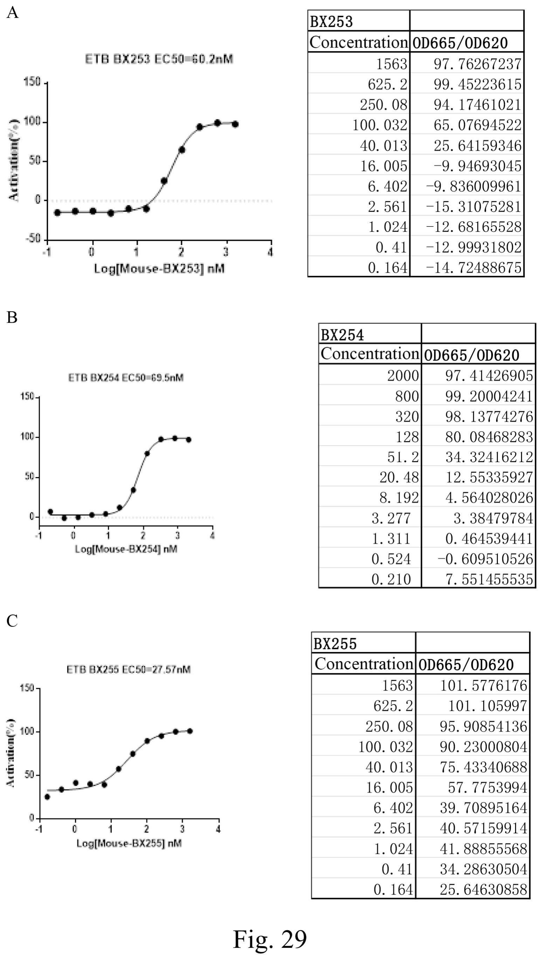

FIG.29 shows an EC50 assay of the activity of the polypeptides in Example 5 on a mouse ETB receptor; wherein A shows the EC50 of Mouse-BX-253 and its assay results; B shows the EC50 of Mouse-BX-254 and its assay results; and C shows the EC50 of Mouse-BX-255 and its assay results.

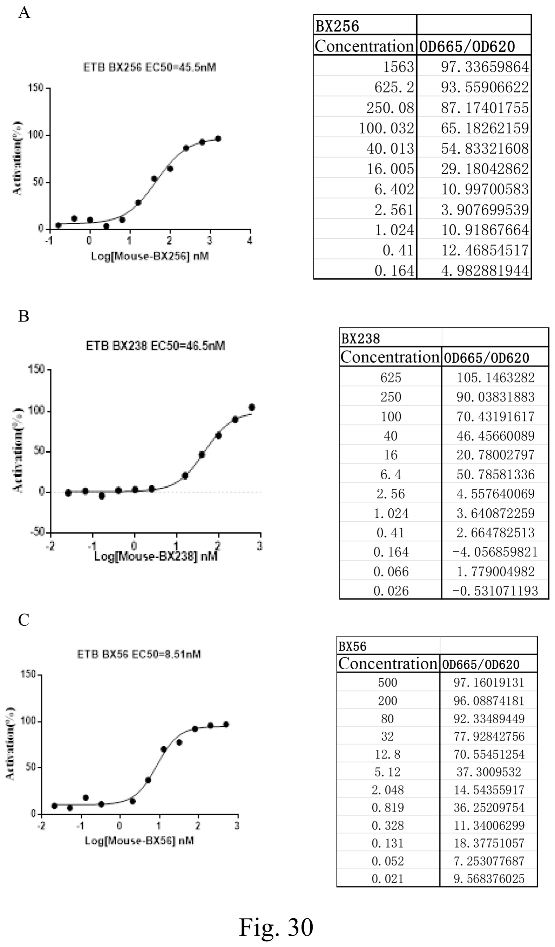

FIG.30 shows an EC50 assay of the activity of the polypeptides in Example 5 on a mouse ETB receptor; wherein A shows the EC50 of Mouse-BX-256 and its assay results; B shows the EC50 of Mouse-BX-238 and its assay results; and C shows the EC50 of Mouse-BX-56 and its assay results.
FIG.31 shows an EC50 assay of the activity of the polypeptides in Example 5 on a mouse ETB receptor; wherein A shows the EC50 of Mouse-BX-229 and its assay results.
FIG.32 shows the Garcia JH score on Day 7 after modeling and administration in Example 13.
FIG.33 shows the Garcia JH score on Day 14 after modeling and administration in Example 13.
FIG.34 shows the change in VEGF-A protein expression in Example 13.
FIG.35 shows the change in BDNF protein expression in Example 13.

### DETAILED DESCRIPTION

The present invention discloses polypeptides and use thereof. With reference to the content herein, those skilled in the art may implement the present invention by appropriately modifying process parameters. Particularly, it should be noted that all similar alternatives and modifications will be apparent to those skilled in the art, and all of them are considered to be included in the present invention. The methods and use of the present invention have been described in terms of preferred Examples. Apparently, those skilled in the art can make modifications or appropriate changes and combinations to the methods and use described herein without departing from the content, spirit, and scope of the present invention to implement and use the technology of the present invention.

Structures and biological experimental results of certain polypeptides of the present invention are as follows:

**Table 1. Structures and Activities on Human ETB Receptor of Polypeptides**

| Serial Numbe r SEQ ID No: | ID | Sequence | hETBR agonist activity EC50/nM | MW |
|---|---|---|---|---|
| 1 | Control Compound (IRL1620) | Suc-DEEAVYFAHLDIIW | EC50=1.333nM | 1820 |
| 2 | BX-5 | Suc-DEEAVYFAHKDIIW | EC50=25.16nM | 1836 |
| 3 | F-BX-5 | Fum-DEEAVYFAHKDIIW | EC50=7.608nM | 1834 |
| 4 | BX-7 | Suc-DEEAVYFAHLDVIW | EC50=0.8973nM | 1806 |
| 5 | BX-8 | Suc-DEEAVYFAHLD(2-Abu)IW | EC50=1.356nM | 1792 |
| 6 | BX-34 | Fum-DEEAVYFAHK(-AEEA-AEEA-γE-ODDA )DIIW | EC50=18.31nM | 2549 |
| 7 | BX-43 | Suc-DEEAKYFAHLDIIW | EC50=4.774nM | 1850 |
| 8 | BX-45 | Suc-DEEEVYFAHKDIIW | EC50=28.97nM | 1893 |
| 9 | BX-48 | Suc-D cyclo(EEAK)YFAHLDIIW | EC50=1.769nM | 1832 |
| 10 | BX-49 | Suc-D cyclo(EEADab)YFAHLDI IW | EC50=4.439nM | 1804 |
| 11 | BX-50 | Suc-DEE cyclo(EVYFAHK)DIIW | EC50=30.52nM | 1876 |
| 12 | BX-51 | Fum-DEEAVYFAHOrn(-AEEA-AEEA-γE - ODDA)DIIW | 37.60%@100nM | 2535 |
| | | | 47.83%@10nM | |
| 13 | BX-52 | Fum-DEEEVYFAHKDIIW | 121.00%@100nM | 1891 |
| | | | 145.60%@10nM | |
| 14 | BX-53 | Suc-DEEEVYFAHOrnDIIW | EC50=10.74nM | 1880 |
| 15 | BX-54 | Suc-DEEEVYFAHK(-AEEA-AEEA-γE-ODDA)DIIW | EC50=34.05nM | 2610 |
| 16 | BX-57 | Suc-DE cyclo(CEEC)YFAHKDII W | 121.58%@100nM | 1999 |
| | | | 86.20%@10nM | |
| 17 | BX-58 | MeO-Fum-DEEEVYFAHKDIIW | EC50=4.477nM | 1906 |
| 18 | BX-59 | Fum-DEEEVYFAHOrnDIIW | 93.46%@100nM | 1878 |
| | | | 125.64%@10nM | |
| 19 | BX-60 | Fum-DEEEVYFAHK(-AEEA-AEEA-γE-ODDA)DIIW | 4.84%@100nM | 2608 |
| | | | -15.72%@10nM | |
| 20 | BX-61 | Suc-DEEAVYFAHOrn(-AEEA-AEEA-γE - ODDA)DIIW | 40.94%@100nM | 2538 |
| | | | 13.97%@10nM | |
| 21 | BX-62 | Fum-DEE cyclo(EVYFK)HKD(2-Abu)IW | 129.39%@100nM | 1903 |
| | | | 99.31%@10nM | |
| 22 | BX-63 | Suc-D cyclo(EEAK)YFAHLD(2-Abu)IW | 122.93%@100nM | 1804 |
| | | | 90.08%@10nM | |
| 23 | BX-64 | Fum-D cyclo(EEAK)YFAHLD(2-Abu)IW | 132.16%@100nM | 1802 |
| | | | 79.92%@10nM | |
| 24 | BX-65 | MeO-Fum-D cyclo(EEAK)YFAHLD(2-Abu)IW | 99.24%@100nM | 1814 |
| | | | 59.02%@10nM | |
| 25 | BX-66 | Fum-DEEK(-γE-AEEA-AEEA-ODDA)VYFAHLDIIW | 60.51%@100nM | 2592 |
| | | | 7.67%@10nM | |
| 26 | BX-67 | Fum-DEEAK(-γE-AEEA-AEEA-ODDA)YFAHLDIIW | 46.98%@100nM | 2564 |
| | | | 2.68%@10nM | |
| 27 | BX-68 | Fum-DEEAVYFK(-γE-AEEA-AEEA-ODDA)HLDIIW | -18.64%@100nM | 2592 |
| | | | -39.42%@10nM | |
| 28 | BX-69 | Fum-DEEAVYFAHK(-γE-AEEA-AEEA-C18)DIIW | T1/2=30min; | 2520 |
| | | | EC50=1153nM | |
| 29 | BX-70 | Fum-DEEAVYFAHK(-γE-AEEA-AEEA-C16)DIIW | T1/2=41min; | 2492 |
| | | | EC50=19.11nM | |
| 30 | BX-71 | Fum-DEEAVYFAHK(-γE-AEEA-AEEA-C12)DIIW | T1/2=23min; | 2436 |
| | | | EC50=74.7nM | |
| 31 | BX-72 | Fum-DEEAVYFAHK(-γE-AEEA-AEEA-B)DIIW | 18.34%@100nM, | 2433 |
| | | | 23.56%@10nM | |
| 32 | BX-74 | Suc-DEEAVYFAHK(-γE-AEEA-AEEA-ODDA )DIIW | 1.82%@100nM | 2552 |
| | | | -14.10%@10nM | |
| 33 | BX-75 | MeO-Fum-DEEAVYFAHK(-γE-AEEA-AEEA-ODDA)DIIW | -10.59%@100nM | 2564 |
| | | | 14.68%@10nM | |
| 34 | BX-76 | Suc-D cyclo(EEAK)YFAHLDVIW | EC50=20.00nM | 1818 |
| 35 | BX-77 | Suc-D cyclo(EEAK)YFAHK(-yE-AEEA-AEEA-ODDA)DIIW | 4.69%@100nM | 2563 |
| | | | -16.59%@10nM | |
| 36 | BX-78 | Suc-DEEAVYFAHK(-γE-AEEA-AEEA-ODDA)DVIW | 23.41%@100nM | 2538 |
| | | | -14.2%@10nM | |
| 37 | BX-79 | Suc-D cyclo(EEAK)YFAHK(-yE-AEEA-AEEA-ODDA)DVIW | 19.85%@100nM | 2550 |
| | | | 2.13%@10nM | |
| 38 | BX-86 | Fum-DEEAVYFAHK(-γE-AEEA-AEEA-ODDA)DIIW-NH2 | 70.55%@100nM | 2548 |
| | | | 105.53%@10nM | |
| 39 | BX-95 | Fum-DEEAVYFAHK(CPAK-MPA-)DIIW | EC50=45.6nM | 2661 |
| | | 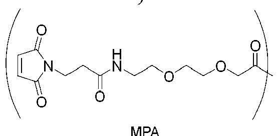 | | |
| 40 | BX-99 | Fum-D cyclo(EEAK)YFAHLDVIW | EC50=33.12nM | 1916 |
| 41 | BX-100 | Fum-D cyclo(EEAK)YFAHK(-γE-AEEA-AEEA-ODDA)DVIW | 15.03%@100nM | 2547 |
| | | | 18.79%@10nM | |
| 42 | BX-101 | Fum-DEEAVYFAHK(-γE-AEEA-AEEA-ODDA)DVIW | 30.96%@100nM, - 5.97%@10nM | 2536 |
| 43 | BX-102 | Fum-D cyclo(EEAK)YFAHLDIIW | 155.47%@100nM, 113.81%@10nM | 1829 |
| 44 | BX-103 | Fum-D cyclo(EEAK)YFAHK(-yE-AEEA-AEEA-ODDA)DIIW | 14.93%@100nM | 2561 |
| | | | 45.51%@10nM | |
| 45 | BX-104 | Fum-D cyclo(EEAK)YFAHK(-yE-AEEA-AEEA-ODDA)D(2-Abu)IW | -21.93%@100nM | 2534 |
| | | | -1299.77%@10nM | |
| 46 | BX-105 | Fum-DEEAKYFAHLDIIW | 253.05%@100nM | 1847 |
| | | | 64.66%@10nM | |
| 47 | BX-106 | Fum-DEEAKYFAHLDVIW | 117.63%@100nM | 1836 |
| | | | 117.57%@10nM | |
| 48 | BX-107 | Fum-DEEAKYFAHK(-γE-AEEA-AEEA-ODDA)DIIW | 100.37%@100nM | 2579 |
| | | | 50.48%@10nM | |
| 49 | BX-108 | Fum-DEEAKYFAHK(-γE-AEEA-AEEA-ODDA)DVIW | 9.56%@100nM | 2567 |
| | | | -1185.11%@10nM | |
| 50 | BX-109 | Fum-DEEAK(-γE-AEEA-AEEA-ODDA)YFAHLDIIW | 74.1%@100nM | 2566 |
| | | | -1095.4%@10nM | |
| 51 | BX-110 | Fum-DEEAK(-γE-AEEA-AEEA-ODDA)YFAHLDVIW | 132.61%@100nM | 2549 |
| | | | 138.99%@10nM | |
| 52 | BX-111 | SA-DEEAVYFAHLDIIW | 64.98%@100nM | 1844 |
| | | | -11.84%@10nM | |
| 53 | BX-112 | MA-DEEAVYFAHLDIIW | 114.05%@100nM | 1844 |
| | | | 466.59%@10nM | |
| 54 | BX-13-2 | Fum-DEEAVYFAHKDIIW1 | EC50=11.76nM | 1843.8 |
| | | 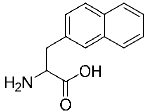 W1: | | |
| | | W1 is termed as 2-amino-3-(naphthalen-2-yl)propanoic acid | | |
| 55 | BX-9 | suc-DEEAVYFAHR1DIIW (R1=L-2-amino-4-guanidinobutyric acid) | EC50=23.18nM | 1849.98 |
| 56 | BX-31 | Fum-DEEAVYFAHOrnDIIW | EC50=56.67nM | 1819 |
| 57 | BX-32 | Fum-DEEAVYFAHCitDIIW | EC50=118.3nM | 1862 |
| 58 | BX-38 | Suc-DE cyclo(CEAC)YFAHLDIIW | EC50=45.71nM | 1927 |
| 59 | BX-23 | Fum-DEEAVYFAHKDVIW | 89.28%@100nM | 1704.9 |
| | | | 26.12%@10nM | |
| 60 | BX-24 | Fum-DEEAVYFAHKD(2-Abu)IW | EC50=8.515nM | 1805.9 |
| 61 | BX-25 | Suc-DEEAVYFAHLDI(N-Me)IW | 28.86%@100nM | 1833.85 |
| | | | 19.19%@10nM | |
| 62 | BX-26 | Suc-DEEAVYFAHL(N-Me)DIIW | 30.58%@100nM | 1833.85 |
| | | | 11.94%@10nM | |
| 63 | BX-27 | Suc-DEEAVYFAHL(N-Me)DI(N-Me)IW | 0.42%@100nM | 1847.86 |
| | | | 1.72%@10nM | |
| 64 | BX-28 | Fum-DEEAVYFAHL(N-Me)DIIW | -10.73%@100nM | 1845.85 |
| | | | 8.27%@10nM | |
| 65 | BX-29 | Fum-DEEAVYFAHL(N-Me)DI(N-Me)IW | 6.51%@100nM | 1847.86 |
| | | | 15.61%@10nM | |
| 66 | FBX-7 | Fum-DEEAVYFAHLDVIW | 99.81%@100nM | 1805.9 |
| | | | 91.89%@10nM | |
| 67 | FBX-8 | Fum-DEEAVYFAHLD(2-Abu)IW | EC50=1.766nM | 1790.9 |
| 68 | QP-1 | 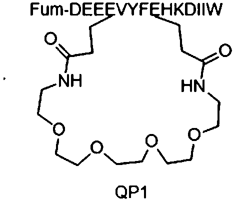 | EC50=9.653nM | 1848 |
| 69 | QP-3 | 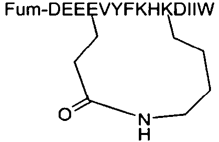 | EC50=3.852nM | 1931 |
| 70 | BX-15 | FA-DEEAVYFAHKDIIW | EC50=41.14 | 1912.87 |
| 71 | BX-16 | Fum-DEEE(Biotin)VYFAHKDI IW | 22.24%@1µM | 2160.4 |
| | | | 14.10%@10µM | |
| 72 | BX-10 | suc-DEEAVYFAHLDPIW | EC50=30.85nM | 1804.9 6 |
| 73 | BX14-1 | Fum-DEE(C16)AVYFAHLDIIW | 15.80 %@1µM | 2044.1 |
| | | | 18.63%@10µM | |
| 74 | BX-6 | Suc-DEEAVYFAHRDIIW | EC50=25.13nM | 1864.0 3 |
| 75 | BX-21 | Fum-DEEALYFAHKDIIW | EC50=11.96nM | 1848 |
| 76 | BX-22 | Fum-DEEAFYFAHKDIIW | EC50=17.43nM | 1882 |
| 77 | BX-223 | Fum-DEEAVYFAHK(-AEEA-γE-C18 )DIIW | EC50=120nM | 2374 |
| 78 | BX-224 | Fum-DEEAVYFAHK(-AEEA-γE-C16 )DIIW | EC50=75.6nM | 2346 |
| 79 | BX-225-Na | Fum-DEEAVYFAHK(-AEEA-γE-C12 )DIIW | EC50=133.7nM | 2290 |
| 80 | BX-253 | Fum-K(-AEEA-AEEA-γE-ODDA )EEAVYFAHLDIIW | EC50=311nM | 2547 |
| 81 | BX-254 | Fum-DK(-AEEA-AEEA-γE-ODDA )EAVYFAHLDIIW | EC50=40.01nM | 2533 |
| 82 | BX-255 | (ODDA-γE-AEEA-AEEA-)DDEEAVYFAHL DIIW | EC50=33.8nM | 2550 |
| 83 | BX-256 | (ODDA-γE-AEEA-AEEA-)NH-C₂H₄-NH-Suc-DEEAVYFAHLDIIW | EC50=41.05nM | 2594 |
| 84 | BX-221 | 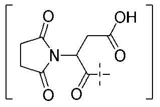 | EC50=111.8nM | 2533 |
| | | EEAVYFAHK(ODDA-γE-AEEA-AEEA-)DIIW X1 and X2 as a whole may 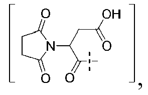 be which is termed as 2-(2,5-dioxopyrrolidin-1-yl)succinic acid | | |
| 86 | BX229-Na | Fum-DEEAVYFAHK(-AEEA-γE-C12)DVIW | EC50=16.7nM | 2276 |
| 87 | BX238 | Fum-DEEAVYFAHK(-AEEA-E-C12)DVIW | EC50=5.07nM | 2276 |
| 88 | BX56 | Suc-DE cyclo(CEEC)YFAHLDIIW | EC50=22.8nM | 1984 |

**Table 6. Structures and Activities on Human ETA Receptor of Certain Polypeptides**

| Serial Number | ID | Sequence | hETAR agonist activity | MW |
|---|---|---|---|---|
| 1 | BX-34 | Fum-DEEAVYFAHK(-AEEA-AEEA-γE-ODDA )DIIW | -23.52%@10nM | 2549 |
| | | | -10.97%@100nM | |
| 2 | BX-43 | Suc-DEEAKYFAHLDIIW | 17.01%@10µM | 1850 |
| | | | 13.51%@1µM | |
| 3 | BX-45 | Suc-DEEEVYFAHKDIIW | 14.69%@1µM | 1893 |
| 4 | BX-48 | Suc-D cyclo(EEAK)YFAHLDIIW | 9.37%@100µM | 1832 |
| | | | 4.45%@10µM | |
| 5 | BX-49 | Suc-D cyclo(EEADab)YFAHLDIIW | -15.45%@100nM | 1804 |
| | | | -20.86%@10nM | |
| 6 | BX-50 | Suc-DEE cyclo(EVYFAHK)DIIW | -6.92%@100nM | 1876 |
| | | | 10.07%@10nM | |
| 7 | BX-52 | Fum-DEEEVYFAHKDIIW | -11.24%@100nM | 1891 |
| | | | -2.49%@10nM | |
| 8 | BX-53 | Suc-DEEEVYFAHOmDIIW | -17.02%@100nM | 1880 |
| | | | -17.65%@10nM | |
| 9 | BX-54 | Suc-DEEEVYFAHK(-AEEA-AEEA-γE-ODDA)DIIW | -8.66%@100uM | 2610 |
| | | | -18.46%@10µM | |
| 10 | BX-57 | Suc-DE cyclo(CEEC)YFAHKDIIW | -10.53%@100µM | 1999 |
| | | | -29.04%@10µM | |
| 11 | BX-58 | MeO-Fum-DEEEVYFAHKDIIW | -2.01%@100µM | 1906 |
| | | | -5.95%@10µM | |
| 12 | BX-59 | Fum-DEEEVYFAHOrnDIIW | -7.67%@100µM | 1878 |
| | | | -10.61%@10µM | |
| 13 | BX-62 | Fum-DEE cyclo(EVYFK)HKD(2-Abu)IW | -19.09%@100nM | 1903 |
| | | | -17.37%@10nM | |
| 14 | BX-63 | Suc-D cyclo(EEAK)YFAHLD(2-Abu)IW | 4.43%@100µM | 1804 |
| | | | -15.24%@10µM | |
| 15 | BX-64 | Fum-D cyclo(EEAK)YFAHLD(2-Abu)IW | 6.41%@100µM | 1802 |
| | | | -13.52%@10µM | |
| 16 | BX-66 | Fum-DEEK(-γE-AEEA-AEEA-ODDA)VYFAHLDIIW | -0.94%@100µM | 2592 |
| | | | 13.13%@10µM | |
| 17 | BX-69 | Fum-DEEAVYFAHK(-γE-AEEA-AEEA-C18)DIIW | 11.53%@100µM | 2520 |
| | | | 13.06%@10µM | |
| 18 | BX-70 | Fum-DEEAVYFAHK(-γE-AEEA-AEEA-C16)DIIW | 13.53%@100µM | 2492 |
| | | | -15.81%@10µM | |
| 19 | BX-71 | Fum-DEEAVYFAHK(-γE-AEEA-AEEA-C12)DIIW | -9.49%@100µM | 2436 |
| | | | -9.66%@10µM | |
| 20 | BX-74 | Suc-DEEAVYFAHK(-γE-AEEA-AEEA-ODDA )DIIW | -13.51%@100µM | 2552 |
| | | | -2.64%@10µM | |
| 21 | BX-76 | Suc-D cyclo(EEAK)YFAHLDVIW | -20.87%@10µM | 1818 |
| | | | -7.56%@100µM | |
| 22 | BX-77 | Suc-D cyclo(EEAK)YFAHK(-yE-AEEA-AEEA-ODDA)DIIW | -6.32%@100µM | 2563 |
| | | | -29.53%@10µM | |
| 23 | BX-78 | Suc-DEEAVYFAHK(-γE-AEEA-AEEA-ODDA)DVIW | -23.02%@100µM | 2538 |
| | | | -6.06%@10µM | |
| 24 | BX-79 | Suc-D cyclo(EEAK)YFAHK(-yE-AEEA-AEEA-ODDA)DVIW | -46.08%@10µM | 2550 |
| | | | -12.17%@100µM | |
| 25 | BX-86 | Fum-DEEAVYFAHK(-γE-AEEA-AEEA-ODDA)DIIW-NH2 | 10.42%@100µM | 2549 |
| | | | 18.26%@10µM | |
| 26 | BX-95 | Fum-DEEAVYFAHK(CPAK-MPA-)DIIW | -7.46%@100µM | 2661 |
| | | 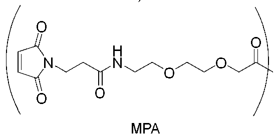 | -9.81%@10µM | |
| 27 | BX-100 | Fum-D cyclo(EEAK)YFAHK(-γE-AEEA-AEEA-ODDA)DVIW | 9.52%@100µM | 2547 |
| | | | -39.09%@10µM | |
| 28 | BX-229-Na | Fum-DEEAVYFAHK(-AEEA-γE-C12 )DVIW | 0.68%@100µM | 2276 |
| 29 | BX-238 | Fum-DEEAVYFAHK(-AEEA-E-C12)DVIW | -5.07%@100µM | 2276 |
| | | | -4.74%@10µM | |
| 30 | BX-225 | Fum-DEEAVYFAHK(-AEEA-γE-C12 )DIIW | 3.59%@100µM | 2290 |
| | | | 0.52%@10µM | |
| 31 | BX-253 | Fum-K(-AEEA-AEEA-γE-ODDA )EEAVYFAHLDIIW | -9.03%@100µM | 2547 |
| | | | -12.86%@10µM | |

**Table 7. Structures and Activities on Dog ETB Receptor of Certain Polypeptides**

| Serial Number | ID | Sequence | DETAR Agonist Activity | MW |
|---|---|---|---|---|
| 1 | BX-34 | Fum-DEEAVYFAHK(-AEEA-AEEA-γE-ODDA )DIIW | EC50=312.7 nM | 2549 |
| 3 | BX-52 | Fum-DEEEVYFAHKDIIW | 104.49%@1 00nM | 1891 |
| | | | 76.82%@10 nM | |
| 4 | BX-57 | Suc-DE cyclo(CEEC)YFAHKDIIW | 49.81%@10 0nM - 20.49%@10 nM | 1999 |
| 5 | BX-59 | Fum-DEEEVYFAHOrnDIIW | 117.46%@1 00nM | 1878 |
| | | | 14.7%@10n M | |
| 6 | BX-69 | Fum-DEEAVYFAHK(-γE-AEEA-AEEA-C18)DIIW | EC50=30.66 nM | 2520 |
| 7 | BX-70 | Fum-DEEAVYFAHK(-γE-AEEA-AEEA-C 16)DIIW | EC50=64.44 nM | 2492 |
| 8 | BX-71 | Fum-DEEAVYFAHK(-γE-AEEA-AEEA-C 12)DIIW | EC50=63.33 nM | 2436 |
| 9 | BX-74 | Suc-DEEAVYFAHK(-yE-AEEA-AEEA- ODDA)DIIW | EC50=519.2 nM | 2552 |
| 10 | BX-76 | Suc-D cyclo(EEAK)YFAHLDVIW | EC50=30.55 nM | 1818 |
| 11 | BX-86 | Fum-DEEAVYFAHK(-γE-AEEA-AEEA-ODDA)DIIW-NH₂ | EC50=411.4 nM | 2548 |
| 12 | BX-95 | Fum-DEEAVYFAHK(CPAK-MPA-)DIIW | EC50=69.1n M | 2661 |
| | | 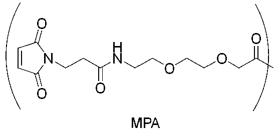 | | |
| 13 | BX-99 | Fum-D cyclo(EEAK)YFAHLDVIW | EC50=17.22 nM | 2547 |
| 14 | BX-56 | Suc-DE cyclo(CEEC)YFAHLDIIW | EC50=8.04n M | 1984 |
| 15 | BX238 | Fum-DEEAVYFAHK(-AEEA-E-C12)DVIW | EC50=11.9n M | 2276 |
| 16 | BX229-Na | Fum-DEEAVYFAHK(-AEEA-γE-C12)DVIW | EC50=17.9n M | 2276 |
| 17 | BX-224 | Fum-DEEAVYFAHK(-AEEA-γE-C16)DIIW | EC50=58.2n M | 2346 |
| 18 | BX-225 | Fum-DEEAVYFAHK(-AEEA-γE-C12)DIIW | EC50=17.7n M | 2290 |
| 19 | BX-253 | Fum-K(-AEEA-AEEA-γE-ODDA)EEAVYFAHLDIIW | EC50=252.4 nM | 2547 |
| 20 | BX-254 | Fum-DK(-AEEA-AEEA-γE-ODDA)EAVYFAHLDIIW | EC50=95.53 nM | 2533 |
| 21 | BX-255 | (ODDA-γE-AEEA-AEEA-)DDEEAVYFAHLDIIW | EC50=223.2 nM | 2550 |
| 22 | BX-256 | (ODDA-γE-AEEA-AEEA-)NH-C₂H₄-NH-Suc-DEEAVYFAHLDIIW | EC50=139.6 nM | 2594 |

**Table 8. Structures and Activities on Rat ETB Receptor of Certain Polypeptides**

| Serial Number | ID | Sequence | DETAR Agonist Activity | MW |
|---|---|---|---|---|
| 1 | BX-34 | Fum-DEEAVYFAHK(-AEEA-AEEA-γE -ODDA)DIIW | EC50=10.2nM | 2549 |
| 2 | F-BX-5 | Fum-DEEAVYFAHKDIIW | EC50=924.3n M | 1834 |
| 3 | BX-52 | Fum-DEEEVYFAHKDIIW | 389.09%@100 nM | 1891 |
| | | | 22.36%@10n M | |
| 4 | BX-57 | Suc-DE cyclo(CEEC)YFAHKDIIW | 218.34%@100 nM 67.42%@10n M | 1999 |
| 5 | BX-59 | Fum-DEEEVYFAHOrnDIIW | 46.94%@100n M | 1878 |
| | | | 314.15%@10n M | |
| 6 | BX-69 | Fum-DEEAVYFAHK(-yE-AEEA-AEEA-C 18)DIIW | EC50=33.23n M | 2520 |
| 7 | BX-70 | Fum-DEEAVYFAHK(-γE-AEEA-AEEA-C16)DIIW | 101.29%@100 nM | 2492 |
| | | | 55.75%@10n M | |
| 8 | BX-71 | Fum-DEEAVYFAHK(-γE-AEEA-AEEA-C12)DIIW | EC50=22.2nM | 2436 |
| 9 | BX-74 | Suc-DEEAVYFAHK(-γE-AEEA-AEEA-ODDA )DIIW | EC50=129.5n M | 2552 |
| 10 | BX-76 | Suc-D cyclo(EEAK)YFAHLDVIW | EC50=505.7n M | 1818 |
| 12 | BX-95 | Fum-DEEAVYFAHK(CPAK-MPA-)DIIW | EC50=23.98n M | 2661 |
| | | 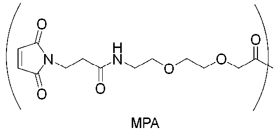 | | |
| 13 | BX-99 | Fum-D cyclo(EEAK)YFAHLDVIW | 19.00nM | 2547 |
| 14 | BX-56 | Suc-DE cyclo(CEEC)YFAHLDIIW | 12nM | 1984 |
| 15 | BX238 | Fum-DEEAVYFAHK(-AEEA-E-C12)DVIW | EC50=9.88nM | 2276 |
| 16 | BX229-Na | Fum-DEEAVYFAHK(-AEEA-γE-C12)DVIW | EC50=7.22nM | 2276 |
| 17 | BX-223 | Fum-DEEAVYFAHK(-AEEA-γE-C18)DIIW | EC50=16.08n M | 2374 |
| 18 | BX-224 | Fum-DEEAVYFAHK(-AEEA-γE-C16)DIIW | EC50=48.3nM | 2346 |
| 19 | BX-225 | Fum-DEEAVYFAHK(-AEEA-γE-C12)DIIW | EC50=21nM | 2290 |
| 20 | BX-253 | Fum-K(-AEEA-AEEA-γE-ODDA)EEAVYFAHLDIIW | EC50=187.2n M | 2547 |
| 21 | BX-254 | Fum-DK(-AEEA-AEEA-γE-ODDA )EAVYFAHLDIIW | EC50=134.9n M | 2533 |
| 22 | BX-255 | (ODDA-γE-AEEA-AEEA-)DDEEAVYFAHLDIIW | EC50=53nM | 2550 |
| 23 | BX-256 | (ODDA-γE-AEEA-AEEA-)NH-C₂H₄-NH-Suc-DEEAVYFAHLDIIW | EC50=116.4n M | 2594 |

**Table 9. Structures and Activities on Mouse ETB Receptor of Certain Polypeptides**

| Serial Number | ID | Sequence | DETAR Agonist Activity | MW |
|---|---|---|---|---|
| 1 | BX-34 | Fum-DEEAVYFAHK(-AEEA-AEEA-γE -ODDA)DIIW | EC50=71.7nM | 2549 |
| 2 | F-BX-5 | Fum-DEEAVYFAHKDIIW | EC50=12.88n M | 1834 |
| 3 | BX-69 | Fum-DEEAVYFAHK(-γE-AEEA-AEEA-C18)DIIW | EC50=71.48n M | 2520 |
| 4 | BX-70 | Fum-DEEAVYFAHK(-γE-AEEA-AEEA-C16)DIIW | EC50=31.21n M | 2492 |
| 5 | BX-71 | Fum-DEEAVYFAHK(-γE-AEEA-AEEA-C12)DIIW | EC50=4.175n M | 2436 |
| 6 | BX-95 | Fum-DEEAVYFAHK(CPAK-MPA-)DIIW | EC50=22.13n M | 2661 |
| | | 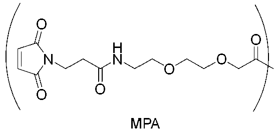 | | |
| 7 | BX56 | Suc-DE cyclo(CEEC)YFAHLDIIW | EC50=8.51nM | 1984 |
| 8 | BX238 | Fum-DEEAVYFAHK(-AEEA-E-C12)DVIW | EC50=46.5nM | 2276 |
| 9 | BX229-Na | Fum-DEEAVYFAHK(-AEEA-γE-C12)DVIW | EC50=9.05nM | 2276 |
| 10 | BX-223 | Fum-DEEAVYFAHK(-AEEA-γE-C18)DIIW | EC50=79.8nM | 2374 |
| 11 | BX-224 | Fum-DEEAVYFAHK(-AEEA-γE-C16)DIIW | EC50=120nM | 2346 |
| 12 | BX-225 | Fum-DEEAVYFAHK(-AEEA-γE-C12)DIIW | EC50=64.8nM | 2290 |
| 13 | BX-253 | Fum-K(-AEEA-AEEA-γE-ODDA)EEAVYFAHLDIIW | EC50=60.2nM | 2547 |
| 14 | BX-254 | Fum-DK(-AEEA-AEEA-γE-ODDA)EAVYFAHLDIIW | EC50=69.5nM | 2533 |
| 15 | BX-255 | (ODDA-γE-AEEA-AEEA-)DDEEAVYFAHLDIIW | EC50=27.57n M | 2550 |
| 16 | BX-256 | (ODDA-γE-AEEA-AEEA-)NH-C₂H₄-NH-Suc-DEEAVYFAHLDIIW | EC50=45.5nM | 2594 |

**Table 10. Chinese Interpretation of Abbreviations or Full Names in English**

| Abbreviations or English | Meaning |
|---|---|
| Boc | tert-butoxycarbonyl |
| DCM | dichloromethane |
| DIEA | diisopropylethylamine |
| DMF | N,N-dimethylformamide |
| MeOH | methanol |
| ACN | acetonitrile |
| DIC | diisopropyl carbodiimide |
| HOBt | benzotriazole |
| Fmoc | fluorenylmethyloxycarbonyl |
| HBTU | O-benzotriazole-tetramethyl-uronium-hexafluorophosphate |
| PYBOP | benzotriazol-1-yl-oxytripyrrolidinophosphonium hexafluorophosphate |
| OtBu | -O-C(CH₃)₃ |
| Dde | 1-(4,4-dimethyl-2,6-dioxocyclohexylidene)ethyl |
| Alloc | allyloxycarbonyl |
| Oall | allyl ester |
| Resin | resin |
| Trt | trityl |
| TFA | trifluoroacetic acid |
| TIS | triisopropylsilane |
| Suc | succinyl |
| Fum | fumaryl |
| AEEA | aminoethoxyethoxyacetic acid |
| ODDA | octadecanedioic acid |
| Orn | ornithine |
| Dab | diaminobutyric acid |
| C12 | lauroyl |
| C16 | palmitoyl |
| C18 | stearyl |
| B | borneol formyl |
| SA | Sorbic acid |
| MA | trans,trans-Muconic Acid |
| 2-Abu | 2-aminobutyric acid |
| FA | Ferulic Acid |
| D | Day, D1 represents the first day and D2 represents the second day |
| H | hour |
| Min | minute |
| Saline | saline group |
| Sham | sham group |
| BX-229-Na or BX229Na or BX229-Na | sodium salt form of BX-229 |
| BX34, BX48, BX225, BX229, BX238, BX253, BX254, BX255, BX256, etc. | equivalent to BX-34, BX-48, BX-225, BX-229, BX-238, BX-253, BX-254, BX-255, BX-256, etc., respectively; the dash "-" in the middle of the compound numbering may be omitted |

General description. The dash "-" in the middle of a compound numbering may be omitted, and the dash is omitted in the numbering of part of the experiments. For example, BX229 is equivalent to BX-229. Unless otherwise specified, the polypeptides are in the form of trifluoroacetate. For some compounds, a specific salt form will be labeled after their numberings, such as BX-229-Na and BX229Na, both representing the sodium salt form of BX-229.

**Table 11. Main Reagents and Solvents**

| Numbering | Name of Raw Material | Manufacturer |
|---|---|---|
| 1 | Fmoc-Trp(Boc)-Wang Resin | GL Biochem (Shanghai) Ltd. |
| 2 | Fmoc-Ile-OH | |
| 3 | Fmoc-Asp(OtBu)-OH | |
| 4 | Fmoc-Leu-OH | |
| 5 | Fmoc-His(Trt)-OH | |
| 6 | Fmoc-Ala-OH | |
| 7 | Fmoc-Phe-OH | |
| 8 | Fmoc-Tyr(tBu)-OH | |
| 9 | Fmoc-Val-OH | |
| 10 | Fmoc-Glu(OtBu)-OH | |
| 11 | Fmoc-Glu-OtBu | |
| 12 | Fmoc-Glu(OAll)-OH | |
| 13 | Fmoc-Dab(Boc)-OH | |
| 14 | Fmoc-Om(Boc)-OH | |
| 15 | Fmoc-Lys(Boc)-OH | |
| 16 | Fmoc-Lys(Dde)-OH | |
| 17 | Fmoc-Lys(Alloc)-OH | |
| 18 | Fmoc-Cys(Trt)-OH | |
| 19 | OtBu-Suc | |
| 20 | OtBu-Fum | |
| 21 | Fmoc-AEEA-OH | |
| 22 | OtBu-ODDA | |
| 23 | PCDNA3.1 | Sangon Biotech (Shanghai) Co., Ltd |

The raw materials and reagents utilized in the polypeptides and use thereof provided in the present invention are commercially available unless otherwise specified.

The invention is further illustrated with reference to the following Examples:

### Preparation Example: Synthesis of Compounds in BX-34 Series

### 1. Synthesis of BX-34

0.50 g (0.25 mmol) of Fmoc-Trp(Boc)-Wang Resin was weighed and added into a reactor. 10 mL of DCM was added for swelling for 10 minutes, then filtered by suction, and washed twice with DMF. 25% 4-methylpiperidine/DMF (by volume) was added and allowed to react for 30 minutes to remove the Fmoc group. After filtering by suction, the resin was washed four times with DMF and twice with DCM. A ninhydrin test was performed, and the resin solution was found to be blue. Fmoc-Ile-OH (1 mmol, 4 eq.) and HBTU (1 mmol, 4 eq.) were weighed, dissolved in DMF, and DIEA (2 mmol, 8 eq.) was added and mixed to uniformity. The mixture was added to the resin and allowed to react for 1 hour under magnetic stirring at room temperature (25°C±5°C). After filtering by suction, it was washed sequentially with DMF, MeOH, and DCM, three times each. A ninhydrin test was performed, and the resin was found to be colorless and transparent, while the solution was found to be light yellow, indicating a complete reaction. 25% 4-methylpiperidine/DMF (by volume) was added to remove the Fmoc group. This cycle was repeated to sequentially complete the coupling of Ile, Asp(OtBu), Lys(Dde), His(Trt), Ala, Phe, Tyr(tBu), Val, Ala, Glu(OtBu), Glu(OtBu), Asp(OtBu), and OtBu-Fum. Subsequently, 2% hydrazine hydrate/DMF was added to the peptide resin and allowed to react for 10 minutes×3 times to remove the Dde on the side chain of Lys. AEEA, AEEA, Glu-α-OtBu, and OtBu-ODDA were then condensed sequentially. The peptide resin was dried under suction, and 15 mL of a pre-cooled cleavage solution (TFA: TIS: H₂O = 95:2.5:2.5) was added. The reaction was allowed to proceed for 3 hours at room temperature (25°C±5°C) and under stirring. The reaction solutions were extracted by suction, and the resin was washed twice with a small amount of TFA and then extracted by suction. The reaction solutions were combined, and approximately 200 mL of pre-cooled methyl tert-butyl ether was added. A white precipitate was formed. After centrifuging (5000 rpm×5 min), the supernatant was discarded. Methyl tert-butyl ether was added again to the precipitate. After vortexing and centrifuging, the supernatant was discarded. The precipitate was placed in a vacuum dryer and dried for 12 hours to obtain a crude product. The crude peptide was dissolved in approximately 1% aqueous ammonia, filtered, and the filtrate was purified with preparative liquid phases. Preparative column: C18-10-100, 30×250 mm. Flow rate: 25 mL/min. Phase A: 0.1%TFA/water, and Phase B: 0.1%TFA/90% acetonitrile/water. A gradient elution was performed to obtain a purified target compound. Fractions of the target compound with a purity >95% were combined, and acetonitrile was removed under reduced pressure at around 40°C using a water pump and a rotary evaporator. The resulting concentrated solution was frozen into a solid using liquid nitrogen and then freeze-dried for 48 hours using a freeze-dryer to obtain the target compound. The molecular weight was identified to be correct by mass spectrometry ([M+3]³⁺=850.8).

### 2. Synthesis of BX-5

A purified target compound was obtained according to the method of synthesizing BX-34, and the molecular weight was identified to be correct by mass spectrometry ([M+1]⁺=1837.3).

### 3. Synthesis of F-BX-5

A purified target compound was obtained according to the method of synthesizing BX-34, and the molecular weight was identified to be correct by mass spectrometry ([M+2]²⁺=918).

### 4. Synthesis of BX-7

A purified target compound was obtained according to the method of synthesizing BX-34, and the molecular weight was identified to be correct by mass spectrometry ([M+1]⁺=1807.4).

### 5. Synthesis of BX-8

A purified target compound was obtained according to the method of synthesizing BX-34, and the molecular weight was identified to be correct by mass spectrometry ([M+1]⁺=1792.1).

### 6. Synthesis of BX-43

A purified target compound was obtained according to the method of synthesizing BX-34, and the molecular weight was identified to be correct by mass spectrometry ([M+1]⁺=1851.4).

### 7. Synthesis of BX-45

A purified target compound was obtained according to the method of synthesizing BX-34, and the molecular weight was identified to be correct by mass spectrometry ([M+1]⁺=1893.2).

### 8. Synthesis of BX-51

A purified target compound was obtained according to the method of synthesizing BX-34, and the molecular weight was identified to be correct by mass spectrometry ([M+2]²⁺=1268.5).

### 9. Synthesis of BX-52

A purified target compound was obtained according to the method of synthesizing BX-34, and the molecular weight was identified to be correct by mass spectrometry ([M+2]²⁺=946.9).

### 10. Synthesis of BX-53

A purified target compound was obtained according to the method of synthesizing BX-34, and the molecular weight was identified to be correct by mass spectrometry ([M+1]⁺=1881.5).

### 11. Synthesis of BX-54

A purified target compound was obtained according to the method of synthesizing BX-34, and the molecular weight was identified to be correct by mass spectrometry ([M+2]²⁺=1306.2).

### 12. Synthesis of BX-58

A purified target compound was obtained according to the method of synthesizing BX-34, and the molecular weight was identified to be correct by mass spectrometry ([M+1]⁺=1907.3).

### 13. Synthesis of BX-59

A purified target compound was obtained according to the method of synthesizing BX-34, and the molecular weight was identified to be correct by mass spectrometry ([M+2]²⁺=939.9).

### 14. Synthesis of BX-60

A purified target compound was obtained according to the method of synthesizing BX-34, and the molecular weight was identified to be correct by mass spectrometry ([M+2]²⁺=1305.6).

### 15. Synthesis of BX-61

A purified target compound was obtained according to the method of synthesizing BX-34, and the molecular weight was identified to be correct by mass spectrometry ([M+2]²⁺=1270.4).

### 16. Synthesis of BX-66

A purified target compound was obtained according to the method of synthesizing BX-34, and the molecular weight was identified to be correct by mass spectrometry ([M+2]²⁺=1297.2).

### 17. Synthesis of BX-67

A purified target compound was obtained according to the method of synthesizing BX-34, and the molecular weight was identified to be correct by mass spectrometry ([M+2]²⁺=1283.3).

### 18. Synthesis of BX-68

A purified target compound was obtained according to the method of synthesizing BX-34, and the molecular weight was identified to be correct by mass spectrometry ([M+2]²⁺=1297.6).

### 19. Synthesis of BX-69

A purified target compound was obtained according to the method of synthesizing BX-34, and the molecular weight was identified to be correct by mass spectrometry ([M+3]³⁺=841.1).

### 20. Synthesis of BX-70

A purified target compound was obtained according to the method of synthesizing BX-34, and the molecular weight was identified to be correct by mass spectrometry ([M+3]³⁺=831.7).

### 21. Synthesis of BX-71

A purified target compound was obtained according to the method of synthesizing BX-34, and the molecular weight was identified to be correct by mass spectrometry ([M+3]³⁺=825.8).

### 22. Synthesis of BX-72

A purified target compound was obtained according to the method of synthesizing BX-34, and the molecular weight was identified to be correct by mass spectrometry ([M+3]³⁺=1653.4).

### 23. Synthesis of BX-73

A purified target compound was obtained according to the method of synthesizing BX-34, and the molecular weight was identified to be correct by mass spectrometry ([M+2]²⁺=1066).

### 24. Synthesis of BX-74

A purified target compound was obtained according to the method of synthesizing BX-34, and the molecular weight was identified to be correct by mass spectrometry ([M+3]³⁺=851.6).

### 25. Synthesis of BX-75

A purified target compound was obtained according to the method of synthesizing BX-34, and the molecular weight was identified to be correct by mass spectrometry ([M+3]³⁺=855.7).

### 26. Synthesis of BX-78

A purified target compound was obtained according to the method of synthesizing BX-34, and the molecular weight was identified to be correct by mass spectrometry ([M+3]³⁺=847.1).

### 27. Synthesis of BX-86

A purified target compound was obtained according to the method of synthesizing BX-34, and the molecular weight was identified to be correct by mass spectrometry ([M+3]³⁺=850.8).

### 28. Synthesis of BX-101

A purified target compound was obtained according to the method of synthesizing BX-34, and the molecular weight was identified to be correct by mass spectrometry ([M+3]³⁺=846.3).

### 29. Synthesis of BX-105

A purified target compound was obtained according to the method of synthesizing BX-34, and the molecular weight was identified to be correct by mass spectrometry ([M+1]⁺=1848.2).

### 30. Synthesis of BX-106

A purified target compound was obtained according to the method of synthesizing BX-34, and the molecular weight was identified to be correct by mass spectrometry ([M+1]⁺=1837.3).

### 31. Synthesis of BX-107

A purified target compound was obtained according to the method of synthesizing BX-34, and the molecular weight was identified to be correct by mass spectrometry ([M+3]³⁺=860.7).

### 32. Synthesis of BX-108

A purified target compound was obtained according to the method of synthesizing BX-34, and the molecular weight was identified to be correct by mass spectrometry ([M+3]³⁺=856.7).

### 33. Synthesis of BX-109

A purified target compound was obtained according to the method of synthesizing BX-34, and the molecular weight was identified to be correct by mass spectrometry ([M+3]³⁺=856.3).

### 34. Synthesis of BX-110

A purified target compound was obtained according to the method of synthesizing BX-34, and the molecular weight was identified to be correct by mass spectrometry ([M+3]³⁺=850.7).

### 35. Synthesis of BX-111

A purified target compound was obtained according to the method of synthesizing BX-34, and the molecular weight was identified to be correct by mass spectrometry ([M+1]⁺=1845.5).

### 36. Synthesis of BX-112

A purified target compound was obtained according to the method of synthesizing BX-34, and the molecular weight was identified to be correct by mass spectrometry ([M+1]⁺=1845.3).

### 37. Synthesis of BX-229

0.50 g (0.25 mmol) of Fmoc-Trp(Boc)-Wang Resin was weighed and added into a reactor. 10 mL of DCM was added for swelling for 10 minutes, then filtered by suction, and washed twice with DMF. 25% 4-methylpiperidine/DMF (by volume) was added and allowed to react for 30 minutes to remove the Fmoc group. After filtering by suction, the resin was washed four times with DMF and twice with DCM. A ninhydrin test was performed, and the resin solution was found to be blue. Fmoc-Ile-OH (1 mmol, 4 eq.) and HBTU (1 mmol, 4 eq.) were weighed, dissolved in DMF, and DIEA (2 mmol, 8 eq.) was added and mixed to uniformity. The mixture was added to the resin and allowed to react for 1 hour under magnetic stirring at room temperature (25°C±5°C). After filtering by suction, it was washed sequentially with DMF, MeOH, and DCM, three times each. A ninhydrin test was performed, and the resin was found to be colorless and transparent, while the solution was found to be light yellow, indicating a complete reaction. 25% 4-methylpiperidine/DMF (by volume) was added to remove the Fmoc group. This cycle was repeated to sequentially complete the coupling of Val, Asp(OtBu), Lys(Dde), His(Trt), Ala, Phe, Tyr(tBu), Val, Ala, Glu(OtBu), Glu(OtBu), Asp(OtBu), and OtBu-Fum. Subsequently, 2% hydrazine hydrate/DMF was added to the peptide resin and allowed to react for 10 minutes×3 times to remove the Dde on the side chain of Lys. AEEA, Glu-α-OtBu, and C12-OH were then condensed sequentially. The peptide resin was dried, and 15 mL of a pre-cooled cleavage solution (TFA: TIS: H₂O = 95:2.5:2.5) was added. The reaction was allowed to proceed for 3 hours at room temperature (25°C±5°C) and under stirring. The reaction solution was extracted by suction, and the resin was washed twice with a small amount of TFA and then extracted. The reaction solutions were combined, and approximately 200 mL of pre-cooled methyl tert-butyl ether was added. A white precipitate was formed. After centrifuging (5000 rpm×5 min), the supernatant was discarded. Methyl tert-butyl ether was added again to the precipitate. After vortexing and centrifuging, the supernatant was discarded. The precipitate was placed in a vacuum dryer and dried for 12 hours to obtain a crude product. The crude peptide was dissolved in approximately 1% aqueous ammonia, filtered, and the filtrate was purified with preparative liquid phases. Preparative column: C18-10-100, 30×250 mm. Flow rate: 25 mL/min. Phase A: 0.1%TFA/water, and Phase B: 0.1%TFA/90% acetonitrile/water. A gradient elution was performed to obtain a purified target compound. Fractions of the target compound with a purity >95% were combined, and acetonitrile was removed under reduced pressure at around 40°C using a water pump and a rotary evaporator. The resulting concentrated solution was frozen into a solid using liquid nitrogen and then freeze-dried for 48 hours using a freeze-dryer to obtain a target compound. The molecular weight was identified to be correct by mass spectrometry ([M-2]²⁻=1137.3).

### 38. Preparation of sodium salt of BX-229

The BX-229 solid obtained in the previous step was dissolved in 0.1N of aqueous sodium hydroxide solution (containing 10 equivalents of sodium hydroxide) and converted into a salt using preparative liquid phases. Preparative column: C18-10-100, 30×250 mm. Flow rate: 25mL/min. Phase A: water, and Phase B: acetonitrile. After loading, it was rinsed with water for 15 min, and then the product was eluted from the preparative column by gradient elution. Fractions of the target compound with a purity >95% were combined, and acetonitrile was removed under reduced pressure at around 40°C using a water pump and a rotary evaporator. The resulting concentrated solution was frozen into a solid using liquid nitrogen and then freeze-dried for 48 hours using a freeze-dryer to obtain the target compound.

### 39. Synthesis of BX-238

0.50 g (0.25 mmol) of Fmoc-Trp(Boc)-Wang Resin was weighed and added into a reactor. 10 mL of DCM was added for swelling for 10 minutes, then filtered by suction, and washed twice with DMF. 25% 4-methylpiperidine/DMF (by volume) was added and allowed to react for 30 minutes to remove the Fmoc group. After filtering by suction, the resin was washed four times with DMF and twice with DCM. A ninhydrin test was performed, and the resin solution was found to be blue. Fmoc-Ile-OH (1 mmol, 4 eq.) and HBTU (1 mmol, 4 eq.) were weighed, dissolved in DMF, and DIEA (2 mmol, 8 eq.) was added and mixed to uniformity. The mixture was added to the resin and allowed to react for 1 hour under magnetic stirring at room temperature (25°C±5°C). After filtering by suction, it was washed sequentially with DMF, MeOH, and DCM, three times each. A ninhydrin test was performed, and the resin was found to be colorless and transparent, while the solution was found to be light yellow, indicating a complete reaction. 25% 4-methylpiperidine/DMF (by volume) was added to remove the Fmoc group. This cycle was repeated to sequentially complete the coupling of Val, Asp(OtBu), Lys(Dde), His(Trt), Ala, Phe, Tyr(tBu), Val, Ala, Glu(OtBu), Glu(OtBu), Asp(OtBu), and OtBu-Fum. Subsequently, 2% hydrazine hydrate/DMF was added to the peptide resin and allowed to react for 10 minutes×3 times to remove the Dde on the side chain of the Lys. AEEA, Glu(OtBu), and C12-OH were then condensed sequentially. The peptide resin was dried under suction, and 15 mL of a pre-cooled cleavage solution (TFA: TIS: H₂O = 95:2.5:2.5) was added. The reaction was allowed to proceed for 3 hours at room temperature (25°C±5°C) and under stirring. The reaction solution was extracted by suction, and the resin was washed twice with a small amount of TFA and then extracted by suction. The reaction solutions were combined, and approximately 200 mL of pre-cooled methyl tert-butyl ether was added. A white precipitate was formed. After centrifuging (5000 rpm×5 min), the supernatant was discarded. Methyl tert-butyl ether was added again to the precipitate. After vortexing and centrifuging, the supernatant was discarded. The precipitate was placed in a vacuum dryer and dried for 12 hours to obtain a crude product. The crude peptide was dissolved in approximately 1% aqueous ammonia, filtered, and the filtrate was purified with preparative liquid phases. Preparative column: C18-10-100, 30×250 mm. Flow rate: 25 mL/min. Phase A: 0.1%TFA/water, and Phase B: 0.1%TFA/90% acetonitrile/water. A gradient elution was performed to obtain a purified target compound. Fractions of the target compound with a purity >95% were combined, and acetonitrile was removed under reduced pressure at around 40°C using a water pump and a rotary evaporator. The resulting concentrated solution was frozen into a solid using liquid nitrogen and then freeze-dried for 48 hours using a freeze-dryer to obtain the target compound. The molecular weight was identified to be correct by mass spectrometry ([M-2]²⁻=1136.8).

### 40. Synthesis of BX-76

A backbone was synthesized according to the method of synthesizing BX-34. A DCM solution of 0.3 equivalents of tetrakis(triphenylphosphine)palladium and 12 equivalents of phenylsilane was added to a peptide resin under nitrogen protection, and allowed to react at room temperature (25°C±5°C) for 2 h to remove the allyl ester group on the side chain of Glu and the allyloxycarbonyl group on the side chain of Lys. After filtration by suction, it was washed 5 times with a DCM solvent and 3 times with DMF. 2 equivalents of PYBOP, 2 equivalents of HOBt and 4 equivalents of DIEA were added, dissolved in DMF, and allowed to react at room temperature (25°C±5°C) for 5 h. After filtration by suction, it was washed 3 times with DMF and 3 times with DCM. A ninhydrin test was performed. If the reaction was not complete, the condensation was repeated once. It was then subjected to cleavage and purification according to the method of BX-34, to give a purified target compound. The molecular weight was identified to be correct by mass spectrometry ([M+2]²⁺=910).

### 41. Synthesis of BX-48

A purified target compound was obtained according to the method of synthesizing BX-76, and the molecular weight was identified to be correct by mass spectrometry ([M+2]²⁺=917.5).

### 42. Synthesis of BX-49

A purified target compound was obtained according to the method of synthesizing BX-76, and the molecular weight was identified to be correct by mass spectrometry ([M+2]²⁺=903.2).

### 43. Synthesis of BX-50

A purified target compound was obtained according to the method of synthesizing BX-76, and the molecular weight was identified to be correct by mass spectrometry ([M+2]²⁺=939).

### 44. Synthesis of BX-62

A purified target compound was obtained according to the method of synthesizing BX-76, and the molecular weight was identified to be correct by mass spectrometry ([M+2]²⁺=952.9).

### 45. Synthesis of BX-63

A purified target compound was obtained according to the method of synthesizing BX-76, and the molecular weight was identified to be correct by mass spectrometry ([M+2]²⁺=902.9).

### 46. Synthesis of BX-64

A purified target compound was obtained according to the method of synthesizing BX-76, and the molecular weight was identified to be correct by mass spectrometry ([M+2]²⁺=901.9).

### 47. Synthesis of BX-65

A purified target compound was obtained according to the method of synthesizing BX-76, and the molecular weight was identified to be correct by mass spectrometry ([M+2]²⁺=908.2).

### 48. Synthesis of BX-77

A purified target compound was obtained according to the method of synthesizing BX-76, and the molecular weight was identified to be correct by mass spectrometry ([M+2]²⁺=1282.8).

### 49. Synthesis of BX-79

A purified target compound was obtained according to the method of synthesizing BX-76, and the molecular weight was identified to be correct by mass spectrometry ([M+2]²⁺=1276.4).

### 50. Synthesis of BX-100

A purified target compound was obtained according to the method of synthesizing BX-76, and the molecular weight was identified to be correct by mass spectrometry ([M+2]²⁺=1274.7).

### 51. Synthesis of BX-103

A purified target compound was obtained according to the method of synthesizing BX-76, and the molecular weight was identified to be correct by mass spectrometry ([M+2]²⁺=1281.6).

### 52. Synthesis of BX-104

A purified target compound was obtained according to the method of synthesizing BX-76, and the molecular weight was identified to be correct by mass spectrometry ([M+2]²⁺=1268.4).

### 53. Synthesis of BX-99

A purified target compound was obtained according to the method of synthesizing BX-76, and the molecular weight was identified to be correct by mass spectrometry ([M+2]²⁺=959.3).

### 54. Synthesis of BX-102

A purified target compound was obtained according to the method of synthesizing BX-76, and the molecular weight was identified to be correct by mass spectrometry ([M+2]²⁺=915.5).

### 55. Synthesis of BX-56

A backbone was synthesized according to the method of synthesizing BX-34. A DMF solution of 5 equivalents of iodine was added to the peptide resin and allowed to react at room temperature (25°C±5°C) for 6 h to remove the protecting group Trt on the side chain of Cys while oxidizing. After filtration by suction, it was washed 6 times with DMF and 3 times with DCM, and dried. Cleavage and purification were then performed according to the method of synthesizing BX-34, to give a purified target compound. The molecular weight was identified to be correct by mass spectrometry ([M+2]²⁺=993.2).

### 56. Synthesis of BX-57

A purified target compound was obtained according to the method of synthesizing BX-56, and the molecular weight was identified to be correct by mass spectrometry ([M+2]²⁺=1000.4).

### 57. Synthesis of BX-95

A purified peptide backbone was obtained according to the method of synthesizing BX-34. Similarly, a purified CPAK peptide was obtained according to the method of synthesizing BX-34. The purified peptide backbone and the purified CPAK peptide were dissolved at equal equivalents in a 50% aqueous acetonitrile solution, with pH adjusted to 8-9 with triethylamine. The reaction was allowed to proceed at room temperature (25°C±5°C) until completion. Purification was then performed according to the method of BX-34, to give a purified target compound. The molecular weight was identified to be correct by mass spectrometry ([M+3]³⁺=850.5).

### Example 1: Screening of Polypeptides for Activity on Human ETB Receptor

293T-17 cells were seeded at a density of 650,000 cells per well in a 6-well plate and cultured overnight in an incubator at 37°C and 5% CO₂. Afterward, the cells were transfected with an expression vector, PCDNA3.1-hETB which contained a human ETB receptor gene, at a plasmid concentration of 2.5 µg per well. The cells were further cultured for 48 hours, and green fluorescence expression was observed under a fluorescence microscope. The cells were then digested with trypsin and counted. The activity of compounds on the human ETB receptor was determined with the IP-One Gq kit from Cisbio Bioassays. The cells were suspended in Stim Buffer provided in the kit and the cell suspension was added to a 96-well detection plate at 7 µL cell suspension (containing 30,000 cells) per well. The polypeptides to be screened for activity (prepared according to the Preparation Example of the invention) and polypeptide IRL1620 as positive control were dissolved in Stim B to be at a concentration of 2×, and 7 µL was added to the test cells, while 7 µL of PBS solution was added to the cells in the negative control wells. IRL1620 was used as the positive control polypeptide. The plate was sealed and incubated at 37°C for 1 hour in a cell culture incubator. Test wells, positive control wells, and PBS control wells were each added with 3 µL of IP1 d2 working solution and 3 µL of IP1 Tb cryptotate Antibody working solution. The plate was then sealed and incubated in the dark at room temperature (25°C±5°C) for 1 hour. The plate seal was removed, and the absorbance at 665 nm and 620 nm was measured with an ID5 microplate reader. The ratio of the acceptor emission signal to the donor emission signal, i.e., the value of (signal at 665 nm/signal at 620 nm)×10⁴, was calculated for each well. With F1 representing the average value of values of signal at 665 nm/signal at 620 nm determined for the test sample at a specific concentration, F2 representing the average value of values of signal at 665 nm/signal at 620 nm determined for the positive control IRL1620 at the same concentration, and T1 representing the average value of values of signal at 665 nm/signal at 620 nm determined for the PBS negative control, the percentage of activity of the test polypeptide sample relative to the positive control sample IRL1620 is equal to (F1-T1)×100/(F2-T1). hETB sequence (NCBI accession number NM_000115.5).

### Example 2: Selectivity of Polypeptides on Human ETA Receptor

293T-17 cells were seeded at a density of 650,000 cells per well in a 6-well plate and cultured overnight in an incubator at 37°C and 5% CO₂. Afterward, the cells were transfected with an expression vector, PCDNA3.1-hETA which contained a human ETA receptor gene, at a plasmid concentration of 2.5 µg per well. The cells were further cultured for 48 hours, and green fluorescence expression was observed under a fluorescence microscope. The cells were then digested with trypsin and counted. The activity of compounds on the human ETA receptor was determined with the IP-One Gq kit from Cisbio Bioassays. The cells were suspended in Stim Buffer provided in the kit and the cell suspension was added to a 96-well detection plate at 7 µL cell suspension (containing 30,000 cells) per well. The polypeptides to be screened for activity (prepared according to the Preparation Example of the invention) and polypeptide human endothelin ET1 as positive control were dissolved in Stim B to be at a concentration of 2×, and 7 µL was added to the test cells, while 7 µL of PBS solution was added to the cells in the negative control wells. Human endothelin ET1 (ET1: CSCSSLMDKECVYFCHLDIIW (C1-C15,C3-C11)) set forth in SEQ ID No. 85 was used as the positive control polypeptide. The plate was sealed and incubated at 37°C for 1 h in a cell culture incubator. Detection wells, positive control wells, and PBS control wells were each added with 3 µL of IP1 d2 working solution and 3 µL of IP1 Tb cryptotate Antibody working solution. The plate was sealed and incubated in the dark at room temperature (25°C±5°C) for 1 h. The plate seal was removed, and the absorbance at 665 nm and 620 nm was measured with an ID5 microplate reader. The ratio of the acceptor emission signal to the donor emission signal, i.e., the value of (signal at 665 nm/signal at 620 nm)×10⁴, was calculated for each well. With F3 representing the average value of values of signal at 665 nm/signal at 620 nm determined for the test sample at a specific concentration, F5 representing the average value of values of signal at 665 nm/signal at 620 nm determined for the positive control human endothelin ET1 at the same concentration, and T3 representing the average value of values of signal at 665 nm/signal at 620 nm determined for the PBS negative control, the percentage of activity of the test polypeptide sample relative to the positive control sample ET1 = (F3-T3)×100/(F5-T3) (see Table 6). hETA sequence (NCBI accession number L06622.1).

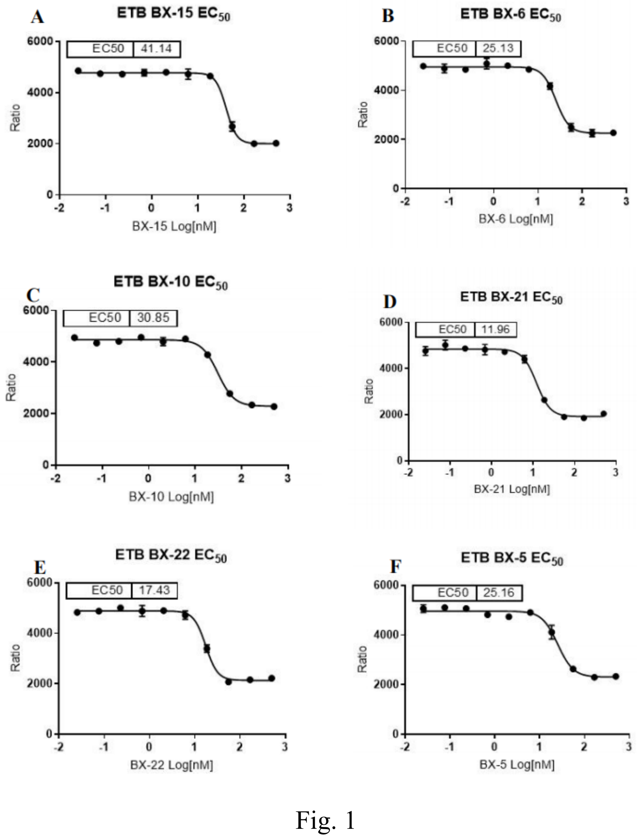

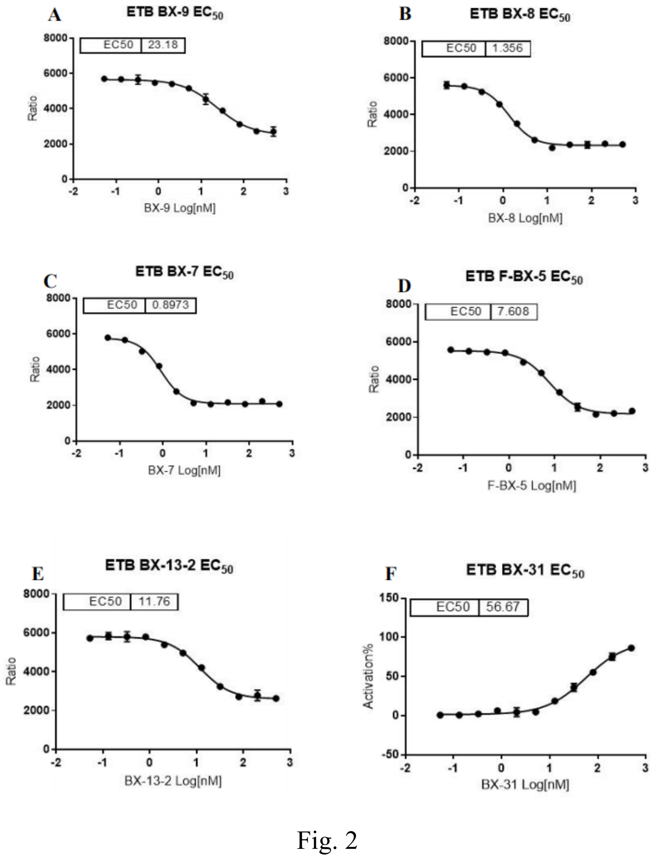

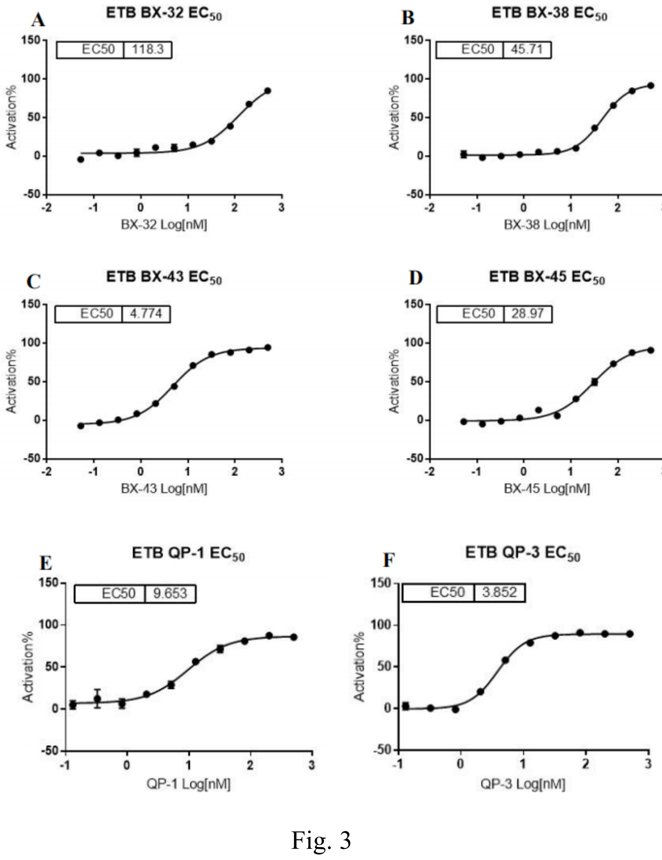

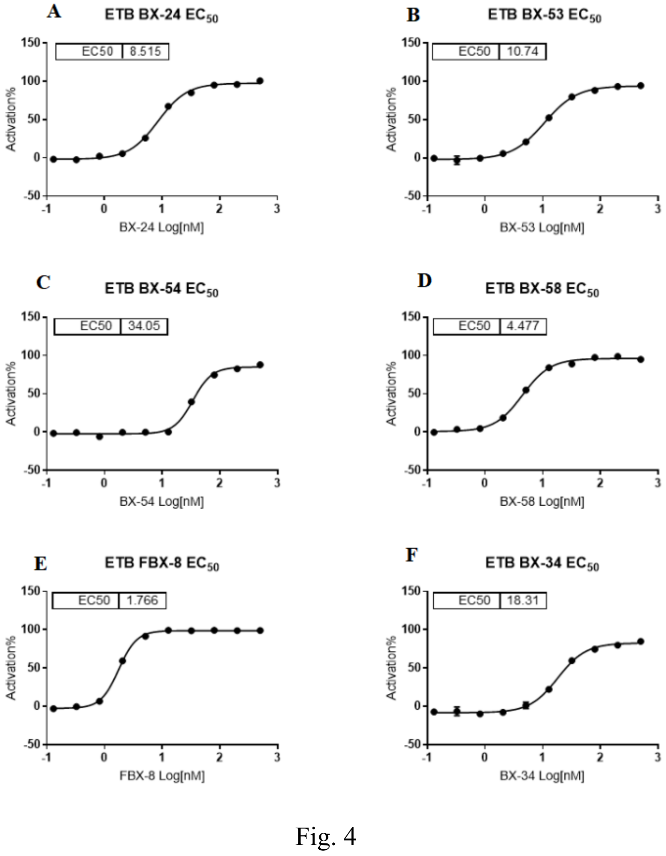

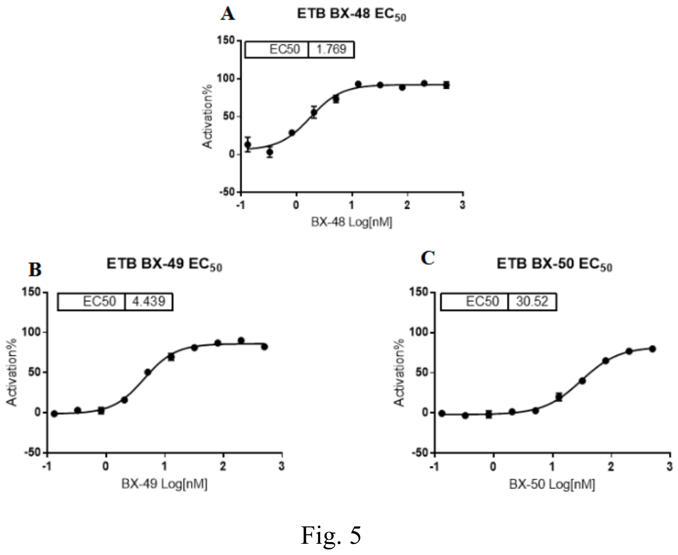

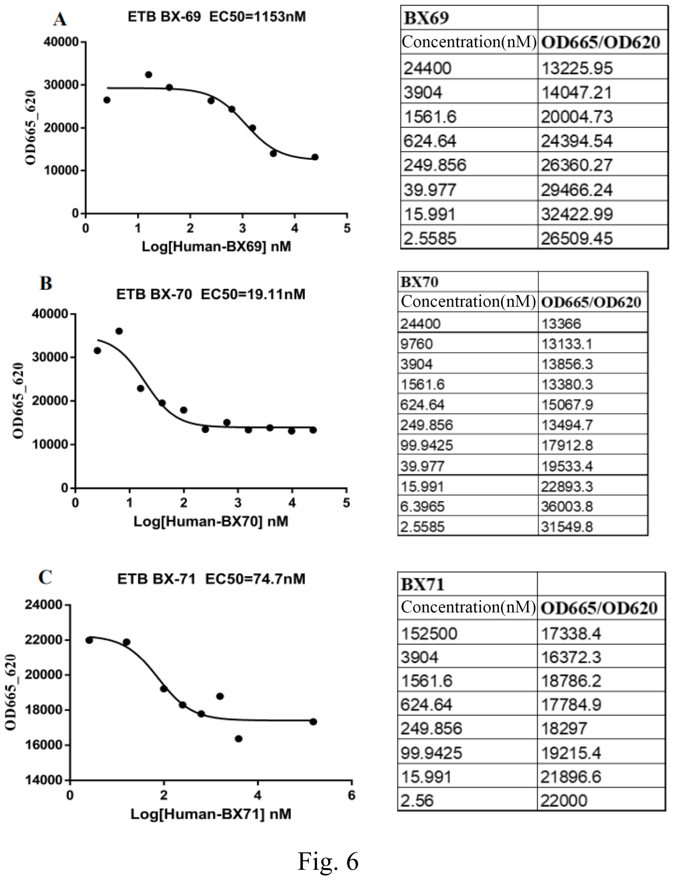

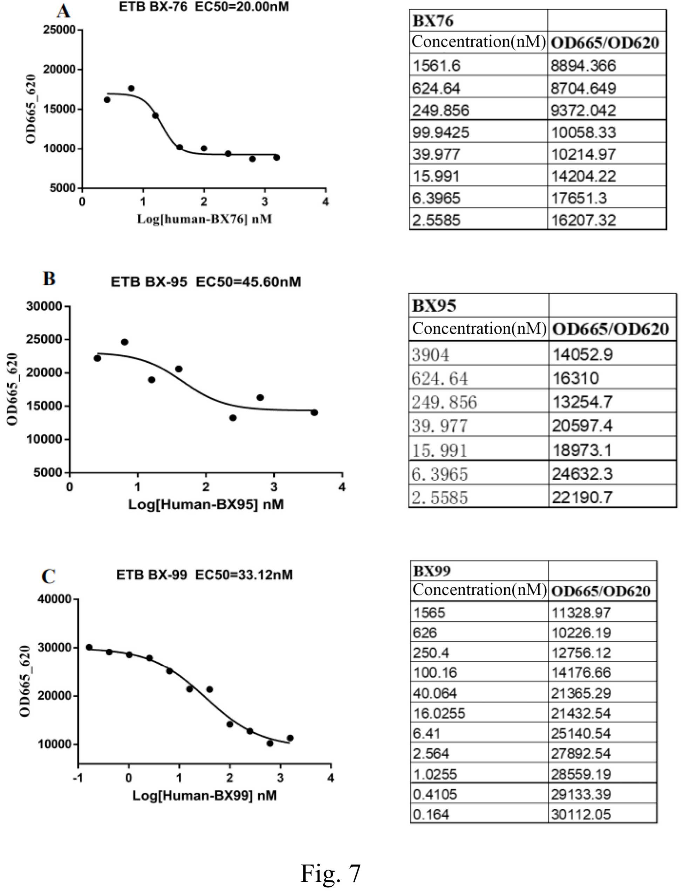

### Example 3: Determination of EC50 of Polypeptides against Human ETB Receptor (See Table 1 and Figures 1-7)

293T-17 cells were seeded at a density of 650,000 cells per well in a 6-well plate and cultured overnight in an incubator at 37°C and 5% CO₂. Afterward, the cells were transfected with an expression vector, PCDNA3.1-hETB which contained a human ETB receptor gene, at a plasmid concentration of 2.5 µg per well. The cells were further cultured for 48 hours, and green fluorescence expression was observed under a fluorescence microscope. The cells were then digested with trypsin and counted. The activity of compounds on the human ETB receptor was determined with the IP-One Gq kit from Cisbio Bioassays. The cells were suspended in Stim Buffer provided in the kit and the cell suspension was added to a 96-well detection plate at 7 µL cell suspension (containing 30,000 cells) per well. The polypeptides to be tested (prepared according to the Preparation Example of the invention) were diluted in Stim B to obtain a gradient of 11 concentration points. For each concentration point, the polypeptides to be tested were dissolved in Stim B to be at a concentration of 2×, and added at 7 µL to the test cells, while 7 µL of PBS solution was added to the cells in the negative control wells. The plate was sealed and incubated at 37°C for 1 hour in a cell culture incubator. Detection wells, positive control wells containing human endothelin ET1 (ET1: CSCSSLMDKECVYFCHLDIIW (C1-C15,C3-C11)), and PBS control wells were each added with 3 µL of IP1 d2 working solution and 3 µL of IP1 Tb cryptotate Antibody working solution. The plate was then sealed and incubated in the dark at room temperature (25°C±5°C) for 1 hour. The plate seal was removed, and the absorbance at 665 nm and 620 nm was measured with an ID5 microplate reader. The ratio of the acceptor emission signal to the donor emission signal, i.e., (signal at 665 nm/signal at 620 nm)×10⁴, was calculated for each well. With F representing the average value of values of signal at 665 nm/signal at 620 nm for the test samples at various concentrations, a scatterplot was plotted, where the abscissa represented the F value, and the ordinate represented the logarithmic value of the peptide concentration. A curve was fitted with an appropriate method to calculate EC50 values of the polypeptides against the human ETB receptor. Another method was provided for fitting a curve where activation rate of the test polypeptides at each concentration relative to the positive control sample human endothelin ET1 was calculated first with the method in Example 1, and a scatterplot was plotted, where the abscissa represented the activation rate, and the ordinate represented the logarithmic value of the polypeptide concentration. A curve was fitted with an appropriate method to calculate the EC50 values of the polypeptides against the human ETB receptor.

### Example 4: Determination of EC50s of Polypeptides against Rat ETB Receptor (See Table 8)

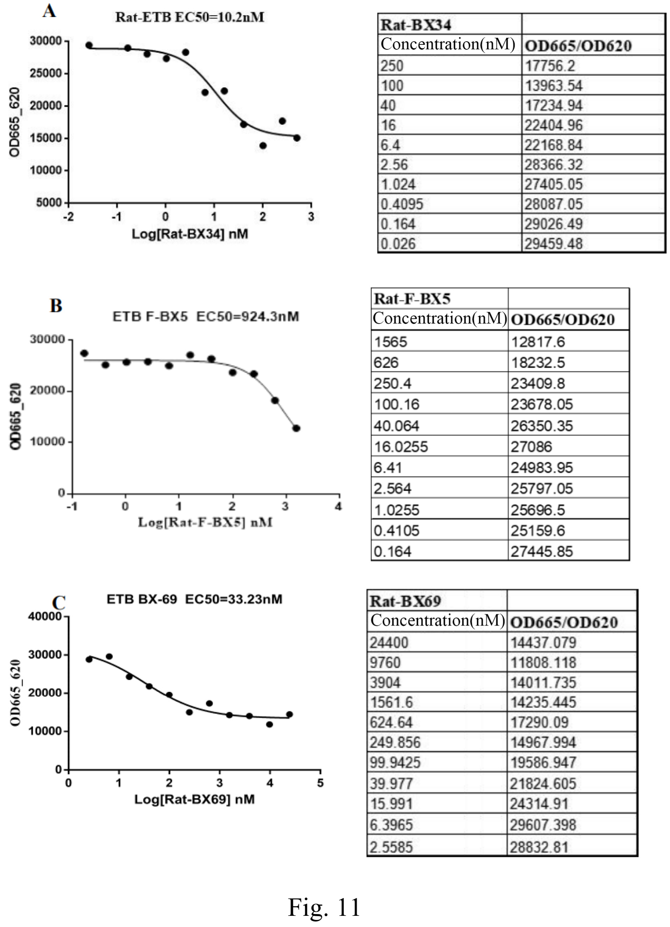

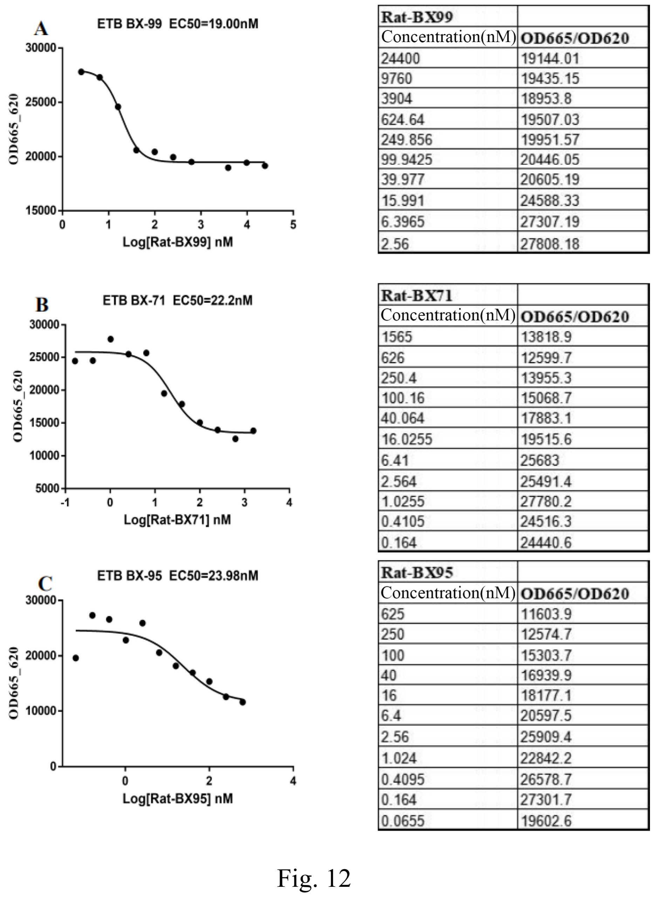

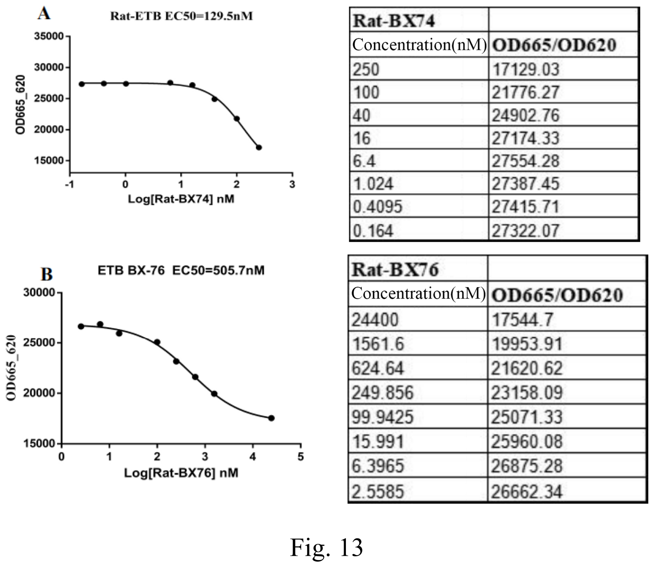

The process is the same as that in Example 3, except that the transfected plasmid was expression vector PCDNA3.1-RETB which contained a rat ETB receptor gene. EC50 values of the polypeptides against the rat ETB receptor were calculated (see Figures 11-13). The sequence of the rat ETB receptor (NCBI accession number: X57764.1).

### Example 5: Determination of EC50s of Polypeptides against Mouse ETB Receptor (See Table 9)

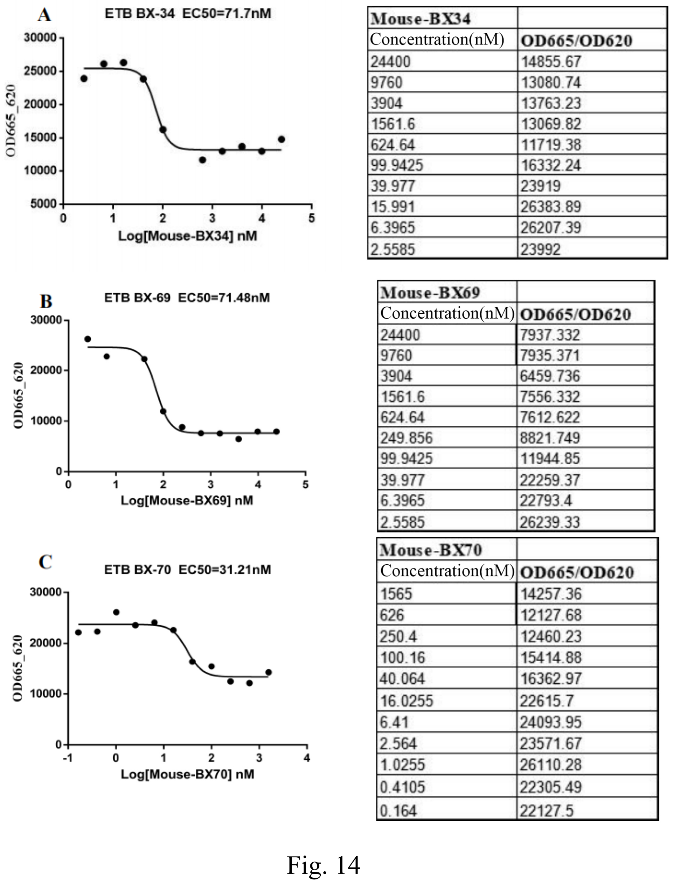

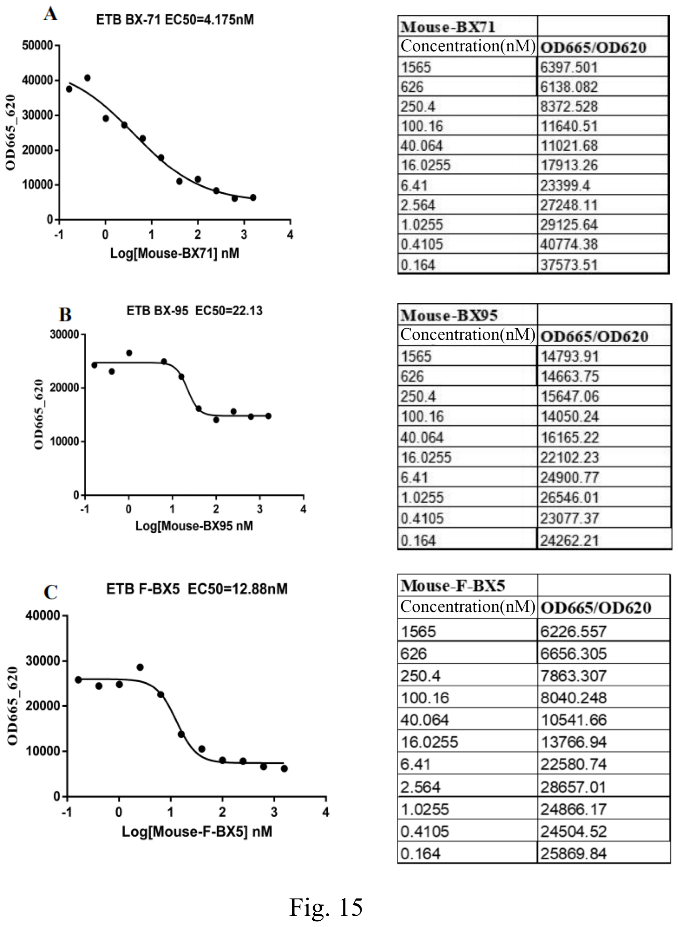

The process is the same as that in Example 3, except that the transfected plasmid was expression vector PCDNA3.1-mETB which contained a mouse ETB receptor gene. EC50 values of the polypeptides against the mouse ETB receptor were calculated (Figures 14 and 15). The sequence of the mouse ETB sequence (NCBI accession number: NM_001276296).

### Example 6: Determination of EC50s of Polypeptides against Dog ETB Receptor (See Table 7)

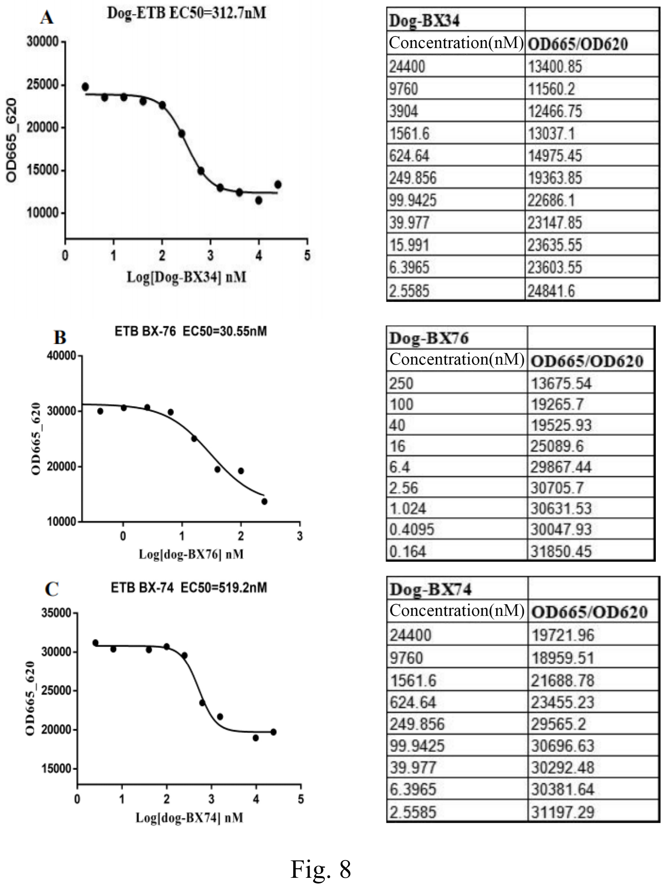

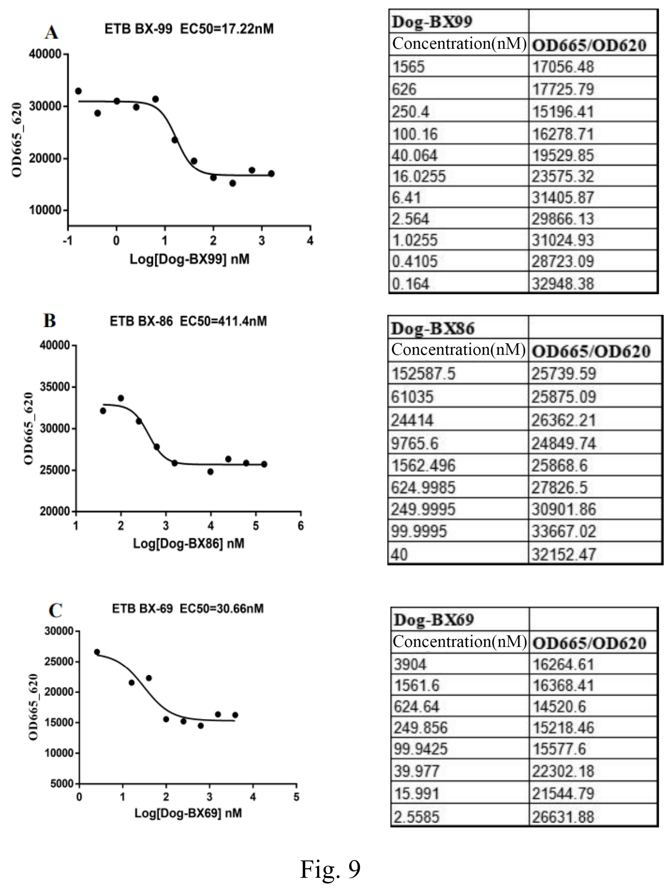

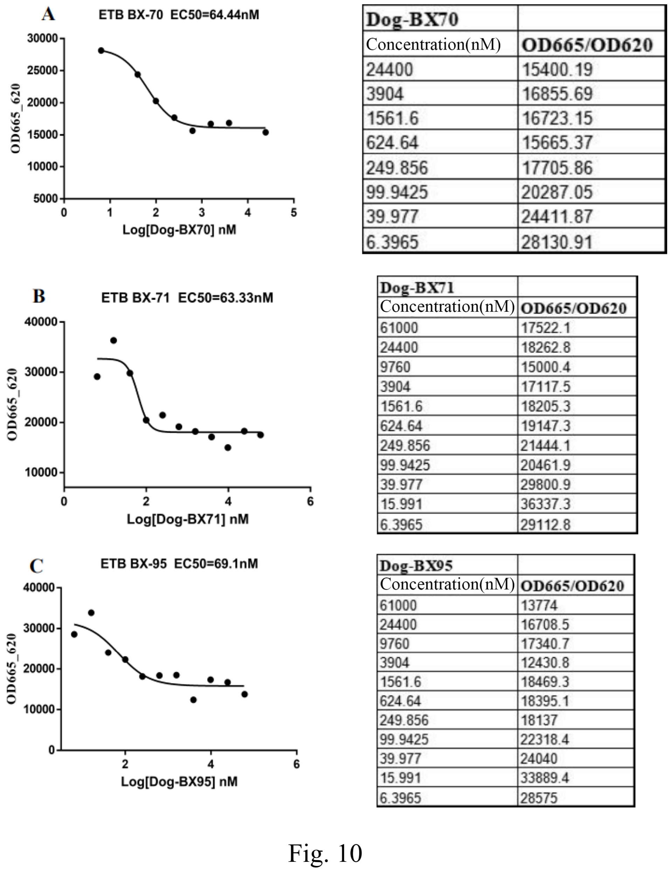

The process is the same as that in Example 3, except that the transfected plasmid was expression vector PCDNA3.1-DETB which contained a dog ETB receptor gene. EC50 values of the polypeptide against the dog ETB receptor were calculated (see Figures 8-10). The sequence of the dog endothelin receptor B (NCBI accession number: NM_001010943.2).

### Example 7: Effect of Polypeptide BX-34 on Cerebral Blood Flow Perfusion in mouse model of dMACO

Test drug: BX-34 prepared according to the Preparation Example of the present invention.

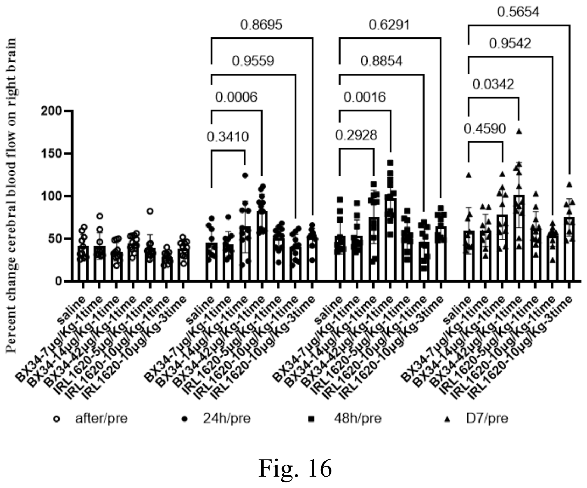

Eight-week-old male C57 BL/6 mice were used and acclimated for at least 3 days. On the day of experiment, the mice were anesthetized with isoflurane, and an incision of approximately 2 cm in length was made in the middle of the cerebral cortex. The mice were placed under a laser speckle flowmeter to capture a video of blood flow on both sides of the brain as images of pre-ischemic blood flow perfusion. Subsequently, a skull incision (3 mm×1.5 mm) was made at the right rear of the intersection of the sagittal suture and the coronal suture with a skull drill. The distal end of the right middle cerebral artery was located under a microscope, and a No.11 suture was used to ligate the middle artery. Meanwhile, the right common carotid artery was separated and ligated. A video of blood flow on both sides of the brain was captured again as images of post-ischemic blood flow perfusion. The scalp was sutured, and the animals were rewarmed in a rewarming box. Successfully modeled dMACO mice were randomly assigned to single-dose BX-34 groups of 7, 14, and 42 µg/Kg, single-dose IRL1620 groups of 5 and 10 µg/Kg, a three-dose IRL1620 group of 10 µg/Kg, and a normal saline control group. The single-dose groups were administered via tail vein 1 h after modeling, while the three-dose group was administered via tail vein at 1 hour, 3 hours, and 5 hours after modeling. A video of blood flow on both sides of the brain of each animal was captured again at 24 hours, 48 hours, and D7 after administration. During data analysis, the ROI was introduced or the area requiring blood flow calculation was selected. The average blood flow of the selected area was calculated by double-clicking on the blood flow image. Ratios of the average blood flow perfusions on right brain after modeling, and 24 hours, 48 hours and D7 after administration, to the average blood flow on right brain before modeling were used for statistical analysis of each group. Results are shown in Figure 16.

It can be seen from the results that at 24 hours, 48 hours, and D7 after administration of a single dose of 42 µg/kg BX-34 in the mouse model of dMCAO, the cerebral blood flow perfusion amount was significantly increased compared to the normal saline group. In contrast, there were no significant differences between the IRL1620 groups (with a single dose of 5 µg/kg, a single dose of 10 µg/kg, or three doses of 10 µg/kg) and the normal saline group. A single dose of 42 µg/kg BX-34 (which was a molar dose that equals to the total dosage of three administrations of 10 µg/kg) significantly increased the cerebral blood flow perfusion in dMCAO mice, showing a dose-dependent effect.

### Example 8: Determination of Half-Lives of Polypeptides

The polypeptides in this Example (F-BX-5, BX-34, BX-48, BX-76, BX-86, BX-69, BX-70, BX-71, BX-56, BX-225, BX-238, BX-229, and BX-253) were prepared according to the Preparation Example of the invention.

Administration and treatment methods for plasma samples were as follows. Three C57 BL/6 mice were injected with the compounds in Table 12 (taking BX-34 as an example) at a concentration of 60 µg/mL, with each mouse receiving 200 µL. Blood samples were collected at 1 minute, 5 minutes, 15 minutes, and 30 minutes after administration. Immediately after the blood collection, a protease inhibitor was added, and the samples were centrifuged at 3200 rpm at 4°C for 10 minutes. The plasma was taken, and then acetonitrile in a two-fold volume was added to precipitate proteins. Afterward, centrifugation was performed at 10000 rpm for 5 minutes. After filtered with a 0.22 µm filter, it was analyzed with a liquid chromatography-mass spectrometry instrument. The plasma drug concentration was calculated based on the integral areas of the control drug and the test drug. The average value of the three detected values at each time point for different drugs were calculated. Meanwhile, a blank mouse plasma sample was prepared, in which proteins were precipitated, and then the sample was used as a control.

**Table 12. Half-life Data of Polypeptides**

| Compound numbering | Dosage, i.v. | Half-life (min) | AUC_{last 0-30 min} (ng.h/mL) |
|---|---|---|---|
| IRL1620 | 0.48 mpk | <2.6 | 130 |
| F-BX-5 | 0.96 mpk | <3 | 127 |
| BX-34 | 0.48 mpk | 63 | 7736 |
| BX-48 | 0.48 mpk | 3.9 | 757 |
| BX-76 | 0.96 mpk | 3.6 | 250 |
| BX-86 | 0.96 mpk | 63 | 1146 |
| BX-69 | 0.96 mpk | 30 | 1611 |
| BX-70 | 0.96 mpk | 41 | 1701 |
| BX-71 | 0.96 mpk | 23 | 2049 |
| BX-56 | 1 mpk | 8.9 | 1376 |
| BX-225 | 1 mpk | 37 | 4981 |
| BX-238 | 1 mpk | 53 | 2302 |
| BX-229 | 1 mpk | 77 | 3393 |
| BX-253 | 1 mpk | 63 | 3989 |

Conditions for the liquid chromatography-mass spectrometry were as follows.
Instrument model: Agilent InfinityLab LC/MSD 1260-G6125C
Chromatographic column: Poroshell 120 SB-C18 4.6*100 mm, 2.7 µm
Mobile phase A: 0.1% formic acid-ultrapure water
Mobile phase B: 0.1% formic acid-acetonitrile
Gradient settings:

| Time (min) | Phase A | Phase B | Flow Rate |
|---|---|---|---|
| 0 | 90% | 10% | 0.4 mL/min |
| 18 | 0 | 100% | 0.4 mL/min |
| 20 | 0 | 100% | 0.4 mL/min |

Column temperature: 45°C; Sample volume loaded: 20 µL;
Ion source: ESI; Dryer temperature: 350°C; Dryer flow rate: 12 L/min; Nebulizing pressure: 45 psi; Capillary voltage: 4000 V.
Scan type: SIM; Fragmentor voltage: 135 V; Scan/dwell time: 200 ms.

### Example 9: In Vivo Efficacy of Polypeptides BX34 and BX48 in Rat Model of pMACO

Test drugs: BX-34 and BX-48, prepared according to the Preparation Example of the present invention (administrated at a same molar dose due to different molecular weights).

Male SD rats weighing 280-300 g were used and acclimated for at least 3 days. Focal cerebral ischemia was induced by middle cerebral artery occlusion with a suture method under isoflurane anesthesia. The animals were placed in a secure supine position, and a midline incision was made to expose the right common carotid artery, external carotid artery, and internal carotid artery. A 4.0 monofilament nylon suture was advanced from the external carotid artery toward the internal carotid artery lumen until resistance was felt ( ~ 20 mm), indicating middle cerebral artery occlusion. The nylon filament was allowed to stay in place to create a permanent model of focal cerebral ischemia. Successfully modeled rats were randomly assigned to four groups: a solvent (normal saline) group (n=10), a 7 µg/Kg BX-34 group (n=10), a 5 µg/Kg BX-48 group (n=10), and a 5 µg/Kg IRL1620 group (n=10). Each group was administered once at 2 hours, 4 hours, and 6 hours, respectively, after modeling, for a total of three administrations. Grip strength was measured before administration and at 24 hours and on D3 and D7 after administration. A rotarod test and a foot fault test were performed before administration and at 24 hours and day 7 after administration. During the rotarod test, time and distance were recorded, and ratios of the measurement at 24 hours and the measurement at D7 to the pretest measurement were used as analysis indicators. The specific method for the foot fault test was as follows. The rats were placed on an elevated grid floor (29×29 cm, grid opening: 3 cm²) for 2 minutes, and the number of foot faults (when a forelimb fell through the grid) and the total steps were recorded. % Foot fault = (the number of forelimb faults/the total number of forelimb steps) × 100. Results of the behavior tests are shown in Tables 13, 14, and 15.

The results showed that the rotarod test and grip strength test results of each administration group on D3 and D7 were better than those of the normal saline group, with significant improvements. On D7 after administration, the percentages of foot faults of the administration groups were lower than that of the saline group. The percentages of foot faults of the BX-34 group and the BX-48 group were lower than that of the saline group and the IRL1620 group.

**Table 13. Results of Grip Strength Test**

| **Grip Strength Test** | **24h/pre** | **3D/pre** | **7D/pre** |
|---|---|---|---|
| **1-IRL1620-5µg/Kg-3Time** | 47% ±27% | 57%±11% | 61%+13% |
| **2-BX34-7µg/Kg-3Time** | 40% ±24% | 63% ±14% | 67% ±19% |
| **4-saline-3Time** | 38% ±22% | 54% ±17% | 56%±23% |
| **5-BX48-5µg/Kg-3Time** | 47% ±23% | 60% ±7% | 66% ±22% |

**Table 14. Results of Rotarod Test**

| **Experimental Groups** | **24h/pretest (%)** | | | **7D/pretest (%)** | | |
|---|---|---|---|---|---|---|
| **Rotarod Test** | **Time, s** | **Velocity** | **Distance, m** | **Time, s** | **Velocity** | **Distance, m** |
| **1-IRLI620-5µg/Kg-3Time** | 59.43%±71.65 % | 57.24%±70.27 % | 55.14%+76.29% | 64.06%±27.89 % | 60.63%±34.62 % | 45.93%±43.82% |
| **2-BX34-7µg/Kg-3Time** | 38.34%±38.08 % | 39.09%±35.03 % | 28.25%±44.00% | 65.5%±29.94% | 62.27%±34.12 % | 49.60%±49.48% |
| **4-saline-3Time** | 17.06%±22.65 % | 23.76%±14.01 % | 6.99%±10.61% | 43.52%±25.05 % | 47.64%±10.12 % | 21.42%±16.99% |
| **5-BX48-5µg/Kg-3Time** | 23.79%±27.04 % | 47.16%±30.9A % | 16.15%±22.62% | 81.64%±10.87 % | 76.52%±13.55 % | 64.97%±19.43% |

**Table 15. Results of Foot Fault Test**

| **Foot fault Analysis** | **Mean of Pretest** | **Mean of 24h** | **Mean of 7D** |
|---|---|---|---|
| **1-IRL1620-5µg/Kg-3Time** | 1%±2% | 14.2%±17.61% | 4.3%+3.14% |
| **2-BX34-7µg/Kg-3Time** | 1%±1% | 10.64%±15.53% | 3.46%±2.92% |
| **4-saline-3Time** | 0.78%±0.72% | 13.88%±17.10% | 5.18%±5.74% |
| **5-BX48-5µg/Kg-3Time** | 1.11%±0.95% | 10.89%±10.53% | 2.24%±3.1% |

### Example 10: Preliminary Study on Acute Toxicity of Intravenous Administration of Polypeptides IRL1620, BX225, BX229, BX238, BX253, BX254, BX255, and BX256

In an experiment about acute toxicity of intravenous administration, male SD rats weighing 200-230 g were dosed in a range between 480 µg/Kg and 30 µg/Kg via tail vein injection in a descending order of dosage. The animals were observed for 24 hours after administration. If a certain dose resulted in a high mortality of the rats, the injection dosage was reduced for further observation. Toxic response and mortality of the rats were preliminarily observed, and time of death and doses without mortality of the rats were recorded. The level of acute toxicity of the polypeptides was preliminarily determined based on the doses without mortality. The acute toxicity study showed that the maximum tolerated doses of IRL1620, BX225, BX229, BX238, BX253, BX254, and BX255 in the rats were 30 µg/Kg, 30 µg/Kg, 60 µg/Kg, 30 µg/Kg, 480 µg/Kg, 120 µg/Kg, and 120 µg/Kg, respectively. The maximum tolerated dosage of polypeptides BX225, BX229, BX238, BX253, BX254, and BX255 injected into the SD rats were higher than that of IRL1620, with intravenous administration of BX253 showing the lowest acute toxicity (Table 16).

**Table 16: Acute Toxicity of Intravenous Administration of IRL1620, BX225, BX229, BX238, BX253, BX254, BX255, and BX256**

| **Animal No.** | **Drug** | **Intravenous Administration Dosage** | **Observations of Animal Status** |
|---|---|---|---|
| 1 | IRL1620 | 120 µg/Kg | Developed rapid breathing and the body turned purple at 5 minutes; died at 9 minutes |
| 2 | IRL1620 | 60 µg/Kg | Developed rapid breathing at 3 minutes; died at 8 minutes |
| 3 | IRL1620 | 30 µg/Kg | Developed rapid breathing at 13 minutes, showed no willingness to move at 30 minutes; recovered at 1.5 hours |
| 4 | IRL1620 | 30 µg/Kg | Developed rapid breathing at 15 minutes, showed no willingness to move at 25 minutes; recovered at 1.5 hours |
| 5 | IRL1620 | 30 µg/Kg | Lay still at 25 minutes, showed no willingness to move at 1.5 hours; found dead the next morning (estimated to have died during the night) |
| 1 | BX238 | 240 µg/Kg | Developed abdominal breathing at 5-6 minutes, developed rapid breathing and collapsed at 9 minutes; died at 11 minutes |
| 2 | BX238 | 120 µg/Kg | Developed rapid breathing at 5 minutes, the eyes turned pale and purple at 14 minutes; died at 16 minutes |
| 3 | BX238 | 60 µg/Kg | Developed rapid breathing at 40-45 minutes; died at 1 hour |
| 4 | BX238 | 30 µg/Kg | Breathing speeded up at 50 minutes, showed no willingness to move at 1.2 hours; found dead the next morning (estimated to have died during the night) |
| 5 | BX238 | 30 µg/Kg | Breathing speeded up at 53 minutes, showed no willingness to move at 1.2 hours; found dead the next morning (estimated to have died during the night) |
| 1 | BX255 | 480 µg/Kg | The body turned purple at 23 minutes after administration; died at 29 minutes after administration |
| 2 | BX255 | 360 µg/Kg | The body turned purple at 12 minutes after administration; died at 22 minutes after administration |
| 3 | BX255 | 240 µg/Kg | No obvious reaction in the first 27 minutes after administration; breathing speeded up at 27 minutes; died at 49 minutes after administration |
| 4 | BX255 | 240 µg/Kg | No obvious reaction in the first 24 minutes after administration; breathing speeded up at 27 minutes; died at 43 minutes after administration |
| 5 | BX255 | 120 µg/Kg | No obvious adverse reactions in the first 1 hour; breathing became slightly faster at nearly 2 hours; was in good condition and recovered when observed the next day |
| 6 | BX255 | 120 µg/Kg | Showed no willingness to move at 9 minutes; recovered at 1 hour and 10 minutes |
| 7 | BX255 | 120 µg/Kg | Showed no willingness to move at 9 minutes; recovered at 1 hour and 10 minutes |
| 8 | BX255 | 120 µg/Kg | Lay still and showed no willingness to move at 12 minutes; became active at 17 minutes; signs of pain were observed in the animal through pupil observation; no pain at 28 minutes |
| 9 | BX255 | 120 µg/Kg | Developed difficulty in breathing at 17 minutes, recovered to normal in about 10 seconds; signs of pain were observed in the animal through pupil observation; no pain at 28 minutes |
| 1 | BX225 | 240 µg/Kg | Developed rapid breathing at 4 minutes, collapsed at 5 minutes, eyes turned dull at 7 minutes; died at 12 minutes |
| 2 | BX225 | 120 µg/Kg | Breathing became slightly faster at 2 minutes, showed no willingness to move, had slow reactions, and breathing speeded up at 25 minutes; the symptoms basically eased at 1.5 hours |
| 3 | BX225 | 120 µg/Kg | Showed no willingness to move, had slow reactions, and breathing became slightly faster at 10 minutes; the symptoms lasted for nearly 1 hour; the symptoms basically eased at 1 hour and 10 minutes |
| 4 | BX225 | 120 µg/Kg | Developed abdominal breathing and lay still at 27 minutes; died of difficulty in breathing at 40 minutes |
| 5 | BX225 | 120 µg/Kg | Developed abdominal breathing and lay still at 25 minutes; died of difficulty in breathing at 32 minutes |
| 6 | BX225 | 60 µg/Kg | Lay still and showed no willingness to move at 7 minutes; became active at 10 minutes; signs of pain were observed in the animal through pupil observation; no pain at 23 minutes |
| 7 | BX225 | 60 µg/Kg | Lay still and showed no willingness to move at 7 minutes; became active at 10 minutes; signs of pain were observed in the animal through pupil observation; developed rapid breathing at 35 minutes; died at 70 minutes |
| 8 | BX225 | 30 µg/Kg | No obvious abnormalities observed within 24 hours |
| 9 | BX225 | 30 µg/Kg | No obvious abnormalities observed within 24 hours |
| 10 | BX225 | 30 µg/Kg | No obvious abnormalities observed within 24 hours |
| 11 | BX225 | 30 µg/Kg | No obvious abnormalities observed within 24 hours |
| 12 | BX225 | 30 µg/Kg | No obvious abnormalities observed within 24 hours |
| 13 | BX225 | 30 µg/Kg | No obvious abnormalities observed within 24 hours |
| 1 | BX253 | 240 µg/Kg | No abnormalities |
| 2 | BX253 | 480 µg/Kg | No abnormalities |
| 3 | BX253 | 480 µg/Kg | No abnormalities |
| 4 | BX253 | 480 µg/Kg | No abnormalities |
| 5 | BX253 | 480 µg/Kg | No abnormalities |
| 6 | BX253 | 480 µg/Kg | No abnormalities |
| 1 | BX254 | 240 µg/Kg | No obvious abnormalities |
| 2 | BX254 | 240 µg/Kg | Developed rapid breathing, the limbs turned purple, developed breathing depression, and limbs moved like swimming at 15 minutes after administration; then died at 20 minutes after administration |
| 3 | BX254 | 240 µg/Kg | Developed rapid breathing, the limbs turned purple, and had weak limbs at 14 minutes after administration; died of breathing depression at 21 minutes after administration |
| 4 | BX254 | 480 µg/Kg | The body turned purple at 22 minutes after administration; died at 25 minutes after administration |
| 5 | BX254 | 360 µg/Kg | The body turned purple at 16 minutes after administration; died at 26 minutes after administration |
| 1 | BX256 | 240 µg/Kg | Developed rapid breathing and limbs turned pale purple, and developed convulsions at 16 minutes after administration; died at 27 minutes after administration |
| 2 | BX256 | 240 µg/Kg | Developed rapid breathing, the limbs and face turned purple, and had weak limbs at 15 minutes after administration; died at 21 minutes after administration |
| 1 | BX229 | 60 µg/Kg | No obvious reaction within 1 hour; developed rapid breathing at 1 hour; recovered to normal after 10 minutes |
| 2 | BX229 | 60 µg/Kg | No obvious reaction within 1 hour; developed rapid breathing at 1 hour; recovered to normal after 10 minutes |
| 3 | BX229 | 120 µg/Kg | Died at 26 minutes after administration; had white discharge in the nasal cavity and developed diarrhea |
| 4 | BX229 | 120 µg/Kg | Died at 26 minutes after administration; had white discharge in the nasal cavity and developed diarrhea |
| 5 | BX229 | 90 µg/Kg | Died at 1 hour after administration |
| 6 | BX229 | 90 µg/Kg | Developed rapid breathing at 25 minutes after administration; recovered after 25 minutes; showed no willingness to move |
| 7 | BX229 | 90 µg/Kg | Developed rapid breathing and the body turned purple at 1 hour after administration; partially recovered after 30 minutes |
| 8 | BX229 | 90 µg/Kg | Developed rapid breathing and diarrhea at 45 minutes after administration; recovered after 25 minutes |
| 9 | BX229 | 90 µg/Kg | Developed rapid breathing at 44 minutes after administration; died of convulsions at 52 minutes after administration |
| 10 | BX229 | 90 µg/Kg | Developed rapid breathing at 43 minutes after administration; recovered after 30 minutes |

### Example 11: Preliminary Study on Acute Toxicity of Subcutaneous Administration of Polypeptides IRL1620, BX229, and BX238

In an experiment about acute toxicity of subcutaneous administration, male SD rats weighing 200-230 g were dosed in a range between 60 µg/Kg and 960 µg/Kg. Observation was made for 48 hours after subcutaneous administration. Toxic response and mortality of the rats were preliminarily observed, and time of death and doses without mortality were recorded. The level of acute toxicity of the polypeptides was preliminarily determined based on the doses without mortality. Results of the experiment are shown in Table 17. It can be seen from the table that the acute toxicity of subcutaneous administration of BX229 was much lower than that of IRL1620.

**Table 17: Preliminary Study on Acute Toxicity of Subcutaneous Administration of Polypeptides IRL1620, BX229, and BX238**

| **Animal No.** | **Drug** | **Subcutaneous Administration Dosage** | **Observations of Animal Status** |
|---|---|---|---|
| 1 | IRL1620 | 60 µg/Kg | Developed rapid breathing at 25 minutes after administration, recovered after 15 minutes |
| 2 | IRL1620 | 60 µg/Kg | Developed rapid breathing at 26 minutes after administration, recovered after 16 minutes |
| 3 | IRL1620 | 60 µg/Kg | Developed rapid breathing at 25 minutes after administration, recovered after 15 minutes |
| 4 | IRL1620 | 60 µg/Kg | Developed rapid breathing at 30 minutes after administration, recovered after 17 minutes |
| 5 | IRL1620 | 60 µg/Kg | Developed rapid breathing at 25 minutes after administration, recovered after 16 minutes |
| 6 | IRL1620 | 120 µg/Kg | Developed rapid breathing at 26 minutes after administration and lasted for 23 minutes; then died |
| 7 | IRL1620 | 120 µg/Kg | Developed rapid breathing at 26 minutes after administration, gradually recovered after 50 minutes |
| 8 | IRL1620 | 120 µg/Kg | Developed rapid breathing at 26 minutes after administration and lasted for 26 minutes; then died |
| 9 | IRL1620 | 120 µg/Kg | Developed rapid breathing at 28 minutes after administration, gradually recovered after 50 minutes |
| 10 | IRL1620 | 120 µg/Kg | Developed rapid breathing at 27 minutes after administration, gradually recovered after 50 minutes |
| 1 | BX229-Na | 120 µg/Kg | No obvious reaction observed within 24 hours |
| 2 | BX229-Na | 120 µg/Kg | No obvious reaction observed within 24 hours |
| 3 | BX229-Na | 240 µg/Kg | No obvious abnormal reaction observed within 48 hours |
| 4 | BX229-Na | 240 µg/Kg | No obvious abnormal reaction observed within 48 hours |
| 5 | BX229-Na | 480 µg/Kg | No obvious abnormal reaction observed within 48 hours |
| 6 | BX229-Na | 480 µg/Kg | No obvious abnormal reaction observed within 48 hours |
| 7 | BX229-Na | 960 µg/Kg | No significant reaction within 2 hours, died at 2 hours and 10 minutes |
| 8 | BX229-Na | 960 µg/Kg | No obvious abnormal reaction observed within 48 hours |
| 9 | BX229-Na | 960 µg/Kg | No obvious abnormal reaction observed within 48 hours |
| 10 | BX229-Na | 960 µg/Kg | No obvious abnormal reaction observed within 48 hours |
| 11 | BX229-Na | 960 µg/Kg | No obvious abnormal reaction observed within 48 hours |
| 1 | BX238-Na | 240 µg/Kg | No obvious abnormal reaction observed within 48 hours |
| 2 | BX238-Na | 240 µg/Kg | No obvious abnormal reaction observed within 48 hours |
| 3 | BX238-Na | 360 µg/Kg | No obvious abnormal reaction observed within 48 hours |
| 4 | BX238-Na | 360 µg/Kg | No obvious abnormal reaction observed within 48 hours |
| 5 | BX238-Na | 480 µg/Kg | Developed rapid breathing at 50 minutes after administration, showed no willingness to move, recovered 2 hours after administration |
| 6 | BX238-Na | 480 µg/Kg | Developed rapid breathing at 21 minutes after administration, died at 26 minutes after administration |
| 7 | BX238-Na | 480 µg/Kg | Developed rapid breathing at 10 minutes after administration, died at 47 minutes after administration |
| 8 | BX238-Na | 480 µg/Kg | Developed rapid breathing at 45 minutes after administration, showed no willingness to move, recovered 2 hours after administration |
| 9 | BX238-Na | 480 µg/Kg | Developed rapid breathing at 20 minutes after administration, died at 55 minutes after administration |

### Example 12: Effect of Intravenous Administration of Polypeptides IRL1620, BX225, and BX229 on Blood Pressure in SD Rats

Test drugs: IRL1620, BX225, and BX229 prepared according to the Preparation Example of the invention.

Male SD rats were anesthetized with isoflurane, and their neck hairs were shaved. A midline incision was made on the neck to expose the trachea. A ventilator and anesthesia machine for small animals were turned on, with concentration of an anesthetic gas set to 1.2, and its exit channel connected to an inlet of the ventilator. Tidal volume of the ventilator was set to 2 mL, the respiratory rate was 90 breaths/minute, and the respiratory ratio was 1:2. An outlet end of the ventilator was connected to a plastic hose, which was inserted into the rat's airway.

A machine and a software of a biological signal data acquisition and analysis system were turned on. One end of a data acquisition plate was connected to the plastic hose, and the other end was connected to a collector valve connected with a 1-mL syringe. The syringe was filled with heparinized saline, which was injected into the data acquisition plate to expel the air in the entire space until the heparinized saline flowed out from the other end of the plastic hose. The right common carotid arteries of the rats were isolated. A dead knot was made at the distal end and a slipknot at the proximal end of a right common carotid artery. A 1/2-sized incision was made in the artery between the knot with a microscissor, and the plastic hose with heparinized saline was inserted into the incision and fixed to prevent back-and-forth movement. The initial value of the blood pressure was set to zero with the zero-adjustment valve of the biological signal data acquisition and analysis system. The slipknot at the proximal end of the common carotid artery was untied, and real-time blood pressure data of the animal appeared on the software. After the animal's blood pressure was stabilized, the drug was administered via tail vein, and changes in the animal's blood pressure was observed in real-time. The dosages of IRL1620, BX34, and BX229 were 5 µg/Kg, 42 µg/kg, and 6.6 µg/Kg, respectively.

Results showed that 5 µg/Kg of polypeptide IRL620, 42 µg/Kg of polypeptide BX34, and 6.6 µg/Kg of polypeptide BX229 had certain effects on the rats' blood pressure, causing a decrease in blood pressure. After the administration of 5 µg/Kg IRL620, the rats' blood pressure decreased rapidly and reached the lowest level within 2 minutes, with the maximum extent of decrease in blood pressure of 16.1%±2.4% of the baseline blood pressure. 42 µg/Kg BX34 and 6.6 µg/Kg BX229 had a relatively mild effect on the rats' blood pressure, causing a slow decrease in blood pressure over 20-35 minutes to the lowest blood pressure, with the maximum extents of decrease in blood pressure of 9.5%±1.7% and 17.0%±2.0% of the baseline blood pressure, respectively (Table 18).

**Table 18 Effect of Intravenous Administration of Polypeptides IRL1620, BX34, and BX229 on Blood Pressure in SD Rats**

| Drug (Administration Concentration) | IRL1620 (5 µg/Kg) | BX34 (42 µg/Kg) | BX229 (6.6 µg/Kg) |
|---|---|---|---|
| Maximum percentage of blood pressure reduction relative to baseline blood pressure (n = 3) | 16.1%±2.4% | 9.5%±1.7% | 17.0%±2.0% |
| Time from baseline blood pressure to the lowest blood pressure | Less than 2 mins | 20-35 mins | 20-30 mins |

### Example 13: Efficacy of BX229-Na Administrated Three Times per Week on Mouse Model of tMCAO

Test drug: BX-229, prepared according to the Preparation Example of the present invention.

Fifty-one male C57/6J mice, aged 10-12 weeks, weighing 25-30g, were housed for at least 2 days prior to the experiment to adapt to the environment (SPF Biotech). A mouse model of tMCAO was established. The mice were anesthetized with isoflurane. Their body temperature was controlled by a thermostatic blanket and monitored and maintained at 37±0.5°C. A middle incision was made in the skin on the mouse skull, the skin was pulled outward, and a bendable microneedle tip was fixed to the surface of the left parietal bone of the mouse skull. A middle incision on the neck was made to separate the right common carotid artery (CCA), external carotid artery (ECA) and internal carotid artery (ICA). According to the method of Longa (Longa et al., 1989), a silicone rubber-coated suture (RWD Life Science, suture end diameter: 0.22 mm-0.23 mm, MSMC23B120PK50) was introduced through an external carotid artery incision surgery and was slowly advanced through the internal carotid artery towards the origin of the middle cerebral artery. To ensure a sustained and successful occlusion of the middle cerebral artery, we monitored local cerebral blood flow of all stroke animals with a laser speckle flowmeter (generally, the blood flow can be reduced by 40-60% after stabilized). The suture was removed 45 min after the insertion for modeling, and the incision was sutured. Afterward, 1 mL of 37°C warm saline was injected subcutaneously, and the mice were placed on a heating pad until recovery. To alleviate pain, the mice were injected subcutaneously with Tilidine after awakening from anesthesia.

Successfully modeled C57 tMCAO mice were randomly assigned into three groups: a normal saline group, a 30µg/Kg BX229-Na administration group, and a 100µg/Kg BX229-Na administration group. Each group was administered once subcutaneously in the neck 4.5 hours after occlusion (D1), and was administered once more at the same sites and at the same time points on D3 and D6. The grouping and dosing regimen of this experiment are shown in Table 19. A sham group included 6 animals. 2 mL/kg was administrated subcutaneously in the neck. Garcia JH scoring was made on D7 and D14 after administration. After the completion of the scoring on D14, the mice were euthanized and harvested for infarcted brain tissues, to extract total proteins. Changes in protein markers VEGF-A and BDNF in each experimental group were detected by ELISA.

**Table 19: Experimental Grouping and Dosing Regimen**

| **Group** | **Number** | **t-MCAO Establishment** | **CPD** | **Dosage (µg/kg)** | **Volume (mL/kg)** | **Administration frequency** |
|---|---|---|---|---|---|---|
| 4 | 6 | Sham | Normal saline | - | 2 | D1: injected subcutaneously in the neck at 2 sites 4.5 hours after tMCAO occlusion |
| 1 | 15 | tMCAO | Normal saline | - | 2 | |
| 2 | 15 | tMCAO | BX229 | 30 | 2 | D3 and D6: each administrated once at the same time points and the same sites as D1 |
| 3 | 15 | tMCAO | BX229 | 100 | 2 | |

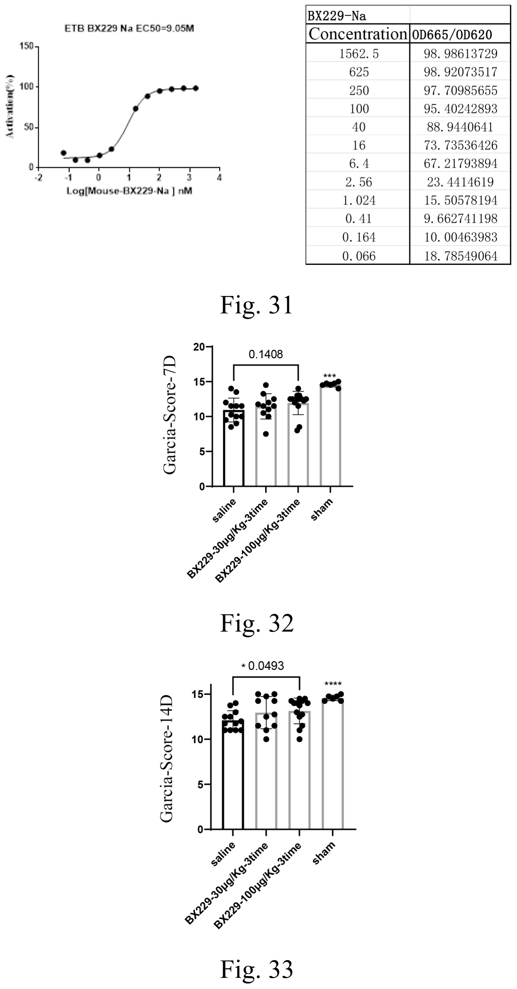

Results of the experiment showed that, from an analysis of the results of Garcia JH-15 scoring on D7, there was a significant difference in D7 scores between the sham group and the normal saline control group, in which the BX229-30µg/Kg and BX229-100µg/Kg administration groups had better scores than the normal saline group and showed a certain dose-dependent trend, and the total score of the Garcia JH-15 score in the BX229-100µg/Kg administration group was 1 point higher than that in the normal saline group. From an analysis of the results of Garcia JH-15 scoring on D14, there was a significant difference in D14 scores between the sham group and the normal saline solvent control group, in which the BX229-30µg/Kg and BX229-100µg/Kg administration groups had better scores than that of the normal saline group and showed a dose-dependent relationship, and the Garcia JH-15 score of the BX229-100µg/Kg administration group was significantly different from that of the normal saline group (P < 0.05). See Figures 32 and 33.

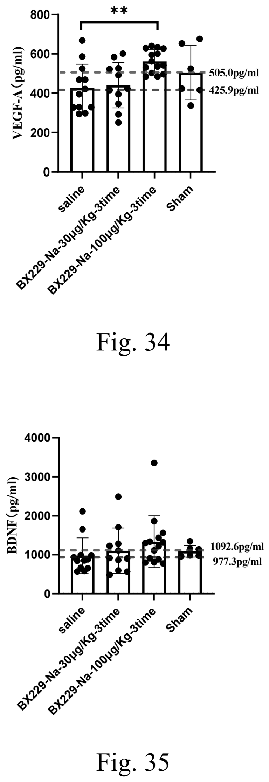

The results showed that expression levels of VEGF-A and BDNF proteins in the BX229Na-30µg/Kg three-dose group and the BX229Na-100µg/Kg three-dose group were higher than that in the normal saline group, and the expression level of VEGF-A protein in the BX229Na-100µg/Kg three-dose group were higher than that in the sham group, and significantly different from that in the normal saline group (FIG. 34, FIG. 35, Table 20, and Table 21). The expression of BDNF protein in BX229Na-100µg/Kg three-dose group was higher than that in sham group.

**Table 20: Changes in VEGF-A protein expression**

| Group | VEGF-A pg/ml |
|---|---|
| Saline | 426±115 |
| BX229Na-30 µg/Kg-3time | 441±102 |
| BX229Na-100 µg/Kg-3time | 562±56 |
| Sham | 505±122 |

**Table 21: Changes in BDNF protein expression**

| Group | BDNF pg/ml |
|---|---|
| Saline | 977±459 |
| BX229Na-30 µg/Kg-3time | 1103±582 |
| BX229Na-100 µg/Kg-3time | 1334±665 |
| Sham | 1093±151 |

### Example 14: In vivo Efficacy of Polypeptides BX225 and IRL1620 on Rat Model of pMACO

Test drugs: BX-225 and IRL1620, prepared from the Preparation Example of the present invention

Male SD rats, weighing 280-300 g, were used and acclimatized for at least 3 days. Focal cerebral ischemia was induced by middle cerebral artery occlusion with a suture method under isoflurane anesthesia. The animals were placed in a supine position, a midline incision was made, and the right common carotid artery, external carotid artery, and internal carotid artery were exposed. A 4.0 monofilament nylon suture was advanced from the external carotid artery toward the internal carotid artery lumen until resistance was felt (~20 mm), indicating middle cerebral artery occlusion. The nylon filament was allowed to stay in place to create a permanent model of focal cerebral ischemia. The day of modeling was defined as D1, the next day as D2, and so on. Since the experiment was not be completed in a single batch, and in order to prevent accidental errors, all operations in this experiment had to follow the principle of parallel operation, i.e., the experiment on each day needed to include animals from each group. Sequential numbering for surgical modeling was obtained by sorting the animals according to their body weight before the experiment. After the surgery, successfully modeled pMCAO rats were randomly assigned into a model group and various treatment groups. The detailed grouping of the experiment was as follows: (1) 15 rats in the 5 µg/Kg IRL1620 group with 9 administrations over 3 days; (2) 15 rats in the 6.6 µg/Kg BX225 group with 9 administrations over 3 days; (3) 15 rats in the 19.8 µg/Kg BX225 group with 3 administrations over 3 days; (4) 15 rats in the model (normal saline) group; and (5) 6 rats in the sham group (the sham group underwent the same procedure without vascular ligation and suture insertion). The administration was performed via tail vein injection (iv) with an administration volume of 2 mL/kg. The first administration was 2 hours after the surgery. Administration frequencies were single and multiple administrations per day according to the experimental design. Administrations were performed on D1, D3, and D6, respectively, as detailed in Table 22. The model group and the sham group were given the same volume of vehicle normal saline.

Results of a mRS-6 scoring showed that the BX225-6.6 µg/Kg three-dose group had a continuous improvement in mRS-6 scores from day 7, day 14, day 21, to day 30 (Table 23). The result on day 14 was close to a significant difference compared with the normal saline group and the IRL1620 three-dose group. The results on days 7, 21, and 30 showed significant differences compared with the normal saline group and the IRL1620 three-dose group (P<0.05, Table 23). Results of a Garcia JH scoring showed that Garcia JH scores of the BX225-6.6 µg/Kg three-dose group from day 7, day 14, day 21, to day 30 showed a continuous improvement trend, and the results on days 21 and 30 had statistical differences compared with the normal saline group (Tables 24 and 25).

**Table 22. Experimental Grouping and Administration Regimens for IRL620 and BX225**

| **Gro up** | **Num ber** | **pMCAO Establis hment** | **CPD** | **Dosage (µg/kg)** | **Volume (mL/kg)** | **Administration frequency** |
|---|---|---|---|---|---|---|
| 5 | 6 | Sham | vehicle | - | 2 | D1: 2 h, 4 h, 6 h after |
| 4 | 15 | pMCAO | vehicle | - | 2 | pMCAO; D3 and D6: |
| 1 | 15 | pMCAO | IRL1620 | 5 | 2 | administrated 3 times each at the same time points as on D0 |
| 1 | **15** | **pMCAO** | **BX225** | **6.6** | **2** | |
| 3 | **15** | **pMCAO** | **BX225** | **19.8** | **2** | D1: 2 h after pMCAO; D3 and D6: administrated once each at the same time points as on D1 |

**Table 23. mRS-6 Scores at 4 Weeks of Animals in Each Experimental Group (*P<0.05&saline)**

| Group | **mRS-7D** | **mRS-14D** | **mRS-21D** | **mRS-30D** |
|---|---|---|---|---|
| IRL1620-5µg/Kg-3Time | 2.54±2.03 | 2.77±2.66 | 2.72±2.71 | 2.52±2.87 |
| BX225-6.6µg/Kg-3Time | 1.67±0.50* | 1.17±0.50 | 0.75±0.41* | 0.50±0.31* |
| BX225-19.8µg/Kg-1Time | 3.14±2.14 | 3.90±2.61 | 3.73±2.81 | 3.65±2.91 |
| Saline | 3.33±2.22 | 2.89±2.57 | 2.70±2.72 | 2.59±2.81 |
| Sham | 0.00±0.00 | 0.00±0.00 | 0.06±0.15 | 0.04±0.10 |

**Table 24. Garcia JH Scores at Day 21 for Each Experimental Group (*P<0.05&saline)**

| **Group-21D** | **5 min Spontaneous Activity** | **Body Symmetry** | **Forelimb Extension Movement** | **Cage Climbi ng** | **Body Tactile Response on Both Sides** | **Beard Touch Reaction on Both Sides** | **Total Score** |
|---|---|---|---|---|---|---|---|
| 4-saline | 1.77±1.48 | 0.86±0.76 | 1.04±0.89 | 1.96± 0.83 | 1.85±0.73 | 1.90±0.76 | 9.38± 5.32 |
| 1-IRL1620-5µg/Kg-3Time | 1.77±1.48 | 0.90±0.80 | 1.26±1.05 | 2.03± 0.86 | 1.79±0.70 | 1.95±0.82 | 9.70± 5.58 |
| 2-BX225-6.6µg/Kg-3Time | 2.89±0.33 | 1.74±0.48 | 1.87±0.52 | 2.30± 0.71 | 2.50±0.23 | 2.70±0.25 | 13.99 ±1.49 * |
| 3-BX225-19.8µg/Kg-1Time | 1.17±1.47 | 0.63±0.80 | 0.80±1.01 | 1.69± 0.85 | 1.60±0.77 | 1.63±0.80 | 7.51± 5.58 |
| sham | 3.00±0.00 | 2.98±0.05 | 3.00±0.00 | 2.98± 0.05 | 2.98±0.05 | 2.98±0.05 | 17.92 ±0.15 |

**Table 25 Garcia JH Scores on Day 30 for Each Experimental Group (*P<0.05&saline)**

| **Group-30D** | **5 min Spontaneous Activity** | **Body Symmetry** | **Forelimb Extension Movement** | **Cage Climbing** | **Body Tactile Response on Both Sides** | **Beard Touch Reaction on Both Sides** | **Total Score** |
|---|---|---|---|---|---|---|---|
| 4-saline | 1.69±1.44 | 1.21±1.00 | 1.38±1.15 | 2.09±0.91 | 1.70±0.59 | 1.81±0.68 | 9.88± 5.66 |
| 1-IRL 1620-5µg/Kg-3Time | 1.77±1.48 | 0.90±0.80 | 1.26±1.05 | 2.03±0.86 | 1.79±0.70 | 1.95±0.82 | 9.70± 5.58 |
| 2-BX225-6.6µg/Kg-3Time | 3.00±0.00 | 2.07±0.29 | 2.25±0.23 | 2.81±0.15 | 2.29±0.18 | 2.39±0.13 | 14.81 ±0.56 * |
| 3-BX225-19.8µg/Kg-1Time | 1.25±1.54 | 0.78±0.98 | 0.93+1.15 | 1.79±0.98 | 1.46±0.59 | 1.50±0.65 | 7.71± 5.82 |
| sham | 2.83±0.41 | 2.98±0.05 | 3.00±0.00 | 2.96±0.10 | 2.69±0.37 | 2.75±0.18 | 17.21 ±1.04 |

The polypeptides and use thereof provided by the present invention are described in detail above. The principles and implementations of the present invention have been elucidated with reference to specific examples, the description of which is merely intended to assist in understanding the method of the invention and its core idea. It should be noted that those skilled in the art, without departing from the principles of the invention, may make several variations and modifications of the invention, which also fall within the scope of the claims of the invention.

Sequence Listing
<?xml version="1.0" encoding="UTF-8"?>
<!DOCTYPE ST26SequenceListing SYSTEM "ST26SequenceListing_V1_3.dtd" PUBLIC "-
//WIPO//DTD Sequence Listing 1.3//EN">
<ST26SequenceListing productionDate="2024-04-19" softwareVersion="2.3.0"
softwareName="WIPO Sequence" fileName="OP240340.xml" dtdVersion="V1_3"
nonEnglishFreeTextLanguageCode="zh"
originalFreeTextLanguageCode="zh"><ApplicantFileReference>OP240340</ApplicantFileRefer
ence><EarliestPriorityApplicationIdentification><IPOfficeCode>CN</IPOfficeCode><Applicati
onNumberText>202310461975.3</ApplicationNumberText><FilingDate>2023-04-
26</FilingDate></EarliestPriorityApplicationIdentification><ApplicantName
languageCode="zh">Biocells (Beijing) Biotech Co.,
Ltd.</ApplicantName><ApplicantNameLatin>Biocells (Beijing) Biotech Co.,
Ltd.</ApplicantNameLatin><InventionTitle languageCode="zh">POLYPEPTIDE AND USE
THEREOF</InventionTitle><SequenceTotalQuantity>88</SequenceTotalQuantity><SequenceD
ata
sequenceIDNumber="1"><INSDSeq><INSDSeq_length>14</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q1"><INSDQualifier_name>organism</INSDQualifier_name><INSDQualifier_value>synth
etic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q2"><INSDQualifier_name>note</INSDQualifier_name><NonEnglishQualifier_value>X IS
ASPARTIC ACID WITH A SUCCINYL MONOACYL
MODIFICATION</NonEnglishQualifier_value></INSDQualifier></INSDFeature_quals></INS
DFeature></INSDSeq_feature-
table><INSDSeq_sequence>XEEAVYFAHLDIIW</INSDSeq_sequence></INSDSeq></Sequen
ceData><SequenceData
sequenceIDNumber="2"><INSDSeq><INSDSeq_length>14</INSDSeq_length>
<INSDSeq_moltype>AA</INSDSeq_moltype> <INSDSeq_division>PAT</INSDSeq_division>
<INSDSeq_feature-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier

id="q3"><INSDQualifier_name>organism</INSDQualifier_name><INSDQualifier_value>synth
etic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q4"><INSDQualifier_name>note</INSDQualifier_name><NonEnglishQualifier_value>X IS
ASPARTIC ACID WITH A SUCCINYL MONOACYL
MODIFICATION</NonEnglishQualifier_value></INSDQualifier></INSDFeature_quals></INS
DFeature></INSDSeq_feature-table>
<INSDSeq_sequence>XEEAVYFAHKDIIW</INSDSeq_sequence>
</INSDSeq></SequenceData><SequenceData
sequenceIDNumber="3"><INSDSeq><INSDSeq_length>14</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q5"><INSDQualifier_name>organism</INSDQualifier_name><INSDQualifier_value>synth
etic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q6"><INSDQualifier_name>note</INSDQualifier_name><NonEnglishQualifier_value>X IS
ASPARTIC ACID WITH A FUMARYL MONOACYL
MODIFICATION</NonEnglishQualifier_value></INSDQualifier></INSDFeature_quals></INS
DFeature></INSDSeq_feature-
table><INSDSeq_sequence>XEEAVYFAHKDIIW</INSDSeq_sequence></INSDSeq></Sequen
ceData><SequenceData
sequenceIDNumber="4"><INSDSeq><INSDSeq_length>14</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q7"><INSDQualifier_name>organism</INSDQualifier_name><INSDQualifier_value>synth
etic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q8"><INSDQualifier_name>note</INSDQualifier_name><NonEnglishQualifier_value>X IS
ASPARTIC ACID WITH A SUCCINYL MONOACYL

MODIFICATION</NonEnglishQualifier_value></INSDQualifier></INSDFeature_quals></INS
DFeature></INSDSeq_feature-
table><INSDSeq_sequence>XEEAVYFAHLDVIW</INSDSeq_sequence></INSDSeq></Sequen
ceData><SequenceData
sequenceIDNumber="5"><INSDSeq><INSDSeq_length>14</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q9"><INSDQualifier_name>organism</INSDQualifier_name><INSDQualifier_value>synth
etic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q10"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>12</INSDFeature_location><INSDFeature_quals><INSDQualifier
id="q11"><INSDQualifier_name>note</INSDQualifier_name><NonEnglishQualifier_value>X
IS 2-AMINOBUTYRIC
ACID</NonEnglishQualifier_value></INSDQualifier></INSDFeature_quals></INSDFeature></I
NSDSeq_feature-
table><INSDSeq_sequence>XEEAVYFAHLDXIW</INSDSeq_sequence></INSDSeq></Sequen
ceData><SequenceData
sequenceIDNumber="6"><INSDSeq><INSDSeq_length>14</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q12"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q13"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l

ocation>10</INSDFeature_location><INSDFeature_quals><INSDQualifier
id="q14"><INSDQualifier_name>note</INSDQualifier_name><NonEnglishQualifier_value>X
is lysine with an AEEA-AEEA-γE-ODDA
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="7"><INSDSeq><INSDSeq_length>14</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q15"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q16"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature></INSDSeq_feature-
table><INSDSeq_sequence>XEEAKYFAHLDIIW</INSDSeq_sequence></INSDSeq></Sequen
ceData><SequenceData
sequenceIDNumber="8"><INSDSeq><INSDSeq_length>14</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q17"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q18"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature></INSDSeq_feature-
table><INSDSeq_sequence>XEEEVYFAHKDIIW</INSDSeq_sequence></INSDSeq></Sequen
ceData><SequenceData

sequenceIDNumber="9"><INSDSeq><INSDSeq_length>14</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q19"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q20"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>CROSSLNK</INSDFeature_key><INSDFeature_
location>2..5</INSDFeature_location><INSDFeature_quals><INSDQualifier
id="q21"><INSDQualifier_name>note</INSDQualifier_name><NonEnglishQualifier_value>Glu
tamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHLDIIW</INSDSeq_sequence></INSDSeq></Sequen
ceData><SequenceData
sequenceIDNumber="10"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q22"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q23"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>5</INSDFeature_location><INSDFeature_quals><INSDQualifier
id="q24"><INSDQualifier_name>note</INSDQualifier_name><NonEnglishQualifier_value>X
is 2-aminobutyric
acid</NonEnglishQualifier_value></INSDQualifier></INSDFeature_quals></INSDFeature><IN

SDFeature><INSDFeature_key>CROSSLNK</INSDFeature_key><INSDFeature_location>2..5<
/INSDFeature_location><INSDFeature_quals><INSDQualifier
id="q25"><INSDQualifier_name>note</INSDQualifier_name><NonEnglishQualifier_value>Glu
tamic acid at position 2 is connected to 2-aminobutyric acid at position 5 via an intrastrand
crosslinking</NonEnglishQualifier_value></INSDQualifier></INSDFeature_quals></INSDFeatu
re></INSDSeq_feature-
table><INSDSeq_sequence>XEEAXYFAHLDIIW</INSDSeq_sequence></INSDSeq></Sequen
ceData><SequenceData
sequenceIDNumber="11"><INSDSeq><INSDSeq_length>14</INSDSeq_length><INSDSeq_mol
type>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q26"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q27"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>CROSSLNK</INSDFeature_key><INSDFeature_
location>4..10</INSDFeature_location><INSDFeature_quals><INSDQualifier
id="q28"><INSDQualifier_name>note</INSDQualifier_name><NonEnglishQualifier_value>Glu
tamic acid at position 4 is connected to lysine at position 10 via an intrastrand crosslinking
</NonEnglishQualifier_value></INSDQualifier></INSDFeature_quals></INSDFeature></INSD
Seq_feature-
table><INSDSeq_sequence>XEEEVYFAHKDIIW</INSDSeq_sequence></INSDSeq></Sequen
ceData><SequenceData
sequenceIDNumber="12"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q29"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier

id="q30"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q31"><INSDQualifier_name>note</INSDQualifier_name><NonEnglishQualifier_value>X
is ornithine with an AEEA-AEEA-γE-ODDA
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="13"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q32"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q33"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature></INSDSeq_feature-
table><INSDSeq_sequence>XEEEVYFAHKDIIW</INSDSeq_sequence></INSDSeq></Sequen
ceData><SequenceData
sequenceIDNumber="14"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q34"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q35"><INSDQualifier_name>note</INSDQualifier_name><NonEnglishQualifier_value>X
IS ASPARTIC ACID WITH A SUCCINYL MONOACYL

MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q36"><INSDQualifier_name>note</INSDQualifier_name><NonEnglishQualifier_value>X
IS
ORNITHINE</NonEnglishQualifier_value></INSDQualifier></INSDFeature_quals></INSDFeat
ure></INSDSeq_feature-
table><INSDSeq_sequence>XEEEVYFAHXDIIW</INSDSeq_sequence></INSDSeq></Sequen
ceData><SequenceData
sequenceIDNumber="15"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q37"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q38"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q39"><INSDQualifier_name>note</INSDQualifier_name><NonEnglishQualifier_value>X
is lysine with an AEEA-AEEA-γE-ODDA
modification</NonEnglishQualifier_value></INSDQualifier></INSDFeature_quals></INSDFeat
ure></INSDSeq_feature-
table><INSDSeq_sequence>XEEEVYFAHXDIIW</INSDSeq_sequence></INSDSeq></Sequen
ceData><SequenceData
sequenceIDNumber="16"><INSDSeq><INSDSeq_length>15</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..15</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q40"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS

DFeature_location><INSDFeature_quals><INSDQualifier
id="q41"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>DISULFID</INSDFeature_key><INSDFeature_l
ocation>3..6</INSDFeature_location><INSDFeature_quals><INSDQualifier
id="q42"><INSDQualifier_name>note</INSDQualifier_name><NonEnglishQualifier_value>Cys
teine at position 3 is connected to cysteine at position 6 via interchain disulfide
bonds</NonEnglishQualifier_value></INSDQualifier></INSDFeature_quals></INSDFeature></I
NSDSeq_feature-
table><INSDSeq_sequence>XECEECYFAHKDIIW</INSDSeq_sequence></INSDSeq></Seque
nceData><SequenceData
sequenceIDNumber="17"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q43"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q44"><INSDQualifier_name>note</INSDQualifier_name><NonEnglishQualifier_value>X
IS ASPARTIC ACID WITH A FUMARYL MONOMETHYL MONOACYL
MODIFICATION</NonEnglishQualifier_value></INSDQualifier></INSDFeature_quals></INS
DFeature></INSDSeq_feature-
table><INSDSeq_sequence>XEEEVYFAHKDIIW</INSDSeq_sequence></INSDSeq></Sequen
ceData><SequenceData
sequenceIDNumber="18"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q45"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q46"><INSDQualifier_name>note</INSDQualifier_name><NonEnglishQualifier_value>X

IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q47"><INSDQualifier_name>note</INSDQualifier_name><NonEnglishQualifier_value>X
IS
ORNITHINE</NonEnglishQualifier_value></INSDQualifier></INSDFeature_quals></INSDFeat
ure></INSDSeq_feature-
table><INSDSeq_sequence>XEEEVYFAHXDIIW</INSDSeq_sequence></INSDSeq></Sequen
ceData><SequenceData
sequenceIDNumber="19"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q48"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q49"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q50"><INSDQualifier_name>note</INSDQualifier_name><NonEnglishQualifier_value>X
is lysine with an AEEA-AEEA-γE-ODDA
modification</NonEnglishQualifier_value></INSDQualifier></INSDFeature_quals></INSDFeat
ure></INSDSeq_feature-
table><INSDSeq_sequence>XEEEVYFAHXDIIW</INSDSeq_sequence></INSDSeq></Sequen
ceData><SequenceData
sequenceIDNumber="20"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q51"><INSDQualifier_name>organism</INSDQualifier_name><INSDQualifier_value>synt
hetic
construct</INSDQualifier_value></INSDQualifier></INSDFeature_quals></INSDFeature><INS

DFeature><INSDFeature_key>MOD_RES</INSDFeature_key><INSDFeature_location>1</INS
DFeature_location><INSDFeature_quals><INSDQualifier
id="q52"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q53"><INSDQualifier_name>note</INSDQualifier_name><NonEnglishQualifier_value>X
is ornithine with an AEEA-AEEA-γE-ODDA
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="21"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q54"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q55"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>12</INSDFeature_location><INSDFeature_quals><INSDQualifier
id="q56"><INSDQualifier_name>note</INSDQualifier_name><NonEnglishQualifier_value>X
IS 2-AMINOBUTYRIC
ACID</NonEnglishQualifier_value></INSDQualifier></INSDFeature_quals></INSDFeature><I
NSDFeature><INSDFeature_key>CROSSLNK</INSDFeature_key><INSDFeature_location>4..
8</INSDFeature_location><INSDFeature_quals><INSDQualifier
id="q57"><INSDQualifier_name>note</INSDQualifier_name><NonEnglishQualifier_value>Glu
tamic acid at position 4 is connected to lysine at position 8 via an intrastrand crosslinking
</NonEnglishQualifier_value></INSDQualifier></INSDFeature_quals></INSDFeature></INSD
Seq_feature-
table><INSDSeq_sequence>XEEEVYFKHKDXIW</INSDSeq_sequence></INSDSeq></Sequen
ceData><SequenceData
sequenceIDNumber="22"><INSDSeq><INSDSeq_length>14</INSDSeq_length><INSDSeq_mo
ltype>AA</INSDSeq_moltype><INSDSeq_division>PAT</INSDSeq_division><INSDSeq_featu

re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q58"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q59"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>12</INSDFeature_location><INSDFeature_quals><INSDQualifier
id="q60"><INSDQualifier_name>note</INSDQualifier_name><NonEnglishQualifier_value>X
IS 2-AMINOBUTYRIC
ACID</NonEnglishQualifier_value></INSDQualifier></INSDFeature_quals></INSDFeature><I
NSDFeature><INSDFeature_key>CROSSLNK</INSDFeature_key><INSDFeature_location>2..
5</INSDFeature_location><INSDFeature_quals><INSDQualifier
id="q61"><INSDQualifier_name>note</INSDQualifier_name><NonEnglishQualifier_value>Glu
tamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHLDXIW</INSDSeq_sequence></INSDSeq></Sequen
ceData><SequenceData
sequenceIDNumber="23"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q62"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q63"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>12</INSDFeature_location><INSDFeature_quals><INSDQualifier

id="q64"><INSDQualifier_name>note</INSDQualifier_name><NonEnglishQualifier_value>X
IS 2-AMINOBUTYRIC
ACID</NonEnglishQualifier_value></INSDQualifier></INSDFeature_quals></INSDFeature><I
NSDFeature><INSDFeature_key>CROSSLNK</INSDFeature_key><INSDFeature_location>2..
5</INSDFeature_location><INSDFeature_quals><INSDQualifier
id="q65"><INSDQualifier_name>note</INSDQualifier_name><NonEnglishQualifier_value>Glu
tamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHLDXIW</INSDSeq_sequence></INSDSeq></Sequen
ceData><SequenceData
sequenceIDNumber="24"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q66"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q67"><INSDQualifier_name>note</INSDQualifier_name><NonEnglishQualifier_value>X
IS ASPARTIC ACID WITH A FUMARYL MONOMETHYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>12</INSDFeature_location><INSDFeature_quals><INSDQualifier
id="q68"><INSDQualifier_name>note</INSDQualifier_name><NonEnglishQualifier_value>X
IS 2-AMINOBUTYRIC
ACID</NonEnglishQualifier_value></INSDQualifier></INSDFeature_quals></INSDFeature><I
NSDFeature><INSDFeature_key>CROSSLNK</INSDFeature_key><INSDFeature_location>2..
5</INSDFeature_location><INSDFeature_quals><INSDQualifier
id="q69"><INSDQualifier_name>note</INSDQualifier_name><NonEnglishQualifier_value>Glu
tamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHLDXIW</INSDSeq_sequence></INSDSeq></Sequen
ceData><SequenceData
sequenceIDNumber="25"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1

..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q70"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q71"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>4</INSDFeature_location><INSDFeature_quals><INSDQualifier
id="q72"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEXVYFAHLDIIW</INSDSeq_sequence></INSDSeq></Sequen
ceData><SequenceData
sequenceIDNumber="26"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q73"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q74"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>5</INSDFeature_location><INSDFeature_quals><INSDQualifier
id="q75"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAXYFAHLDIIW</INSDSeq_sequence></INSDSeq></Sequen
ceData><SequenceData
sequenceIDNumber="27"><INSDSeq><INSDSeq_length>14</INSDSeq_length><INSDSeq_mo

ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q76"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q77"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>8</INSDFeature_location><INSDFeature_quals><INSDQualifier
id="q78"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAVYFXHLDIIW</INSDSeq_sequence></INSDSeq></Sequen
ceData><SequenceData
sequenceIDNumber="28"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q79"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q80"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q81"><INSDQualifier_name>note</INSDQualifier_name><NonEnglishQualifier_value>X
is lysine with a γE-AEEA-AEEA-C18
modification</NonEnglishQualifier_value></INSDQualifier></INSDFeature_quals></INSDFeat
ure></INSDSeq_feature-

table><INSDSeq_sequence>XEEAVYFAHXDIIW</INSDSeq_sequence></INSDSeq></Sequen
ceData><SequenceData
sequenceIDNumber="29"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q82"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q83"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q84"><INSDQualifier_name>note</INSDQualifier_name><NonEnglishQualifier_value>X
is lysine with a gamma E-AEEA-AEEA-C16
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="30"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q85"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q86"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q87"><INSDQualifier_name>note</INSDQualifier_name><NonEnglishQualifier_value>X

is lysine with a gamma E-AEEA-AEEA-C12
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="31"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q88"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q89"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q90"><INSDQualifier_name>note</INSDQualifier_name><NonEnglishQualifier_value>X
is lysine with a gamma E-AEEA-AEEA-B
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="32"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q91"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q92"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS

DFeature><INSDFeature><INSDFeature_key>MOD_RES</INSDFeature_key><INSDFeature_l
ocation>10</INSDFeature_location><INSDFeature_quals><INSDQualifier
id="q93"><INSDQualifier_name>note</INSDQualifier_name><NonEnglishQualifier_value>X
is lysine with a gamma E-AEEA-AEEA-ODDA
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="33"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q94"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q95"><INSDQualifier_name>note</INSDQualifier_name><NonEnglishQualifier_value>X
IS ASPARTIC ACID WITH A FUMARYL MONOMETHYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q96"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="34"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q97"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier

id="q98"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>CROSSLNK</INSDFeature_key><INSDFeature_
location>2..5</INSDFeature_location><INSDFeature_quals><INSDQualifier
id="q99"><INSDQualifier_name>note</INSDQualifier_name><NonEnglishQualifier_value>Glu
tamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHLDVIW</INSDSeq_sequence></INSDSeq></Sequen
ceData><SequenceData
sequenceIDNumber="35"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q100"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q101"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q102"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure><INSDFeature><INSDFeature_key>CROSSLNK</INSDFeature_key><INSDFeature_locati
on>2..5</INSDFeature_location><INSDFeature_quals><INSDQualifier
id="q103"><INSDQualifier_name>note</INSDQualifier_name><NonEnglishQualifier_value>Gl
utamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHXDIIW</INSDSeq_sequence></INSDSeq></Sequen
ceData><SequenceData
sequenceIDNumber="36"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1

..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q104"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q105"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q106"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAVYFAHXDVIW</INSDSeq_sequence></INSDSeq></Seque
nceData><SequenceData
sequenceIDNumber="37"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q107"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q108"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q109"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure><INSDFeature><INSDFeature_key>CROSSLNK</INSDFeature_key><INSDFeature_locati
on>2..5</INSDFeature_location><INSDFeature_quals><INSDQualifier
id="q110"><INSDQualifier_name>note</INSDQualifier_name><NonEnglishQualifier_value>Gl
utamic acid at position 2 is connected to lysine at position 5 via an intrastrand

crosslinking</NonEnglishQualifier_value></INSDQualifier></INSDFeature_quals></INSDFeatu
re></INSDSeq_feature-
table><INSDSeq_sequence>XEEAKYFAHXDVIW</INSDSeq_sequence></INSDSeq></Seque
nceData><SequenceData
sequenceIDNumber="38"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q111"><INSDQualifier_name>organism</INSDQualifier_name><INSDQualifier_value>synt
hetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q112"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q113"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="39"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q114"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q115"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l

ocation>10</INSDFeature_location><INSDFeature_quals><INSDQualifier
id="q116"><INSDQualifier_name>note</INSDQualifier_name><NonEnglishQualifier_value>X
IS LYSINE WITH A CPAK-MPA
MODIFICATION</NonEnglishQualifier_value></INSDQualifier></INSDFeature_quals></INS
DFeature></INSDSeq_feature-
table><INSDSeq_sequence>XEEAVYFAHXDIIW</INSDSeq_sequence></INSDSeq></Sequen
ceData><SequenceData
sequenceIDNumber="40"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q117"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q118"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>CROSSLNK</INSDFeature_key><INSDFeature_
location>2..5</INSDFeature_location><INSDFeature_quals><INSDQualifier
id="q119"><INSDQualifier_name>note</INSDQualifier_name><NonEnglishQualifier_value>Gl
utamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHLDVIW</INSDSeq_sequence></INSDSeq></Sequen
ceData><SequenceData
sequenceIDNumber="41"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q120"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q121"><INSDQualifier_name>note</INSDQualifier_name><NonEnglishQualifier_value>X

IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q122"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure><INSDFeature><INSDFeature_key>CROSSLNK</INSDFeature_key><INSDFeature_locati
on>2..5</INSDFeature_location><INSDFeature_quals><INSDQualifier
id="q123"><INSDQualifier_name>note</INSDQualifier_name><NonEnglishQualifier_value>Gl
utamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHXDVIW</INSDSeq_sequence></INSDSeq></Seque
nceData><SequenceData
sequenceIDNumber="42"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q124"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q125"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q126"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAVYFAHXDVIW</INSDSeq_sequence></INSDSeq></Seque
nceData><SequenceData
sequenceIDNumber="43"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol

_type</INSDQualifier_name><INSDQualifier_value>protein</INSDQualifier_value></INSDQu
alifier><INSDQualifier
id="q127"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q128"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>CROSSLNK</INSDFeature_key><INSDFeature_
location>2..5</INSDFeature_location><INSDFeature_quals><INSDQualifier
id="q129"><INSDQualifier_name>note</INSDQualifier_name><NonEnglishQualifier_value>Gl
utamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHLDIIW</INSDSeq_sequence></INSDSeq></Sequen
ceData><SequenceData
sequenceIDNumber="44"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q130"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q131"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q132"><INSDQualifier_name>note</INSDQualifier_name><NonEnglishQualifier_value>X
is lysine with a gamma E-AEEA-AEEA-ODDA
modification</NonEnglishQualifier_value></INSDQualifier></INSDFeature_quals></INSDFeat
ure><INSDFeature><INSDFeature_key>CROSSLNK</INSDFeature_key><INSDFeature_locati
on>2..5</INSDFeature_location><INSDFeature_quals><INSDQualifier
id="q133"><INSDQualifier_name>note</INSDQualifier_name><NonEnglishQualifier_value>Gl
utamic acid at position 2 is connected to lysine at position 5 via an intrastrand
crosslinking</NonEnglishQualifier_value></INSDQualifier></INSDFeature_quals></INSDFeatu

re></INSDSeq_feature-
table><INSDSeq_sequence>XEEAKYFAHXDIIW</INSDSeq_sequence></INSDSeq></Sequen
ceData><SequenceData
sequenceIDNumber="45"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q134"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q135"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q136"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure><INSDFeature><INSDFeature_key>MOD_RES</INSDFeature_key><INSDFeature_locatio
n>12</INSDFeature_location><INSDFeature_quals><INSDQualifier
id="q137"><INSDQualifier_name>note</INSDQualifier_name><NonEnglishQualifier_value>X
IS 2-AMINOBUTYRIC
ACID</NonEnglishQualifier_value></INSDQualifier></INSDFeature_quals></INSDFeature><I
NSDFeature><INSDFeature_key>CROSSLNK</INSDFeature_key><INSDFeature_location>2..
5</INSDFeature_location><INSDFeature_quals><INSDQualifier
id="q138"><INSDQualifier_name>note</INSDQualifier_name><NonEnglishQualifier_value>Gl
utamic acid at position 2 is connected to lysine at position 5 via an intrastrand
crosslinking</NonEnglishQualifier_value></INSDQualifier></INSDFeature_quals></INSDFeatu
re></INSDSeq_feature-
table><INSDSeq_sequence>XEEAKYFAHXDXIW</INSDSeq_sequence></INSDSeq></Seque
nceData><SequenceData
sequenceIDNumber="46"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier

id="q139"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q140"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature></INSDSeq_feature-
table><INSDSeq_sequence>XEEAKYFAHLDIIW</INSDSeq_sequence></INSDSeq></Sequen
ceData><SequenceData
sequenceIDNumber="47"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q141"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q142"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature></INSDSeq_feature-
table><INSDSeq_sequence>XEEAKYFAHLDVIW</INSDSeq_sequence></INSDSeq></Sequen
ceData><SequenceData
sequenceIDNumber="48"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q143"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q144"><INSDQualifier_name>note</INSDQualifier_name><NonEnglishQualifier_value>X
IS ASPARTIC ACID WITH A FUMARYL MONOACYL

MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q145"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAKYFAHXDIIW</INSDSeq_sequence></INSDSeq></Sequen
ceData><SequenceData
sequenceIDNumber="49"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q146"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q147"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q148"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAKYFAHXDVIW</INSDSeq_sequence></INSDSeq></Seque
nceData><SequenceData
sequenceIDNumber="50"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q149"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS

DFeature_location><INSDFeature_quals><INSDQualifier
id="q150"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>5</INSDFeature_location><INSDFeature_quals><INSDQualifier
id="q151"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAXYFAHLDIIW</INSDSeq_sequence></INSDSeq></Sequen
ceData><SequenceData
sequenceIDNumber="51"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q152"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q153"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>5</INSDFeature_location><INSDFeature_quals><INSDQualifier
id="q154"><INSDQualifier_name>note</INSDQualifier_name><NonEnglishQualifier_value>X
is lysine with a γE-AEEA-AEEA-ODDA
modification</NonEnglishQualifier_value></INSDQualifier></INSDFeature_quals></INSDFeat
ure></INSDSeq_feature-
table><INSDSeq_sequence>XEEAXYFAHLDVIW</INSDSeq_sequence></INSDSeq></Sequen
ceData><SequenceData
sequenceIDNumber="52"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q155"><INSDQualifier_name>organism</INSDQualifier_name><INSDQualifier_value>syn

thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q156"><INSDQualifier_name>note</INSDQualifier_name><NonEnglishQualifier_value>X
IS ASPARTIC ACID WITH A SORBYL MONOACYL
MODIFICATION</NonEnglishQualifier_value></INSDQualifier></INSDFeature_quals></INS
DFeature></INSDSeq_feature-
table><INSDSeq_sequence>XEEAVYFAHLDIIW</INSDSeq_sequence></INSDSeq></Sequen
ceData><SequenceData
sequenceIDNumber="53"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q157"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q158"><INSDQualifier_name>note</INSDQualifier_name><NonEnglishQualifier_value>X
IS ASPARTIC ACID WITH A TRANS,TRANS-MUCONYL MONOACYL
MODIFICATION</NonEnglishQualifier_value></INSDQualifier></INSDFeature_quals></INS
DFeature></INSDSeq_feature-
table><INSDSeq_sequence>XEEAVYFAHLDIIW</INSDSeq_sequence></INSDSeq></Sequen
ceData><SequenceData
sequenceIDNumber="54"><INSDSeq><INSDSeq_length>13</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..13</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q159"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q160"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS

DFeature><INSDFeature><INSDFeature_key>MOD_RES</INSDFeature_key><INSDFeature_l
ocation>13</INSDFeature_location><INSDFeature_quals><INSDQualifier
id="q161"><INSDQualifier_name>note</INSDQualifier_name><NonEnglishQualifier_value>X
IS ISOLEUCINE WITH A 2-AMINO-3-(NAPHTHALEN-2-YL) PROPANOIC
MODIFICATION</NonEnglishQualifier_value></INSDQualifier></INSDFeature_quals></INS
DFeature></INSDSeq_feature-
table><INSDSeq_sequence>XEEAVYFAHKDIX</INSDSeq_sequence></INSDSeq></Sequenc
eData><SequenceData
sequenceIDNumber="55"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q162"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q163"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q164"><INSDQualifier_name>note</INSDQualifier_name><NonEnglishQualifier_value>X
IS L-2-AMINO-4-GUANIDINOBUTYRIC
ACID</NonEnglishQualifier_value></INSDQualifier></INSDFeature_quals></INSDFeature></I
NSDSeq_feature-
table><INSDSeq_sequence>XEEAVYFAHXDIIW</INSDSeq_sequence></INSDSeq></Sequen
ceData><SequenceData
sequenceIDNumber="56"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q165"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier

id="q166"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q167"><INSDQualifier_name>note</INSDQualifier_name><NonEnglishQualifier_value>X
IS
ORNITHINE</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="57"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q168"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q169"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q170"><INSDQualifier_name>note</INSDQualifier_name><NonEnglishQualifier_value>X
IS
CITRULLINE</NonEnglishQualifier_value></INSDQualifier></INSDFeature_quals></INSDFe
ature></INSDSeq_feature-
table><INSDSeq_sequence>XEEAVYFAHXDIIW</INSDSeq_sequence></INSDSeq></Sequen
ceData><SequenceData
sequenceIDNumber="58"><INSDSeq><INSDSeq_length>9</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..9</INSDFeature_location><INSDFeature_quals><INSDQualifier><INSDQualifier_name>mol_
type</INSDQualifier_name><INSDQualifier_value>protein</INSDQualifier_value></INSDQual
ifier><INSDQualifier
id="q258"><INSDQualifier_name>organism</INSDQualifier_name><INSDQualifier_value>syn
thetic

construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q172"><INSDQualifier_name>note</INSDQualifier_name><NonEnglishQualifier_value>X
is Suc-DE cyclo
(CEAC)Y</NonEnglishQualifier_value></INSDQualifier></INSDFeature_quals></INSDFeature
></INSDSeq_feature-
table><INSDSeq_sequence>XFAHLDIIW</INSDSeq_sequence></INSDSeq></SequenceData>
<SequenceData
sequenceIDNumber="59"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q174"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q175"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature></INSDSeq_feature-
table><INSDSeq_sequence>XEEAVYFAHKDVIW</INSDSeq_sequence></INSDSeq></Seque
nceData><SequenceData
sequenceIDNumber="60"><INSDSeq><INSDSeq_length>13</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..13</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q260"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q177"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l

ocation>11</INSDFeature_location><INSDFeature_quals><INSDQualifier
id="q261"><INSDQualifier_name>note</INSDQualifier_name><NonEnglishQualifier_value>X
is aspartic acid with a 2-Abu
modification</NonEnglishQualifier_value></INSDQualifier></INSDFeature_quals></INSDFeat
ure></INSDSeq_feature-
table><INSDSeq_sequence>XEEAVYFAHKXIW</INSDSeq_sequence></INSDSeq></Sequenc
eData><SequenceData
sequenceIDNumber="61"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q179"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q180"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>12</INSDFeature_location><INSDFeature_quals><INSDQualifier
id="q181"><INSDQualifier_name>note</INSDQualifier_name><NonEnglishQualifier_value>X
is isoleucine with a N-Me
modification</NonEnglishQualifier_value></INSDQualifier></INSDFeature_quals></INSDFeat
ure></INSDSeq_feature-
table><INSDSeq_sequence>XEEAVYFAHLDXIW</INSDSeq_sequence></INSDSeq></Sequen
ceData><SequenceData
sequenceIDNumber="62"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q182"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q183"><INSDQualifier_name>note</INSDQualifier_name><NonEnglishQualifier_value>X

IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q184"><INSDQualifier_name>note</INSDQualifier_name><NonEnglishQualifier_value>X
is leucine with a N-Me
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="63"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q185"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q186"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q187"><INSDQualifier_name>note</INSDQualifier_name><NonEnglishQualifier_value>X
is leucine with a N-Me
modification</NonEnglishQualifier_value></INSDQualifier></INSDFeature_quals></INSDFeat
ure><INSDFeature><INSDFeature_key>MOD_RES</INSDFeature_key><INSDFeature_locatio
n>12</INSDFeature_location><INSDFeature_quals><INSDQualifier
id="q188"><INSDQualifier_name>note</INSDQualifier_name><NonEnglishQualifier_value>X
is isoleucine with a N-Me
modification</NonEnglishQualifier_value></INSDQualifier></INSDFeature_quals></INSDFeat
ure></INSDSeq_feature-
table><INSDSeq_sequence>XEEAVYFAHXDXIW</INSDSeq_sequence></INSDSeq></Seque
nceData><SequenceData
sequenceIDNumber="64"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol

_type</INSDQualifier_name><INSDQualifier_value>protein</INSDQualifier_value></INSDQu
alifier><INSDQualifier
id="q189"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q190"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q191"><INSDQualifier_name>note</INSDQualifier_name><NonEnglishQualifier_value>X
is leucine with a N-Me
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="65"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q192"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q193"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q194"><INSDQualifier_name>note</INSDQualifier_name><NonEnglishQualifier_value>X
is leucine with a N-Me
modification</NonEnglishQualifier_value></INSDQualifier></INSDFeature_quals></INSDFeat
ure><INSDFeature><INSDFeature_key>MOD_RES</INSDFeature_key><INSDFeature_locatio
n>12</INSDFeature_location><INSDFeature_quals><INSDQualifier
id="q195"><INSDQualifier_name>note</INSDQualifier_name><NonEnglishQualifier_value>X
is isoleucine with a N-Me
modification</NonEnglishQualifier_value></INSDQualifier></INSDFeature_quals></INSDFeat

ure></INSDSeq_feature-
table><INSDSeq_sequence>XEEAVYFAHXDXIW</INSDSeq_sequence></INSDSeq></Seque
nceData><SequenceData
sequenceIDNumber="66"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q196"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q197"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature></INSDSeq_feature-
table><INSDSeq_sequence>XEEAVYFAHLDVIW</INSDSeq_sequence></INSDSeq></Sequen
ceData><SequenceData
sequenceIDNumber="67"><INSDSeq><INSDSeq_length>13</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..13</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q259"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q199"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>11</INSDFeature_location><INSDFeature_quals><INSDQualifier
id="q200"><INSDQualifier_name>note</INSDQualifier_name><NonEnglishQualifier_value>X
is aspartic acid with a 2-Abu
modification</NonEnglishQualifier_value></INSDQualifier></INSDFeature_quals></INSDFeat
ure></INSDSeq_feature-
table><INSDSeq_sequence>XEEAVYFAHLXIW</INSDSeq_sequence></INSDSeq></Sequenc

eData><SequenceData
sequenceIDNumber="68"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q201"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q202"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>CROSSLNK</INSDFeature_key><INSDFeature_
location>4..8</INSDFeature_location><INSDFeature_quals><INSDQualifier
id="q203"><INSDQualifier_name>note</INSDQualifier_name><NonEnglishQualifier_value>Gl
utamic acid at position 4 is connected to glutamic acid at position 8 via an intrastrand
crosslinking</NonEnglishQualifier_value></INSDQualifier></INSDFeature_quals></INSDFeatu
re></INSDSeq_feature-
table><INSDSeq_sequence>XEEEVYFEHKDIIW</INSDSeq_sequence></INSDSeq></Sequenc
eData><SequenceData
sequenceIDNumber="69"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q204"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q205"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>CROSSLNK</INSDFeature_key><INSDFeature_
location>4..10</INSDFeature_location><INSDFeature_quals><INSDQualifier
id="q206"><INSDQualifier_name>note</INSDQualifier_name><NonEnglishQualifier_value>Gl
utamic acid at position 4 is connected to lysine at position 10 via an intrastrand

crosslinking</NonEnglishQualifier_value></INSDQualifier></INSDFeature_quals></INSDFeatu
re></INSDSeq_feature-
table><INSDSeq_sequence>XEEEVYFKHKDIIW</INSDSeq_sequence></INSDSeq></Sequen
ceData><SequenceData
sequenceIDNumber="70"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q207"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q208"><INSDQualifier_name>note</INSDQualifier_name><NonEnglishQualifier_value>X
IS ASPARTIC ACID WITH A FERULIC
MODIFICATION</NonEnglishQualifier_value></INSDQualifier></INSDFeature_quals></INS
DFeature></INSDSeq_feature-
table><INSDSeq_sequence>XEEAVYFAHKDIIW</INSDSeq_sequence></INSDSeq></Sequen
ceData><SequenceData
sequenceIDNumber="71"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q209"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q210"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>4</INSDFeature_location><INSDFeature_quals><INSDQualifier
id="q211"><INSDQualifier_name>note</INSDQualifier_name><NonEnglishQualifier_value>X
is glutamic acid with a Biotin
modification</NonEnglishQualifier_value></INSDQualifier></INSDFeature_quals></INSDFeat
ure></INSDSeq_feature-

table><INSDSeq_sequence>XEEXVYFAHKDIIW</INSDSeq_sequence></INSDSeq></Sequen
ceData><SequenceData
sequenceIDNumber="72"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q212"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q213"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature></INSDSeq_feature-
table><INSDSeq_sequence>XEEAVYFAHLDPIW</INSDSeq_sequence></INSDSeq></Sequen
ceData><SequenceData
sequenceIDNumber="73"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q214"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q215"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>3</INSDFeature_location><INSDFeature_quals><INSDQualifier
id="q216"><INSDQualifier_name>note</INSDQualifier_name><NonEnglishQualifier_value>X
is glutamic acid with a C16
modification</NonEnglishQualifier_value></INSDQualifier></INSDFeature_quals></INSDFeat
ure></INSDSeq_feature-
table><INSDSeq_sequence>XEXAVYFAHLDIIW</INSDSeq_sequence></INSDSeq></Sequen
ceData><SequenceData

sequenceIDNumber="74"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q217"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q218"><INSDQualifier_name>note</INSDQualifier_name><NonEnglishQualifier_value>X
IS ASPARTIC ACID WITH A SUCCINYL MONOACYL
MODIFICATION</NonEnglishQualifier_value></INSDQualifier></INSDFeature_quals></INS
DFeature></INSDSeq_feature-
table><INSDSeq_sequence>XEEAVYFAHRDIIW</INSDSeq_sequence></INSDSeq></Sequen
ceData><SequenceData
sequenceIDNumber="75"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q219"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q220"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature></INSDSeq_feature-
table><INSDSeq_sequence>XEEALYFAHKDIIW</INSDSeq_sequence></INSDSeq></Sequen
ceData><SequenceData
sequenceIDNumber="76"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier

id="q221"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q222"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature></INSDSeq_feature-
table><INSDSeq_sequence>XEEAFYFAHKDIIW</INSDSeq_sequence></INSDSeq></Sequenc
eData><SequenceData
sequenceIDNumber="77"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q223"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q224"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q225"><INSDQualifier_name>note</INSDQualifier_name><NonEnglishQualifier_value>X
is lysine with an AEEA-gamma E-C18
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="78"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q226"><INSDQualifier_name>organism</INSDQualifier_name><INSDQualifier_value>syn
thetic

construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q227"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q228"><INSDQualifier_name>note</INSDQualifier_name><NonEnglishQualifier_value>X
is lysine with an AEEA-gamma E-C16
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="79"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q229"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q230"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q231"><INSDQualifier_name>note</INSDQualifier_name><NonEnglishQualifier_value>X
is lysine with an AEEA-gamma E-C12
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="80"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu

alifier><INSDQualifier
id="q232"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q233"><INSDQualifier_name>note</INSDQualifier_name><NonEnglishQualifier_value>X
is lysine with a fumaryl monoacyl modification and an AEEA-AEEA-γE -ODDA
modification</NonEnglishQualifier_value></INSDQualifier></INSDFeature_quals></INSDFeat
ure></INSDSeq_feature-
table><INSDSeq_sequence>XEEAVYFAHLDIIW</INSDSeq_sequence></INSDSeq></Sequen
ceData><SequenceData
sequenceIDNumber="81"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q234"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q235"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>2</INSDFeature_location><INSDFeature_quals><INSDQualifier
id="q236"><INSDQualifier_name>note</INSDQualifier_name><NonEnglishQualifier_value>X
is lysine with an AEEA-AEEA-gamma E-ODDA
modification</NonEnglishQualifier_value></INSDQualifier></INSDFeature_quals></INSDFeat
ure></INSDSeq_feature-
table><INSDSeq_sequence>XXEAVYFAHLDIIW</INSDSeq_sequence></INSDSeq></Sequen
ceData><SequenceData
sequenceIDNumber="82"><INSDSeq><INSDSeq_length>15</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..15</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q237"><INSDQualifier_name>organism</INSDQualifier_name><INSDQualifier_value>syn

thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q238"><INSDQualifier_name>note</INSDQualifier_name><NonEnglishQualifier_value>X
is aspartic acid with an ODDA-gamma E-AEEA-AEEA
modification</NonEnglishQualifier_value></INSDQualifier></INSDFeature_quals></INSDFeat
ure></INSDSeq_feature-
table><INSDSeq_sequence>XDEEAVYFAHLDIIW</INSDSeq_sequence></INSDSeq></Seque
nceData><SequenceData
sequenceIDNumber="83"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q239"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q240"><INSDQualifier_name>note</INSDQualifier_name><NonEnglishQualifier_value>X
is aspartic acid with an ODDA-γE-AEEA-AEEA-NH-C2H4-NH-succinyl monoacyl
modification</NonEnglishQualifier_value></INSDQualifier></INSDFeature_quals></INSDFeat
ure></INSDSeq_feature-
table><INSDSeq_sequence>XEEAVYFAHLDIIW</INSDSeq_sequence></INSDSeq></Sequen
ceData><SequenceData
sequenceIDNumber="84"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q241"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q242"><INSDQualifier_name>note</INSDQualifier_name><NonEnglishQualifier_value>X
IS 2-(2,5-DIOXOPYRROLIDIN-1-YL)
SUCCINATE</NonEnglishQualifier_value></INSDQualifier></INSDFeature_quals></INSDFea

ture><INSDFeature><INSDFeature_key>MOD_RES</INSDFeature_key><INSDFeature_locatio
n>10</INSDFeature_location><INSDFeature_quals><INSDQualifier
id="q243"><INSDQualifier_name>note</INSDQualifier_name><NonEnglishQualifier_value>X
is lysine with an ODDA-gamma E-AEEA-AEEA
modification</NonEnglishQualifier_value></INSDQualifier></INSDFeature_quals></INSDFeat
ure></INSDSeq_feature-
table><INSDSeq_sequence>XEEAVYFAHXDIIW</INSDSeq_sequence></INSDSeq></Sequen
ceData><SequenceData
sequenceIDNumber="85"><INSDSeq><INSDSeq_length>21</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..21</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q244"><INSDQualifier_name>organism</INSDQualifier_name><INSDQualifier_value>syn
thetic
construct</INSDQualifier_value></INSDQualifier></INSDFeature_quals></INSDFeature></IN
SDSeq_feature-
table><INSDSeq_sequence>CSCSSLMDKECVYFCHLDIIW</INSDSeq_sequence></INSDSeq
></SequenceData><SequenceData
sequenceIDNumber="86"><INSDSeq><INSDSeq_length>14</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..14</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q267"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q263"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q268"><INSDQualifier_name>note</INSDQualifier_name><NonEnglishQualifier_value>X
is lysine with an AEEA-gamma E-C12
modification</NonEnglishQualifier_value></INSDQualifier></INSDFeature_quals></INSDFeat
ure></INSDSeq_feature-
table><INSDSeq_sequence>XEEAVYFAHXDVIW</INSDSeq_sequence></INSDSeq></Seque

nceData><SequenceData
sequenceIDNumber="87"><INSDSeq><INSDSeq_length>15</INSDSeq_length><INSDSeq_mo
ltype>AA</INSDSeq_moltype><INSDSeq_division>PAT</INSDSeq_division><INSDSeq_featu
re-
table><INSDFeature><INSDFeature_key>source</INSDFeature_key><INSDFeature_location>1
..15</INSDFeature_location><INSDFeature_quals><INSDQualifier><INSDQualifier_name>mol
_type</INSDQualifier_name><INSDQualifier_value>protein</INSDQualifier_value></INSDQu
alifier><INSDQualifier
id="q252"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q253"><INSDQualifier_name>note</INSDQualifier_name><NonEnglishQualifier_value>X
IS ASPARTIC ACID WITH A FUMARYL MONOACYL
MODIFICATION</NonEnglishQualifier_value></INSDQualifier></INSDFeature_quals></INS
DFeature><INSDFeature><INSDFeature_key>MOD_RES</INSDFeature_key><INSDFeature_l
ocation>10</INSDFeature_location><INSDFeature_quals><INSDQualifier
id="q254"><INSDQualifier_name>note</INSDQualifier_name><NonEnglishQualifier_value>X
is lysine with an AEEA-E-C12
modification</NonEnglishQualifier_value></INSDQualifier></INSDFeature_quals></INSDFeat
ure></INSDSeq_feature-
table><INSDSeq_sequence>XDEEAVYFAHXDVIW</INSDSeq_sequence></INSDSeq></Sequ
enceData><SequenceData
sequenceIDNumber="88"><INSDSeq><INSDSeq_length>9</INSDSeq_length><INSDSeq_molt
ype>AA</INSDSeq_moltype><INSDSeq_division>PAT</INSDSeq_division><INSDSeq_featur
e-
table><INSDFeature><INSDFeature_key>source</INSDFeature_key><INSDFeature_location>1
..9</INSDFeature_location><INSDFeature_quals><INSDQualifier><INSDQualifier_name>mol_
type</INSDQualifier_name><INSDQualifier_value>protein</INSDQualifier_value></INSDQual
ifier><INSDQualifier
id="q269"><INSDQualifier_name>organism</INSDQualifier_name><INSDQualifier_value>syn
thetic
construct</INSDQualifier_value></INSDQualifier></INSDFeature_quals></INSDFeature><INS
DFeature><INSDFeature_key>MOD_RES</INSDFeature_key><INSDFeature_location>1</INS
DFeature_location><INSDFeature_quals><INSDQualifier
id="q262"><INSDQualifier_name>note</INSDQualifier_name><NonEnglishQualifier_value>X
is tyrosine with a Suc-DE cyclo (CEEC)
modification</NonEnglishQualifier_value></INSDQualifier></INSDFeature_quals></INSDFeat
ure></INSDSeq_feature-
table><INSDSeq_sequence>XFAHLDIIW</INSDSeq_sequence></INSDSeq></SequenceData>
</ST26SequenceListing>

## Claims

1. A polypeptide comprising:
(I) a structure as shown in Formula I,
X1-X2-X3-(A5)-X4-X5-(A1)-X6-(A2)-X7-X8-X9-(A3)-X10-X11-(A4)- X12-X13-X14-X15-X16 Formula I;
or
(II) a sequence having one or more amino acid substitutions, deletions, additions and/or replacements in the structure as shown in (I); or
(III) a sequence having 90% or more similarity to the structure as shown in (I); or
(IV) a pharmaceutically acceptable salt, solvate, chelate or noncovalent complex formed from the structure as shown in Formula I; and/or
(V) a prodrug based on the structure as shown in Formula I; and/or
(VI) any mixture comprising (IV) and/or (V);
wherein X1 is selected from Suc, Fum, MeO-Fum, Ac, SA, MA, FA, A1-D or Al-NH-C₂H₄-NH-Suc;
wherein X2 is selected from D, E or K(Al):
wherein X1 and X2 as a whole can be 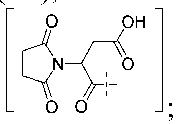
wherein X3 is selected from E, D or K(Al);
wherein X4 is selected from E, E(C16) or D;
wherein X5 is selected from A, K, R, H, E or D;
wherein X6 is selected from V, I, K, Dab, Orn, L, F, W or C;
wherein X7 is selected from Y, F or W;
wherein X8 is selected from F, Y or W;
wherein X9 is selected from A, K, R, H, D, E, Dab or Orn;
wherein X10 is selected from H, K or R;
wherein X11 is selected from K, L, L(N-Me), I, V, Cit, H, R, L-2-amino-4-guanidinobutyric acid or Orn;
wherein X12 is selected from D or E;
wherein X13 is selected from I, I(N-Me), 2-Abu, L, P or V;
wherein X14 is selected from I, 2-Abu, L or V;
wherein X15 is selected from W, F, Y or 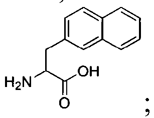
wherein X16 is selected from OH or NH₂;
wherein A5 is selected from C or absent;
wherein A1, A2, A3 or A4 independently comprises (Y1-Y2-Y3-Y4);
wherein Y1 is selected from -NH-(CH₂-CH₂-O)ml-(CH₂)m2-CO-, L/D-Glu, -CPAK- or absent, wherein L/D-Glu is attached to the amino group on the side chain of an amino acid on the backbone of the polypeptide through the α- or γ-carboxyl group, and to the carboxyl group of Y2 through the amino group;
wherein Y2 is selected from -NH-(CH₂-CH₂-O)m3-(CH₂)m4-CO-, 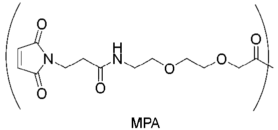 or absent;
wherein Y3 is selected from -NH-(CH₂-CH₂-O)m5-(CH₂)m6-CO-, L/D-Glu, or absent, wherein L/D-Glu is attached to the amino group of Y2 through the α- or γ-carboxyl group, and to the carboxyl group of Y4 through the amino group;
wherein Y4 is selected from D-Biotin, a structure as shown in Formula 2, HOOC-(CH₂)ₙ-CO, CH₃-(CH₂)ₙ-CO or absent, 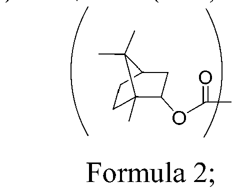
wherein m1, m2, m3, m4, m5 or m6 is an integer from 1 to 10;
wherein n is an integer selected from 10 to 25;
wherein A1, A2, A3, or A4 can only exist individually, or all of them are absent; and
wherein the polypeptide having the structure of Formula I does not include Suc-DEEAVYFAHLDIIW.

2. The polypeptide of claim 1, wherein
X1 is selected from Suc, Fum, MeO-Fum, Ac, SA, MA, FA, (ODDA-γE-AEEA-AEEA-)D or (ODDA-γE-AEEA-AEEA-)NH-C₂H₄-NH-Suc;
X2 is D or K(A1);
X1 and X2 as a whole can be 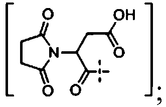
X3 is E or K(A1);
X4 is selected from E or E(C16);
X5 is selected from A, K or E;
X6 is selected from V, K, Dab, L, F or C;
X7 is Y;
X8 is F;
X9 is selected from A, K or E;
X10 is H;
X11 is selected from K, L, L(N-Me), R, Cit, L-2-amino-4-guanidinobutyric acid or Orn;
X12 is D;
X13 is selected from I, I(N-Me), 2-Abu, P or V;
X14 is I;
X15 is selected from W or 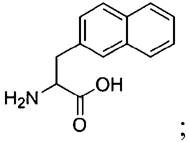
A1 is selected from -γE-AEEA-AEEA-ODDA, ODDA-γE-AEEA-AEEA-, Biotin, -AEEA-AEEA-γE-ODDA or absent;
A2 is selected from -γE-AEEA-AEEA-ODDA or absent;
A3 is selected from -γE-AEEA-AEEA-ODDA or absent;
A4 is selected from -AEEA-AEEA-γE-ODDA, -γE-AEEA-AEEA-C18, -γE-AEEA-AEEA-C16, -γE-AEEA-AEEA-C12, -γE-AEEA-AEEA-B, -γE-AEEA-AEEA-ODDA, CPAK-MPA-, -AEEA-γE-C18, -AEEA-γE-C16, -AEEA-γE-C12, -AEEA-E-C12 or absent.

3. The polypeptide of claim 1 or 2, wherein the structure shown in Formula I is subjected to cyclization by any one of:
(I) introducing a C to form a disulfide bond; and/or
(II) forming an amide bond between the carboxyl group at the side chain of a D or a E and the amino group at the side chain of a K or a Dab.

4. The polypeptide of claim 3, wherein the cyclization comprises any one of:
(I) inserting a C between X3 and X4 while replacing X6 with a C, and cyclizing through a disulfide bond formed by the two Cs; and/or
(II) cyclizing through an amide bond formed by the E of X3 and the K or Dab of X6; and/or
(III) cyclizing through an amide bond formed by the E of X5 and the K of X11.

5. The polypeptide of claim 1, wherein the polypeptide sequence is as follows:
| Serial Number SEQ ID No. | ID | Sequence | MW |
|---|---|---|---|
| 2 | BX-5 | Suc-DEEAVYFAHKDIIW | 1836 |
| 3 | F-BX-5 | Fum-DEEAVYFAHKDIIW | 1834 |
| 4 | BX-7 | Suc-DEEAVYFAHLDVIW | 1806 |
| 5 | BX-8 | Suc-DEEAVYFAHLD(2-Abu)IW | 1792 |
| 6 | BX-34 | Fum-DEEAVYFAHK(-AEEA-AEEA-γE-ODDA)DIIW | 2549 |
| 7 | BX-43 | Suc-DEEAKYFAHLDIIW | 1850 |
| 8 | BX-45 | Suc-DEEEVYFAHKDIIW | 1893 |
| 9 | BX-48 | Suc-D cyclo(EEAK)YFAHLDIIW | 1832 |
| 10 | BX-49 | Suc-D cyclo(EEADab)YFAHLDIIW | 1804 |
| 11 | BX-50 | Suc-DEE cyclo(EVYFAHK)DIIW | 1876 |
| 12 | BX-51 | Fum-DEEAVYFAHOrn(-AEEA-AEEA-γE-ODDA)DIIW | 2535 |
| 13 | BX-52 | Fum-DEEEVYFAHKDIIW | 1891 |
| 14 | BX-53 | Suc-DEEEVYFAHOrnDIIW | 1880 |
| 15 | BX-54 | Suc-DEEEVYFAHK(-AEEA-AEEA-γE-ODDA)DIIW | 2610 |
| 16 | BX-57 | Suc-DE cyclo(CEEC)YFAHKDIIW | 1999 |
| 17 | BX-58 | MeO-Fum-DEEEVYFAHKDIIW | 1906 |
| 18 | BX-59 | Fum-DEEEVYFAHOmDIIW | 1878 |
| 19 | BX-60 | Fum-DEEEVYFAHK(-AEEA-AEEA-γE-ODDA)DIIW | 2608 |
| 20 | BX-61 | Suc-DEEAVYFAHOrn(-AEEA-AEEA-γE-ODDA)DIIW | 2538 |
| 21 | BX-62 | Fum-DEE cyclo(EVYFK)HKD(2-Abu)IW | 1903 |
| 22 | BX-63 | Suc-D cyclo(EEAK)YFAHLD(2-Abu)IW | 1804 |
| 23 | BX-64 | Fum-D cyclo(EEAK)YFAHLD(2-Abu)IW | 1802 |
| 24 | BX-65 | MeO-Fum-D cyclo(EEAK)YFAHLD(2-Abu)IW | 1814 |
| 25 | BX-66 | Fum-DEEK(-γE-AEEA-AEEA-ODDA)VYFAHLDIIW | 2592 |
| 26 | BX-67 | Fum-DEEAK(-γE-AEEA-AEEA-ODDA)YFAHLDIIW | 2564 |
| 27 | BX-68 | Fum-DEEAVYFK(-γE-AEEA-AEEA-ODDA)HLDIIW | 2592 |
| 28 | BX-69 | Fum-DEEAVYFAHK(-γE-AEEA-AEEA-C18)DIIW | 2520 |
| 29 | BX-70 | Fum-DEEAVYFAHK(-γE-AEEA-AEEA-C16)DIIW | 2492 |
| 30 | BX-71 | Fum-DEEAVYFAHK(-γE-AEEA-AEEA-C12)DIIW | 2436 |
| 31 | BX-72 | Fum-DEEAVYFAHK(-γE-AEEA-AEEA-B)DIIW | 2433 |
| 32 | BX-74 | Suc-DEEAVYFAHK(-γE-AEEA-AEEA-ODDA)DIIW | 2552 |
| 33 | BX-75 | MeO-Fum-DEEAVYFAHK(-γE-AEEA-AEEA-ODDA)DIIW | 2564 |
| 34 | BX-76 | Suc-D cyclo(EEAK)YFAHLDVIW | 1818 |
| 35 | BX-77 | Suc-D cyclo(EEAK)YFAHK(-γE-AEEA-AEEA-ODDA)DIIW | 2563 |
| 36 | BX-78 | Suc-DEEAVYFAHK(-γE-AEEA-AEEA-ODDA)DVIW | 2538 |
| 37 | BX-79 | Suc-D cyclo(EEAK)YFAHK(-γE-AEEA-AEEA-ODDA)DVIW | 2550 |
| 38 | BX-86 | Fum-DEEAVYFAHK(-γE-AEEA-AEEA-ODDA)DIIW-NH2 | 2549 |
| 39 | BX-95 | Fum-DEEAVYFAHK(CPAK-MPA-)DIIW | 2661 |
| | | 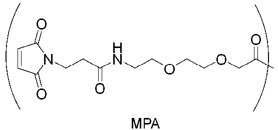 | |
| 40 | BX-99 | Fum-D cyclo(EEAK)YFAHLDVIW | 1916 |
| 41 | BX-100 | Fum-D cyclo(EEAK)YFAHK(-γE-AEEA-AEEA-ODDA)DVIW | 2547 |
| 42 | BX-101 | Fum-DEEAVYFAHK(-γE-AEEA-AEEA-ODDA)DVIW | 2536 |
| 43 | BX-102 | Fum-D cyclo(EEAK)YFAHLDIIW | 1829 |
| 44 | BX-103 | Fum-D cyclo(EEAK)YFAHK(-γE-AEEA-AEEA-ODDA)DIIW | 2561 |
| 45 | BX-104 | Fum-D cyclo(EEAK)YFAHK(-γE-AEEA-AEEA-ODDA)D(2-Abu)IW | 2534 |
| 46 | BX-105 | Fum-DEEAKYFAHLDIIW | 1847 |
| 47 | BX-106 | Fum-DEEAKYFAHLDVIW | 1836 |
| 48 | BX-107 | Fum-DEEAKYFAHK(-γE-AEEA-AEEA-ODDA)DIIW | 2579 |
| 49 | BX-108 | Fum-DEEAKYFAHK(-γE-AEEA-AEEA-ODDA)DVIW | 2567 |
| 50 | BX-109 | Fum-DEEAK(-γE-AEEA-AEEA-ODDA)YFAHLDIIW | 2566 |
| 51 | BX-110 | Fum-DEEAK(-γE-AEEA-AEEA-ODDA)YFAHLDVIW | 2549 |
| 52 | BX-111 | SA-DEEAVYFAHLDIIW | 1844 |
| 53 | BX-112 | MA-DEEAVYFAHLDIIW | 1844 |
| 54 | BX-13-2 | Fum-DEEAVYFAHKDIIW1 | 1843.8 |
| | | 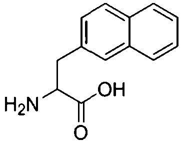 W1: | |
| 55 | BX-9 | suc-DEEAVYFAHR1DIIW (R1=L-2-amino-4-guanidinobutyric acid) | 1849.98 |
| 56 | BX-31 | Fum-DEEAVYFAHOrnDIIW | 1819 |
| 57 | BX-32 | Fum-DEEAVYFAHCitDIIW | 1862 |
| 58 | BX-38 | Suc-DE cyclo(CEAC)YFAHLDIIW | 1927 |
| 59 | BX-23 | Fum-DEEAVYFAHKDVIW | 1704.9 |
| 60 | BX-24 | Fum-DEEAVYFAHKD(2-Abu)IW | 1805.9 |
| 61 | BX-25 | Suc-DEEAVYFAHLDI(N-Me)IW | 1833.85 |
| 62 | BX-26 | Suc-DEEAVYFAHL(N-Me)DIIW | 1833.85 |
| 63 | BX-27 | Suc-DEEAVYFAHL(N-Me)DI(N-Me)IW | 1847.86 |
| 64 | BX-28 | Fum-DEEAVYFAHL(N-Me)DIIW | 1845.85 |
| 65 | BX-29 | Fum-DEEAVYFAHL(N-Me)DI(N-Me)IW | 1847.86 |
| 66 | FBX-7 | Fum-DEEAVYFAHLDVIW | 1805.9 |
| 67 | FBX-8 | Fum-DEEAVYFAHLD(2-Abu)IW | 1790.9 |
| 68 | QP-1 | 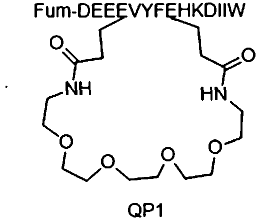 | 1848 |
| 69 | QP-3 | 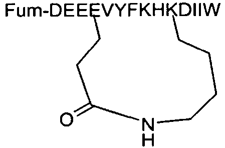 | 1931 |
| 70 | BX-15 | FA-DEEAVYFAHKDIIW | 1912.87 |
| 71 | BX-16 | Fum-DEEE(Biotin)VYFAHKDIIW | 2160.4 |
| 72 | BX-10 | suc-DEEAVYFAHLDPIW | 1804.96 |
| 73 | BX14-1 | Fum-DEE(C16)AVYFAHLDIIW | 2044.1 |
| 74 | BX-6 | Suc-DEEAVYFAHRDIIW | 1864.03 |
| 75 | BX21 | Fum-DEEALYFAHKDIIW | 1848 |
| 76 | BX22 | Fum-DEEAFYFAHKDIIW | 1882 |
| 77 | BX-223 | Fum-DEEAVYFAHK(-AEEA-γE-C18 )DIIW | 2374 |
| 78 | BX-224 | Fum-DEEAVYFAHK(-AEEA-γE-C16 )DIIW | 2346 |
| 79 | BX-225 | Fum-DEEAVYFAHK(-AEEA-γE-C12 )DIIW | 2290 |
| 80 | BX-253 | Fum-K(-AEEA-AEEA-γE-ODDA)EEAVYFAHLDIIW | 2547 |
| 81 | BX-254 | Fum-DK(-AEEA-AEEA-γE-ODDA)EAVYFAHLDIIW | 2533 |
| 82 | BX-255 | (ODDA-γE-AEEA-AEEA-)DDEEAVYFAHLDIIW | 2550 |
| 83 | BX-256 | (ODDA-γE-AEEA-AEEA-)NH-C₂H₄-NH-Suc-DEEAVYFAHLDIIW | 2594 |
| 84 | BX-221 | 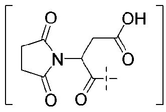 | 2533 |
| | | BEAVYFAHK(ODDA-γE-AEEA-AEEA-)DIIW | |
| 86 | BX-229 | Fum-DEEAVYFAHK(-AEEA-γE-C12)DVIW | 2276 |
| 87 | BX-238 | Fum-DEEAVYFAHK(-AEEA-E-C12)DVIW | 2276 |
| 88 | BX-56 | Suc-DE cyclo(CEEC)YFAHLDIIW | 1984 |

6. A method of preparing the polypeptide of any one of claims 1 to 5, comprising the following steps:
Step 1: obtaining a peptide resin of the polypeptide via solid-phase polypeptide synthesis; and
Step 2: cleaving and purifying to obtain the polypeptide.

7. A polypeptide prepared according to the method of claim 6.

8. Use of any one of:
(I) the polypeptide of any one of claims 1 to 5; and/or
(II) the polypeptide of claim 7,
in the manufacture of a medicament for the treatment of an ETBR receptor-related disease.

9. The use of claim 8, wherein the ETBR receptor-related disease comprises one or more of stroke, Alzheimer's disease, spinal cord injury, aortic stenosis, or neonatal hypoxic-ischemic encephalopathy; and
wherein the stroke comprises ischemic stroke and/or hemorrhagic stroke.

10. A medicament comprising any one of:
(I) the polypeptide of any one of claims 1 to 5; and/or
(II) the polypeptide of claim 7;
and a pharmaceutically acceptable excipient or adjuvant.

11. A pharmaceutical composition comprising the medicament of claim 10 and any other active ingredient.

12. A method of treating an ETBR receptor-related disease, comprising administering to a subject any one of:
(I) the polypeptide of any one of claims 1 to 5; and/or
(II) the polypeptide of claim 7; and/or
(III) the medicament of claim 10; and/or
(IV) the pharmaceutical composition of claim 11.

13. The method of claim 12, wherein the ETBR receptor-related disease comprises one or more of stroke, Alzheimer's disease, spinal cord injury, aortic stenosis, or neonatal hypoxic-ischemic encephalopathy; and
wherein the stroke comprises ischemic stroke and/or hemorrhagic stroke.
